# EUROPEAN PATENT SPECIFICATION

(11) **EP 0 595 557 B1**
(45) Date of publication and mention of the grant of the patent: **30.06.2004**
(21) Application number: 93308421.2
(22) Date of filing: 22.10.1993
(51) Int. Cl.: C07D 205/08, C07D 405/12, C07D 403/12, C07D 401/12, A61K 31/395

(54) **New substituted azetidinones as anti-inflammatory and antidegenerative agents**
Substituierte Azetidinone als entzüngdungshemmende und antidegenerative Wirkstoffe
Azetidinones substituées comme agents anti-inflammatoires et antidégénératifs

(30) Priority: 27.10.1992 US 966800; 17.12.1992 US 991838
(43) Date of publication of application: 04.05.1994
(73) Proprietor: Merck & Co., Inc., Rahway New Jersey 07065-0900 (US)
(72) Inventor: Doherty, James B., Montvale, NJ 07645 (US); Finke, Paul E., Milltown, NJ 08850 (US); Dorn, Conrad P., Plainfield, NJ 07062 (US); Maccoss, Malcolm, Freehold, NJ 07729 (US); Durette, Philippe L., New Providence, NJ 07974 (US); Mills, Sander G., Woodbridge, NJ 07095 (US); Shah, Shrenik K., Metuchen, NJ 08840 (US); Lanza, Thomas J., Edison, NJ 08820 (US); Sahoo, Soumya P., Old Bridge, NJ 08857 (US); Hagmann, William K., Westfield, NJ 07090 (US); Hale, Jeffrey J., Westfield, NJ 07090 (US)
(74) Representative: Horgan, James Michael Frederic

(56) References cited:
- EP-A- 0 337 549
- EP-A- 0 481 671
- EP-A- 0 525 973

## Description

We have found that a group of new substituted azetidinones are potent elastase inhibitors and therefore are useful anti-inflammatory and antidegenerative agents.

Proteases from granulocytes and macrophages have been reported to be responsible for the chronic tissue destruction mechanisms associated with inflammation, including rheumatoid arthritis and emphysema. Accordingly, specific and selective inhibitors of these proteases are candidates for potent anti-inflammatory agents useful in the treatment of inflammatory conditions resulting in connective tissue destruction, e.g. rheumatoid arthritis, emphysema, bronchial inflammation, chronic bronchitis, glomerulonephritis, osteoarthritis, spondylitis, lupus, psoriasis, atherosclerosis, sepsis, septicemia, shock, myocardial infarction, reperfusion injury, periodontitis, cystic fibrosis and acute respiratory distress syndrome.

The role of proteases from granulocytes, leukocytes or macrophages are related to a rapid series of events which occurs during the progression of an inflammatory condition:
(1) There is a rapid production of prostaglandins (PG) and related compounds synthesized from arachidonic acid. This PG synthesis has been shown to be inhibited by aspirin-related nonsteroidal anti-inflammatory agents including indomethacin and phenylbutazone. There is some evidence that protease inhibitors prevent PG production;
(2) There is also a change in vascular permeability which causes a leakage of fluid into the inflamed site and the resulting edema is generally used as a marker for measuring the degree of inflammation. This process has been found to be induced by the proteolytic or peptide cleaving activity of proteases, especially those contained in the granulocyte, and thereby can be inhibited by various synthetic protease inhibitors, for example, N-acyl benzisothiazolones and the respective 1,1-dioxides. Morris Zimmerman et al., J. Biol. Chem., 255, 9848 (1980); and
(3) There is an appearance and/or presence of lymphoid cells, especially macrophages and polymorphonuclear leukocytes (PMN). It has been known that a variety of proteases are released from the macrophages and PMN, further indicating that the proteases do play an important role in inflammation.

In general, proteases are an important family of enzymes within the peptide bond cleaving enzymes whose members are essential to a variety of normal biological activities, such as digestion, formation and dissolution of blood clots, the formation of active forms of hormones, the immune reaction to foreign cells and organisms, etc., and in pathological conditions such as the degradation of structural proteins at the articular cartilage/pannus junction in rheumatoid arthritis etc.

Elastase is one of the proteases. It is an enzyme capable of hydrolyzing the connective tissue component elastin, a property not contained by the bulk of the proteases present in mammals. It acts on a protein's nonterminal bonds which are adjacent to an aliphatic amino acid. Neutrophil elastase is of particular interest because it has the broadest spectrum of activity against natural connective tissue substrates. In particular, the elastase of the granulocyte is important because, as described above, granulocytes participate in acute inflammation and in acute exacerbation of chronic forms of inflammation which characterize many clinically important inflammatory diseases.

Proteases may be inactivated by inhibitors which block the active site of the enzyme by binding tightly thereto. Naturally occurring protease inhibitors form part of the control or defense mechanisms that are crucial to the well-being of an organism. Without these control mechanisms, the proteases would destroy any protein within reach. The naturally occurring enzyme inhibitors have been shown to have appropriate configurations which allow them to bind tightly to the enzyme. This configuration is part of the reason that inhibitors bind to the enzyme so tightly (see Stroud, "A Family of Protein-Cutting Proteins" Sci. Am. July 1974, pp. 74-88). For example, one of the natural inhibitors, α₁-Antitrypsin, is a glycoprotein contained in human serum that has a wide inhibitory spectrum covering, among other enzymes, elastase both from the pancreas and the PMN. This inhibitor is hydrolyzed by the proteases to form a stable acyl enzyme in which the active site is no longer available. Marked reduction in serum α₁-antitrypsin, either genetic or due to oxidants, has been associated with pulmonary emphysema which is a disease characterized by a progressive loss of lung elasticity and resulting respiratory difficulty. It has been reported that this loss of lung elasticity is caused by the progressive, uncontrolled proteolysis or destruction of the structure of lung tissue by proteases such as elastase released from leukocytes. J. C. Powers, TIBS, 211 (1976).

Rheumatoid arthritis is characterized by a progressive destruction of articular cartilage both on the free surface bordering the joint space and at the erosion front built up by synovial tissue toward the cartilage. This destruction process, in turn, is attributed to the protein-cutting enzyme elastase which is a neutral protease present in human granulocytes. This conclusion has been supported by the following observations:
(1) Recent histochemical investigations showed the accumulation of granulocytes at the cartilage/pannus junction in rheumatoid arthritis; and
(2) a recent investigation of mechanical behavior of cartilage in response to attack by purified elastase demonstrated the direct participation of granulocyte enzymes, especially elastase, in rheumatoid cartilage destruction. H. Menninger et al., in Biological Functions of Proteinases, H. Holzer and H. Tschesche, eds. Springer-Verlag, Berlin, Heidelberg, New York, pp. 196-206, 1979.

In a second aspect this invention concerns the use of novel azetidinones in the treatment of certain cancers including nonlymphoblastic leukemias, acute myelogenous leukemia (FAB M1 and FAB M2), acute promyelocytic leukemia (FAB M3), acute myelomonocytic leukemia (FAB M4), acute monocytic leukemia (FAB M5), erythroleukemia, chronic myelogenous leukemia, chronic myelomonocytic leukemia, chronic monocytic leukemia and conditions associated with leukemia involving activity of PMN neutral proteases e.g. disseminated intravascular coagulation. We have found that the substituted azetidinones disclosed herein are inhibitors of proteinase 3 (PR-3), also known as myeloblastin.

See C. Labbaye, et al., Proc. Natl. Acad. Sci. USA, vol. 88, 9253-9256, (1991), Wegner autoantigen and myeloblastin are encoded by a single mRNA; D. Campanelli, et al., J. Exp. Med., vol. 172, 1709-1714, (1990), Cloning of cDNA for proteinase 3: A serine protease, antibiotic, and autoantigen from human neutrophils; and Bories, et. al., Cell vol. 59, 959-968, (1989) Down-regulation of a serine protease, myeloblastin, causes growth arrest and differentiation of promyelocytic leukemia cells.

Recently, down regulation of PR-3 has been implicated in the proliferation and maintenance of a differentiated state of certain leukemia cells. In particular, Bories, et. al., have shown that expression of this enzyme, hereinafter designated proteinase 3/myeloblastin, can be inhibited by treatment of HL-60 human leukemia cells with an antisense oligodeoxynucleotide and that such treatment induces differentiation and inhibits proliferation of these cells. Moreover, we have now demonstrated that the treatment of the HL-60 cell human leukemia cell line, among others, with the compounds of the instant invention, likewise results in the inhibition of proliferation and induction of differentiation in such cells.

EP-A-0 337 549 (Merck & Co., Inc.) discloses substituted azetidinones as elastase inhibitors.

EP-A-0 481 671 (Merck & Co. Inc.) discloses substituted azetidinones as elastase inhibitors.

Accordingly, we believe that treatment of leukemia such as nonlymphoblastic leukemias, acute myelogenous leukemia (FAB M1 and FAB M2), acute promyelocytic leukemia (FAB M3), acute myelomonocytic leukemia (FAB M4), acute monocytic leukemia (FAB M5), erythroleukemia, chronic myelogenous leukemia, chronic myelomonocytic leukemia, chronic monocytic leukemia and conditions associated with leukemia involving activity of PMN neutral proteases e.g. disseminated intravascular coagulation, comprising: administration of a therapeutically effective amount of compound of Formula I will result in remission of the disease state. Administration may be either oral or parenteral.

### BRIEF DESCRIPTION OF THE INVENTION

The instantly claimed invention is directed to specifically substituted azetidinones of Formula I

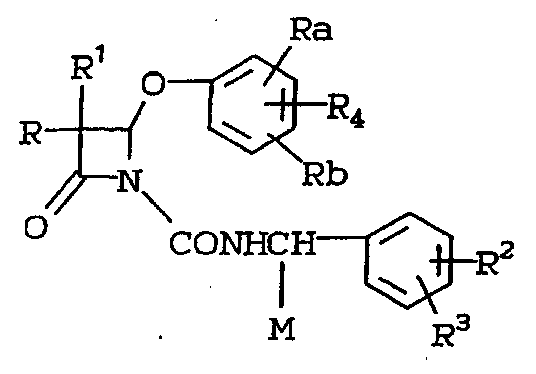

These substituted azetidinones have been found to be useful anti-inflammatory and antidegenerative agents. This invention is also directed to pharmaceutical compositions and methods of using these specifically substituted azetidinones. These compounds will also be useful in the treatment of certain leukemias and leukemia related conditions.

### DETAILED DESCRIPTION OF THE INVENTION

This invention relates to potent elastase inhibitors of Formula (I), 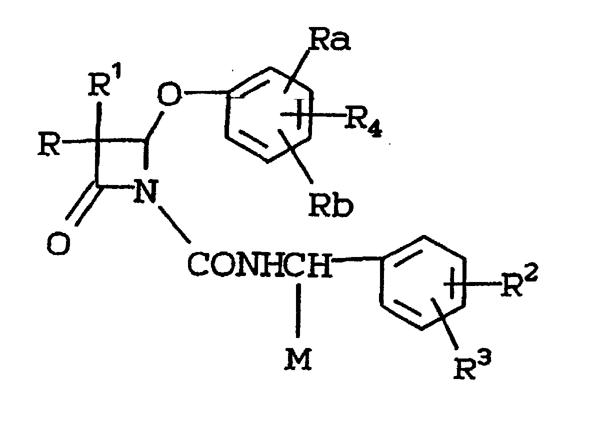 which are useful in the prevention, control and treatment of inflammatory and degenerative conditions especially arthritis and emphysema.

More particularly, the instant invention is directed to the compounds of the Formula (I) 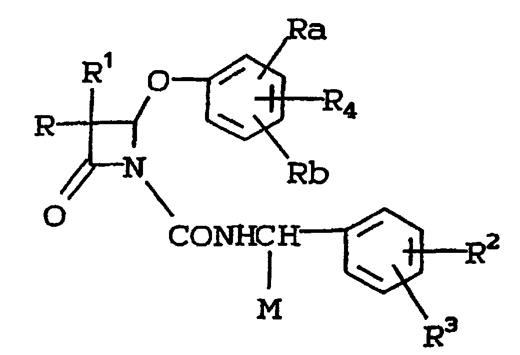 and pharmaceutically acceptable salts thereof wherein:
R is C₁₋₆alkyl;
R¹ is C₁₋₆alkyl or C₁₋₆alkoxy-C₁₋₆alkyl;
M is
   (1) hydrogen,
   (2) C₁₋₆alkyl,
   (3) hydroxy C₁₋₆alkyl,
   (4) halo C₁₋₆alkyl,
   (5) C₂₋₆alkenyl, or
   (6) C₁₋₆alkoxy-C₁₋₆alkyl;
Ra and Rb are each individually
   (1) hydrogen,
   (2) C₁₋₆alkyl,
   (3) halo,
   (4) carboxy,
   (5) C₁₋₆alkoxy,
   (6) phenyl,
   (7) C₁₋₆alkylcarbonyl,
   (8) di-(C₁₋₆alkyl)amino;
   (9) hydroxy;
R² and R³ are each independently
   (1) hydrogen,
   (2) C₁₋₆alkyl,
   (3) halo,
   (4) carboxy,
   (5) C₁₋₆alkoxy,
   (6) phenyl,
   (7) C₁₋₆alkylcarbonyl,
   (8) aminoC₂₋₃alkyloxy carbonyl wherein the amino is optionally mono or di substituted with C₁₋₆alkyl,
   (9) aminoC₂₋₃alkylamino carbonyl wherein the amino is optionally mono or di substituted with C₁₋₆alkyl,
   (10) hydroxy,
   (11) aminocarbonyl wherein.the amino is optionally mono or di substituted with C₁₋₆alkyl,
   (12) hydroxymethyl,
   (13) aminocarbonyloxy C₁₋₃alkyloxy wherein the amino is optionally mono or di substituted with C₁₋₆alkyl,
   (14) cyano,
   (15) morpholinocarbonylphenyl,
   (16) amino wherein the amino is optionally mono or di substituted with C₁₋₆alkyl, or R² and R³ may be joined together to form a methylenedioxy group or a furan ring,
   (17) morpholinocarbonyl;
R₄ is 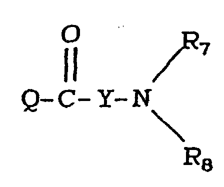
wherein
Q is a covalent bond and
Y is 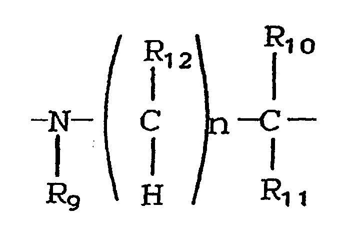
R₁₂ is hydrogen or C₁₋₃alkyl;
R₇ and R₈ are each individually
   (a) hydrogen,
   (b) C₁₋₆alkyl,
   (c) C₁₋₆alkyloxy C₂₋₃alkyl,
   (d) hydroxy C₂₋₆alkyl,
   (e) polyhydroxyC₂₋₆alkyl,
   (f) carboxamido C₁₋₆alkyl,
   (h) C₁₋₆alkanoyl,
   (i) substituted phenyl or phenyl C₁₋₆alkyl, wherein the substituents are X₁ and X₂ as defined immediately below,
   (j) C₂₋₆alkenyl,
   (k) C₆₋₁₀cycloalkenyl,
   (l) heteroaryl C₁₋₆alkyl wherein the hetero aryl is pyridinyl, imidazolyl, triazolyl, benzylimidazolyl, and furyl,
   (m) carboxy C₁₋₆alkyl,
   (n) carbo C₁₋₆alkoxy C₁₋₃alkyl,
   (o) phenylsulfonyl,
   (p) C₁₋₆alkylsulfonyl,
   (q) benzyloxy,
   (r) morpholinyl C₁₋₃alkylsulfonyl,
   (s) tetrahydropyranyl,
   (t) aminoC₁₋₃alkylsulfonyl wherein the amino is optionally mono or di substituted with C₁₋₆alkyl,
   (u) aminocarbonyl wherein the amino is optionally mono or di substituted with C₁₋₆alkyl,
   (v) aminocarbonyloxyC₂₋₆alkyl wherein the amino is optionally mono or di substituted with C₁₋₆alkyl,
   (w) azabicyclo of 7 to 12 atoms,
   (x) di C₁₋₃alkylamino C₂₋₆alkyl wherein the amino is optionally mono or di substituted with C₁₋₆alkyl,
   (y) bicycloalkyl of 7 to 12 atoms,
   (z) C₃₋₁₀cycloalkyl optionally substituted with C₁₋₆alkyl,
   (aa) pyrazolidinyl,
   (bb) substituted piperidinyl or prrrolidinyl wherein the substituent is hydrogen, C₁₋₃alkyl, hydroxyC₁₋₃alkylbenzyl, carboxamido or amino wherein the amino is optionally mono or di substituted with C₁₋₆alkyl,
   (cc) substituted pyrrolidinyl wherein the substituent is carboxamido or amino wherein the amino is optionally mono or di substituted with C₁₋₆alkyl,
   (dd) pyrimidinyl,
   (ee) N-cyano-N'-phenylamidino,
   (ff) phosphonoC₁₋₆alkyl, or
   (gg) α-C₁₋₃alkyl benzyl or mono or di substituted benzyl or mono or di substituted pyridylmethyl, wherein the substituents are X₁ and X₂,
wherein
X₁ is
   (1) hydrogen,
   (2) halo,
   (3) C₁₋₆alkyl,
   (4) halo-C₁₋₆alkyl,
   (5) C₂₋₆alkenyl,
   (6) hydroxy-C₁₋₆alkyl,
   (7) C₁₋₆alkylcarbonyl,
   (8) C₁₋₆alkylcarbonylamino;
   (9) CN,
   (10) CF₃,
   (11) CH₃O,
   (12) amino wherein the amino is optionally mono or di substituted with C₁₋₆alkyl;
   (13) carboxy, or
   (14) phenylsulfonylaminocarbonyl;
X₂ is hydrogen, halo or C₁₋₆alkyl;
n is 1, 2, 3, 4 or 5;
R₉ is selected from hydrogen, C₁₋₄ alkyl, and C₁₋₃alkoxyC₁₋₃alkyl; or phenyl, phenyl C₁₋₃alkyl, pyridyl, and pyridyl C₁₋₃alkyl;
R₁₀ and R₁₁ are each independently selected from hydrogen, C₁₋₄alkyl, and C₁₋₃alkoxy C₁₋₃alkyl, or are together 0=; or
R₇ and R₈ are joined together to form mono or di substituted ring of 4, 5, 6, or 7 atoms or 7 to 12 atoms such as
(1) piperidinyl or homopiperidinyl,
(2) piperazinyl,
(3) morpholinyl, thiomorpholinyl or 1,1-dioxo-4-thiomorpholinyl,
(4) pyrrolidinyl,
(5) pyrryl,
(6) imidazolyl,
(7) triazolyl,
(8) saturated azabicyclo of 7 to 12 atoms,
(9) azaspiro having 3 to 9 carbon atoms, said ring being saturated,
(10) tetrazolyl,
(11) pyrazolidinyl,
(12) dihydrodimethylisoquinolyl,
(13) azetidinyl, or
(14) diazabicyclo ring of 7-12 atoms,
wherein the substituents are each selected from the group consisting of hydrogen and C₁₋₃alkyl, benzyloxycarbonyl, carboxy, phenyl C₁₋₃alkyl amino carbonyl, pyrrolidinylmethyl, hydroxy C₁₋₃alkyl, C₁₋₆alkyloxy, C₁₋₄alkyloxy carbonyl, aminocarbonyl wherein the amino is optionally mono or di substituted with C₁₋₆alkyl, and oxo; or -N(R7)R8 may be a lysine residue; or
R₈ and R₉ are joined together to form a mono or di substituted saturated monocyclic ring of 6 to 7 atoms and having two hetero atoms which are the nitrogens to which R₈ and R₉ are attached; said rings to include piperazinyl and homopiperazinyl; or R₉ and R₁₀ are joined together to form a mono or di substituted monocyclic saturated ring of 5 to 7 atoms and having one hetero atom which is the nitrogen to which R₉ is attached; or
R₉ and R₁₂ are joined together to form a mono or di substituted saturated monocyclic ring of 5, 6, or 7 atoms, said ring having one hetero atom which is the nitrogen to which R₉ is attached; or
R₁₀ and R₁₂ are joined together to form a mono or di substituted saturated monocyclic ring of 5, 6, or 7 carbon atoms; or
R₈ and R₁₁ are joined together to form a mono or di substituted saturated monocyclic ring of 5, 6, or 7 atoms, said ring having one hetero atom which is the nitrogen to which R₈ is attached; and the substituents are independently selected from hydrogen and C1-3alkyl.

As appreciated by those of skill in the art the term "alkyl" such as in C₁₋₆alkyl, includes, methyl, ethyl, propyl, butyl, pentyl, and hexyl, and where appropriate, branched chained forms including isopropyl and tert-butyl.

As may also be appreciated by those of skill in the art, the 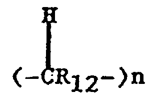 spacer in definition Y, may, in the alternative be placed to the right of CR₁₀R₁₁.

As may also be appreciated, the group 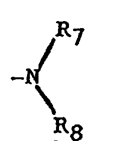 may also be oxidized to the corresponding oxide

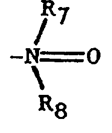

In one class the instant invention is directed to the compounds of the Formula (I) 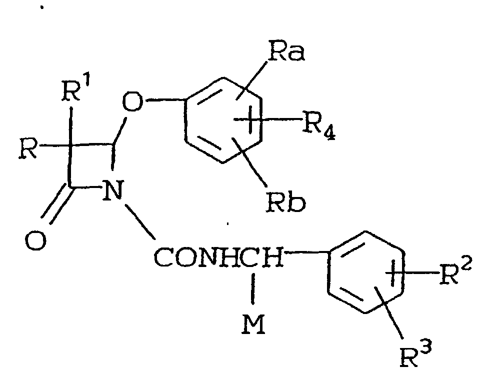 and pharmaceutically acceptable salts thereof wherein:
R is C₁₋₆alkyl;
R¹ is C₁₋₆alkyl or C₁₋₆alkoxy-C₁₋₆alkyl;
M is
   (1) hydrogen,
   (2) C₁₋₆alkyl,
   (3) hydroxy C₁₋₆alkyl,
   (4) halo C₁₋₆alkyl,
   (5) C₂₋₆alkenyl, or
   (6) C₁₋₆alkoxy-C₁₋₆alkyl;
Ra is
   (1) hydrogen,
   (2) C₁₋₆alkyl,
   (3) halo,
   (4) carboxy,
   (5) C₁₋₆alkoxy,
   (6) phenyl,
   (7) C₁₋₆alkylcarbonyl,
   (8) di-(C₁₋₆alkyl)amino;
Rb is hydrogen or C₁₋₆alkyl,
R² and R³ are each independently
   (1) hydrogen,
   (2) C₁₋₆alkyl,
   (3) halo,
   (4) carboxy,
   (5) C₁₋₆alkoxy,
   (6) phenyl,
   (7) C₁₋₆alkylcarbonyl,
   (8) di-(C₁₋₆alkyl)amino, or R² and R³ may be joined together to form a methylenedioxy group or a furan ring;
R₄ is (a) 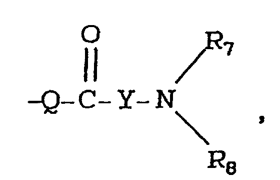 or
wherein
Q is a covalent bond,
Y is 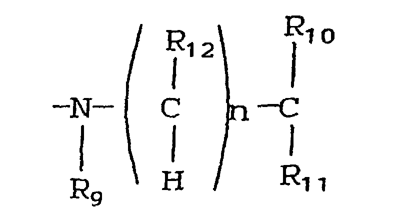
R₁₂ is hydrogen or C₁₋₃alkyl;
R₇ and R₈ are each individually
   (a) hydrogen,
   (b) C₁₋₆alkyl,
   (c) C₁₋₆alkyloxy C₂₋₃alkyl,
   (d) hydroxy C₂₋₆alkyl,
   (e) carboxamido C₁₋₆alkyl,
   (f) C₁₋₆alkanoyl,
   (g) phenyl or phenyl C₁₋₆alkyl,
   (h) C₂₋₆alkenyl,
   (i) C₆₋₁₀cycloalkenyl,
   (j) heteroaryl C₁₋₆alkyl wherein the hetero aryl is pyridinyl, imidazolyl, triazolyl, benzylimidazolyl, and furyl,
   (k) carboxy C₁₋₆alkyl,
   (l) C₁₋₆alkylsulfonyl,
   (m) carboC₁₋₆alkyloxyC₂₋₃alkyl,
   (n) morpholinyl C₁₋₃alkylsulfonyl,
   (o) aminoC₁₋₃alkylsulfonyl wherein the amino may be optionally substituted with C₁₋₆alkyl, (p)aminocarbonyl wherein the amino is optionally mono or di substituted with C₁₋₆alkyl,
   (q) aminocarbonyloxyC₁₋₆alkyl wherein the amino is optionally mono or di substituted with C₁₋₆alkyl,
   (r) di C₁₋₃alkylamino C₁₋₆alkyl wherein the amino is optionally mono or di substituted with C₁₋₆alkyl,
   (s) pyrazolidinyl,
   (t) piperidinyl
   (u) pyrrolidinyl,
   (v) pyrimidinyl,
   (w) C₃₋₁₀cycloalkyl,
   (x) α-C₁₋₃alkyl benzyl or mono or di substituted benzyl or mono or di substituted pyridylmethyl, wherein the substitutents are X₁ and X₂,
wherein
X₁ is
   (1) hydrogen,
   (2) halo,
   (3) C₁₋₆alkyl,
   (4) halo-C₁₋₆alkyl,
   (5) C₂₋₆alkenyl,
   (6) hydroxy-C₁₋₆alkyl,
   (7) C₁₋₆alkylcarbonyl,
   (8) C₁₋₆alkylcarbonylamino;
   (9) di-C₁₋₃alkylamino; or
   (10) carboxy,
X₂ is hydrogen, halo or C₁₋₆alkyl;
n is 1, 2, 3 or 4;
R₉ is selected from hydrogen, C₁₋₄ alkyl, and C₁₋₃alkoxyC₁₋₃alkyl;
R₁₀ and R₁₁ are each independently selected from hydrogen, C₁₋₄alkyl, and C₁₋₃alkoxy C₁₋₃alkyl; or
R₇ and R₈ are joined together to form mono or di substituted ring selected from
(1) piperidinyl,
(2) piperazinyl,
(3) morpholinyl,
(4) pyrroylidinyl,
(5) pyrryl,
(6) imidazolyl,
(7) triazolyl,
(8) tetrazolyl,
(9) pyrazolidinyl,
(10) azetidinyl,
wherein the substituents are each selected from the group consisting of hydrogen and C₁₋₃alkyl, benzyloxycarbonyl, carboxy, phenyl C₁₋₃alkyl amino carbonyl, pyrrolidinyl, methyl, hydroxy C₁₋₃alkyl, C₁₋₆alkyloxy, C₁₋₄alkyloxy carbonyl, and oxo; or
R₈ and R₉ are joined together to form a mono or di substituted saturated monocyclic ring of 6 to 7 atoms and having two hetero atoms which are the nitrogens to which R₈ and R₉ are attached; said rings to include piperazinyl and homopiperazinyl; or R₉ and R₁₀ are joined together to form a mono or di substituted monocyclic saturated ring of 5 to 7 atoms and having one hetero atom which is the nitrogen to which R₉ is attached; or R₉ and R₁₂ are joined together to form a mono or di substituted monocyclic saturated ring of 5, 6 or 7 atoms, said ring having one hetero atom which is the nitrogen to which R₉ is attached; or R₁₀ and R₁₂ are joined together to form a mono or di substituted saturated monocyclic ring of 5, 6 or 7 carbon atoms; or R₈ and R₁₁ are joined together to form a mono or di substituted saturated monocyclic ring of 5, 6 or 7 atoms, said ring having one hetero atom which is the nitrogen atom to which R₈ is attached; and the substituents are independently selected from hydrogen and C₁₋₃ alkyl.

In one subclass, the invention concerns compounds of Formula I
wherein
R is C₁₋₃alkyl;
R₁ is C₁₋₃alkyl;
M is
   (a) C₁₋₆alkyl, or
   (b) C₂₋₆alkenyl;
R² is
   (a) hydrogen,
   (b) C₁₋₆alkyl, or C₁₋₆alkoxy, and
R³ is hydrogen, or
R² and R³ are joined together to form a methylenedioxy group or a furan ring;
R₇ and R₈ are each independently selected from
   (a) hydrogen,
   (b) C₁₋₃alkyl,
   (c) C₁₋₃alkoxy C₂₋₃alkyl,
   (d) C₃₋₇cycloalkyl,
   (e) hydroxyC₂₋₃alkyl,
   (d) carbo C₁₋₄alkyloxymethyl,
   (g) substituted benzyl wherein the substituents are X₁ and X₂
   wherein X₁ is hydrogen and X₂ is
   (1) hydrogen,
   (2) halo, or
   (3) C₁₋₃alkyl;
   n is 1, 2 or 3, and
R₉, R₁₀ and R₁₁ are each independently selected from hydrogen, C₁₋₄alkyl, and C₁₋₃alkoxy C₁₋₃alkyl; or
R₇ and R₈ are joined together to form a substituted ring selected from
   (a) piperidinyl,
   (b) piperazinyl, and
   (c) morpholinyl;
or
R₈ and R₉ are joined together to form a mono or di substituted saturated monocyclic ring of 6 to 7 atoms and having two hetero atoms which are the nitrogen atoms to which R₈ and R₉ are attached.

In a narrower sub-class are the compounds wherein
Q is a covalent bond;
R is methyl or ethyl;
R₁ is methyl or ethyl;
M is
   (a) C₁₋₄alkyl, or
   (b) C₂₋₃alkenyl;
R² is
   (a) hydrogen,
   (b) C₁₋₃alkyl, or C₁₋₃alkoxy, and
R³ is hydrogen, or
R² and R³ are joined together to form a furan or dioxacyclopentane ring;
n is 1 or 2;
R₉ and R₁₀ are each independently selected from
   (a) C₁₋₃alkyl,
   (b) C₁₋₃alkoxy C₁₋₃alkyl,
   (c) hydrogen,
R₇ and R₈ are each independently selected from
   (a) C₁₋₃alkyl,
   (b) C₁₋₃alkoxy C₂₋₃alkyl,
   (c) hydrogen,
   (d) hydroxyethyl,
   (e) carboethoxymethyl,
   (f) cyclopropyl,
or
or R₇ and R₈ are joined together to form a substituted ring selected from
   (a) piperidinyl, and
   (b) morpholinyl, or
R₈ and R₉ are joined together to form a piperazine ring.

As is defined above, various rings are formed when R8, R9, R10 and R12 are joined. The following is a non-limiting description of some of the preferred rings that are formed when these various substituents are joined.
R8 and R9 are joined 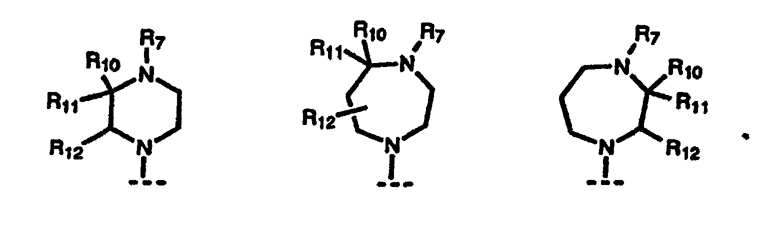
R9 and R10 are joined 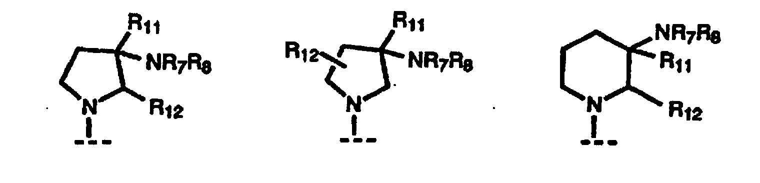 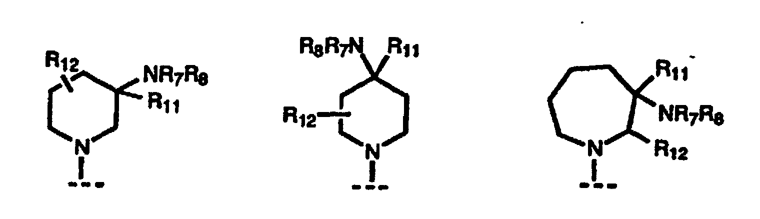 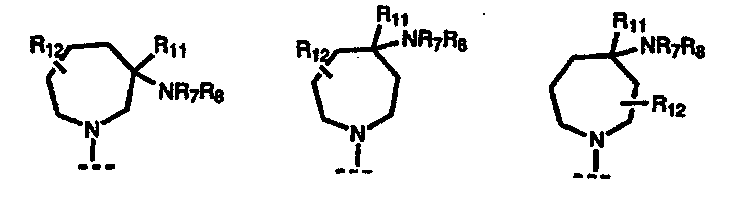
R9 and R12 are joined 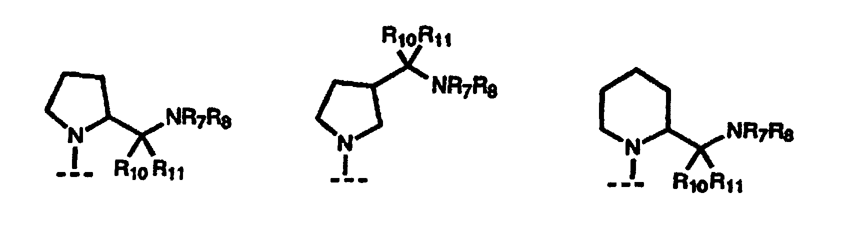 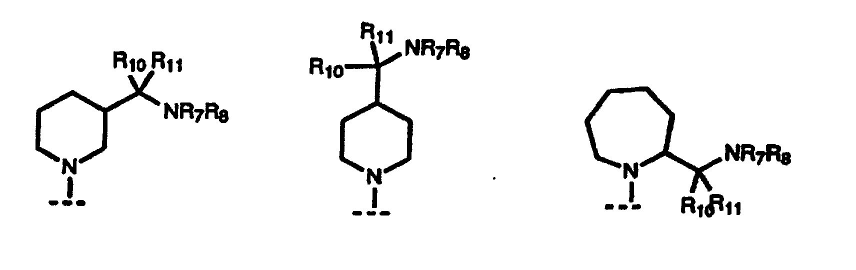 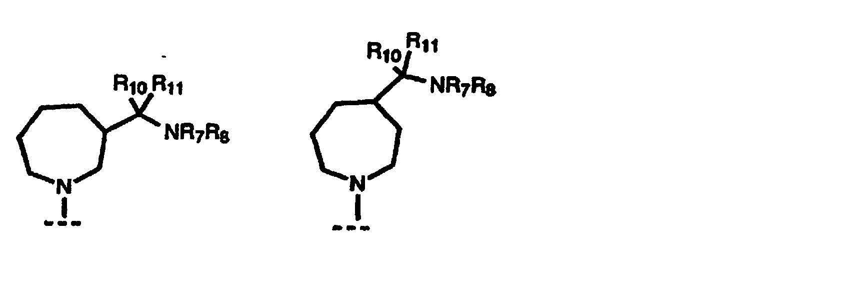
R10 and R12 are joined 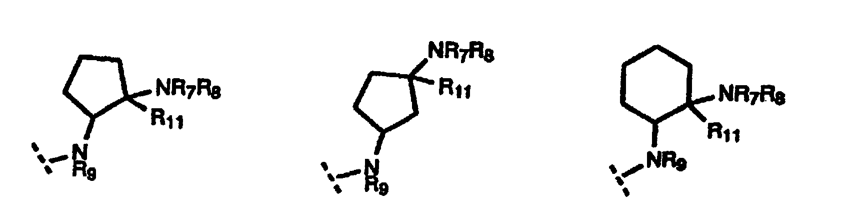 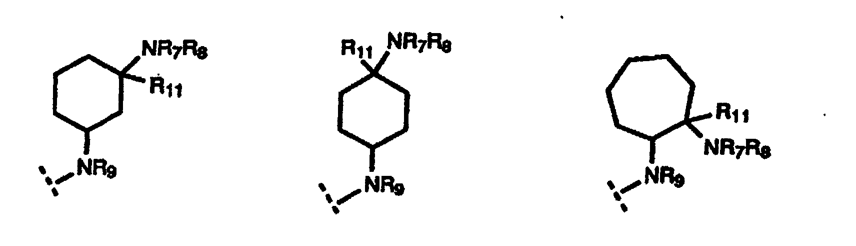 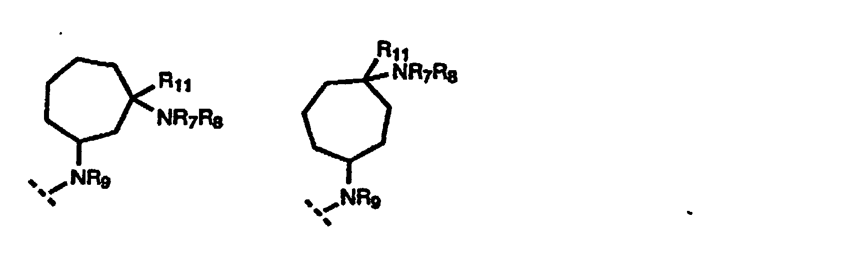
R10 and R12 are joined

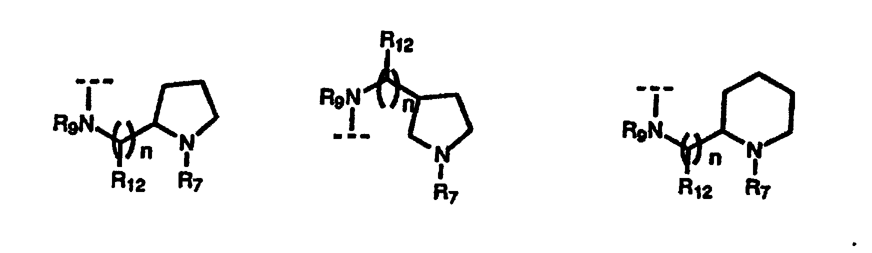

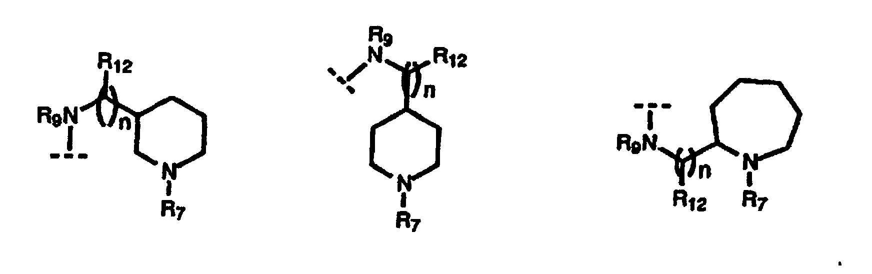

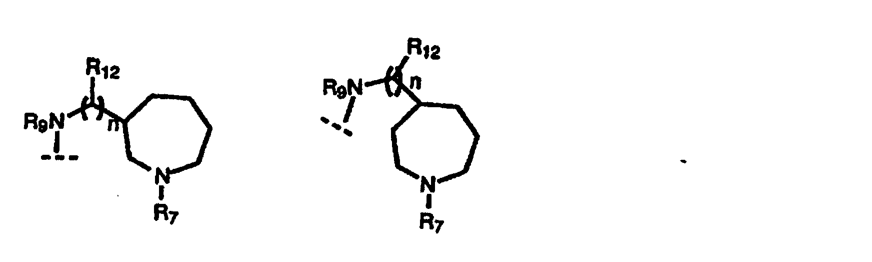

In another aspect the present invention is directed to the treatment of leukemia, such as nonlymphoblastic leukemias, acute myelogenous leukemia (FAB M1 and FAB M2), acute promyelocytic leukemia (FAB M3), acute myelomonocytic leukemia (FAB M4), acute monocytic leukemia (FAB M5), erythroleukemia, chronic myelogenous leukemia, chronic myelomonocytic leukemia, chronic monocytic leukemia and conditions associated with leukemia involving activity of PMN neutral proteases e.g. disseminated intravascular coagulation with compounds of Formula I.

Treatment of leukemia cells comprises: administration of a therapeutically effective amount of a compound of Formula I results in the inhibition of proteinase 3/myeloblastin, inhibition of elastase, inhibition of proliferation of the leukemia cells, induction of differentiation of the leukemia cells, and remission of the disease state.

In one alternative embodiment there is disclosed a method of treating leukemia comprising: administration to a patient in need of such treatment of a therapeutically effective amount of compound of Formula I.

In a second alternative embodiment there is disclosed a method of inhibiting proteinase 3/myeloblastin, comprising: administration to a patient in need of such inhibition of a therapeutically effective amount of compound of Formula I as defined above.

In a third alternative embodiment there is disclosed a method of inhibiting proteinase 3/myeloblastin and elastase, comprising: administration to a patient in need of such inhibition of a therapeutically effective amount of compound of Formula I as or a pharmaceutically acceptable salt thereof as defined above.

In a fourth embodiment there is disclosed a method of inducing cellular differentiation in leukemia cells comprising:
administration to a patient in need of such inhibition of a therapeutically effective amount of compound of Formula I or a pharmaceutically acceptable salt thereof as defined above.

Each of the above alternative embodiments (i.e., those relating to PR3 or cancer), also concerns co-administration of a compound of Formula I as defined above, with an agent or agents known in the art for treatment of leukemia, including, but not limited to epsilon-aminocaproic acid, heparin, trasylol (aprotinin); prednisolone; cytosine-arabinoside; β-mercaptopurine; cytarabine; an anthracycline (see Young et. al. (1981) N. Engl. J. Med. 305:139) such as daunorubicin, doxorubicin and epidoxorubicin; Vitamin A derivatives including retinoids and all-trans-retinoic acid (See Ellison R.R. et.al. (1968) Blood 32:507, Arabinosyl Cytosine: A useful agent in the treatment of leukemia in adults; Cytarabine: Therapeutic new dimensions, Semin. Oncol. 12:1 (1985, supp 3); Weinstein H.J. et. al. (1983) Blood 62:315, Chemotherapy for acute myelogenous leukemia in children and adults results in an enhanced therapeutic response.

Accordingly, in a fifth alternative embodiment the invention concerns a pharmaceutical composition comprising:
a pharmaceutical carrier, a therapeutically effective amount of compound selected from the group consisting of epsilon-aminocaproic acid, heparin, trasylol, prednisolone, cytosine arabinoside, β-mercaptopurine, cytarabine, an anthracycline and a vitamin A derivative; and a therapeutically effective amount of compound of Formula I as defined above.

In a sixth alternative embodiment the invention concerns a method of treating leukemia comprising:
co-administration to a patient in need of such treatment of a therepeutically effective amount of compound selected from the group consisting of epsilon-aminocaproic acid, heparin, trasylol, prednisolone, cytosine arabinoside, b-mercaptopurine, cytarabine, an anthracycline, and a vitamin A derivative; and a therapeutically effective amount of compound of Formula I as defined above.

In a seventh alternative embodiment the invention concerns a method of inhibiting proteinase 3/myeloblastin, comprising:
co-administration to a patient in need of such inhibition of a therapeutically effective amount of compound selected from the group consisting of epsilon-aminocaproic acid, heparin, trasylol, prednisolone, cytosine arabinoside, β-mercaptopurine, cytarabine, an anthracycline, and a vitamin A derivative; and a therapeutically effective amount of compound of Formula I as defined above.

In an eighth alternative embodiment the invention concerns a method of inhibiting proteinase 3/myeloblastin and elastase, comprising:
administration to a patient in need of such inhibition of a therapeutically effective amount of compound selected from the group consisting of epsilon-aminocaproic acid, heparin, trasylol, prednisolone, cytosine arabinoside, β-mercaptopurine, cytarabine, an anthracycline, and a vitamin A derivative; and a therapeutically effective amount of compound of Formula I as defined above.

In a ninth alternative embodiment the invention concerns a method of inducing cell differentiation in leukemia cells comprising:
administration to a patient in need of such inducing of a therapeutically effective amount of compound selected from the group consisting of epsilon-aminocaproic acid, heparin, trasylol, prednisolone, cytosine arabinoside, β-mercaptopurine, cytarabine, an anthracycline and a vitamin A derivative; and a therapeutically effective amount of compound of Formula I as defined above.

In a tenth alternative embodiment of the invention the instant compounds can also be used in the treatment of diseases associated with over-expression of cDNa, such as those pulmonary diseases is with abnormal, viscous, or inspissated purulent secretions. Such conditions are found in acute or chronic bronchopulmonary disease including infectious pneumonia, bronchitis or tracheobronchitis, bronchiectasis, cystic fibrosis, asthma, tuberculosis or fungal infections. Utility is also found in atelactasis due to tracheal or btonchial impaction and complications of tracheostomy.

In addition, the instant compounds can be co-administered with cDNase which also finds utility in these pulmonary diseases, and which is described in WO 90/07572.

The compounds of the invention are prepared by known methods or are prepared among other methods by the following representative schemes. For example, methods for making such compounds are disclosed in EP 0 337 549, published October 18, 1989, which is hereby incorporated by reference.

This invention also relates to a method of treating inflammation in patients using a compound of Formula (I), particularly a preferred compound as the active constituent.

It has been found that the following compounds are effective inhibitors of the proteolytic function of human neutrophil elastase as shown below in Table 4 to 10.

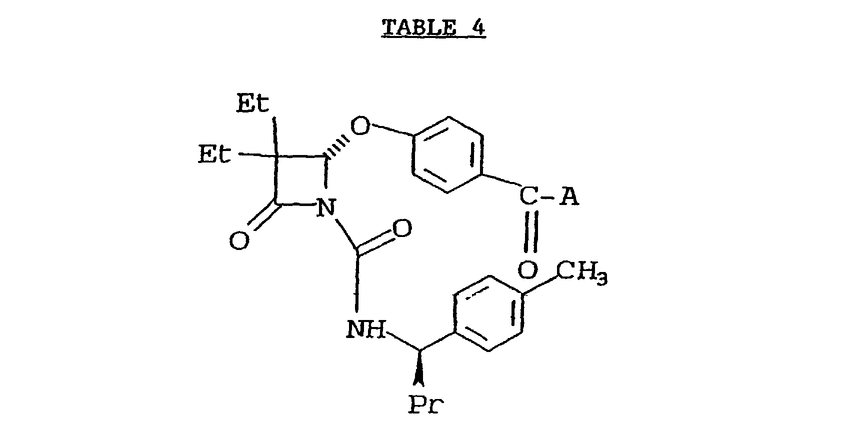

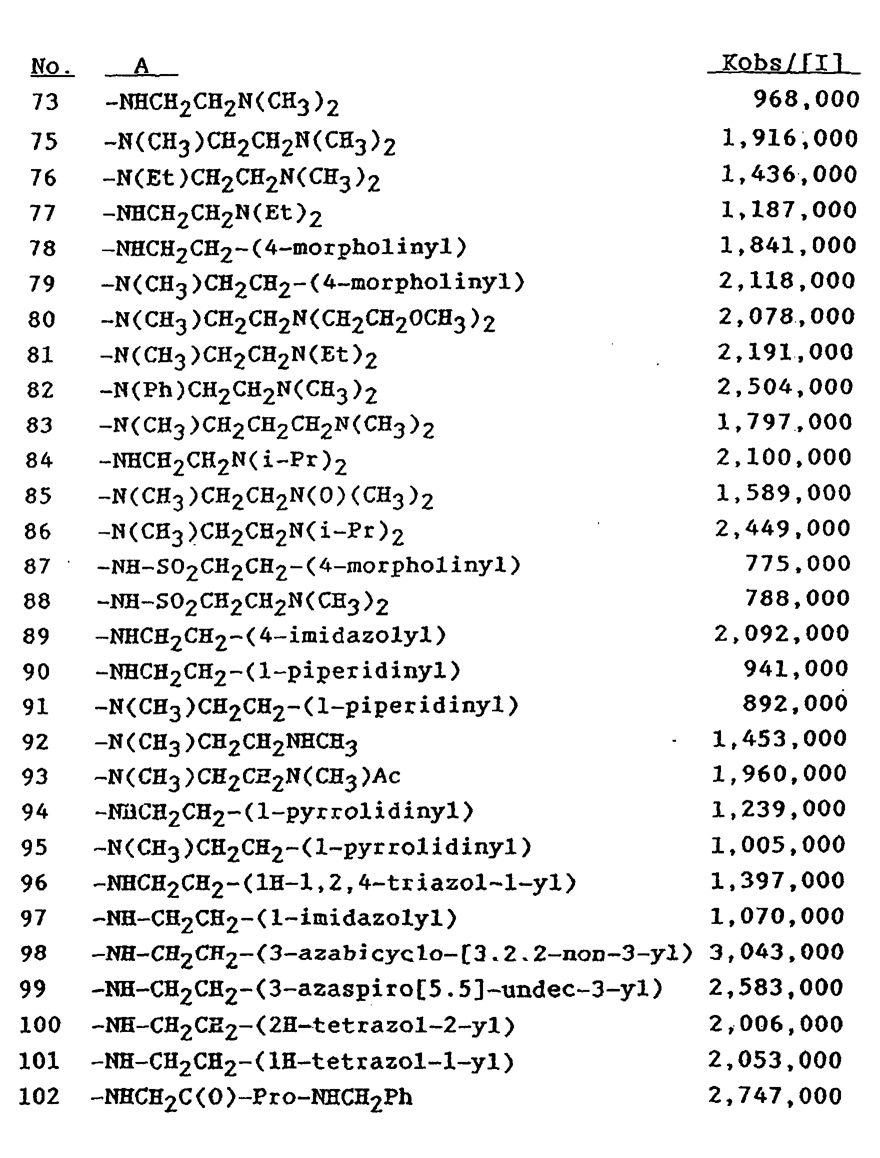

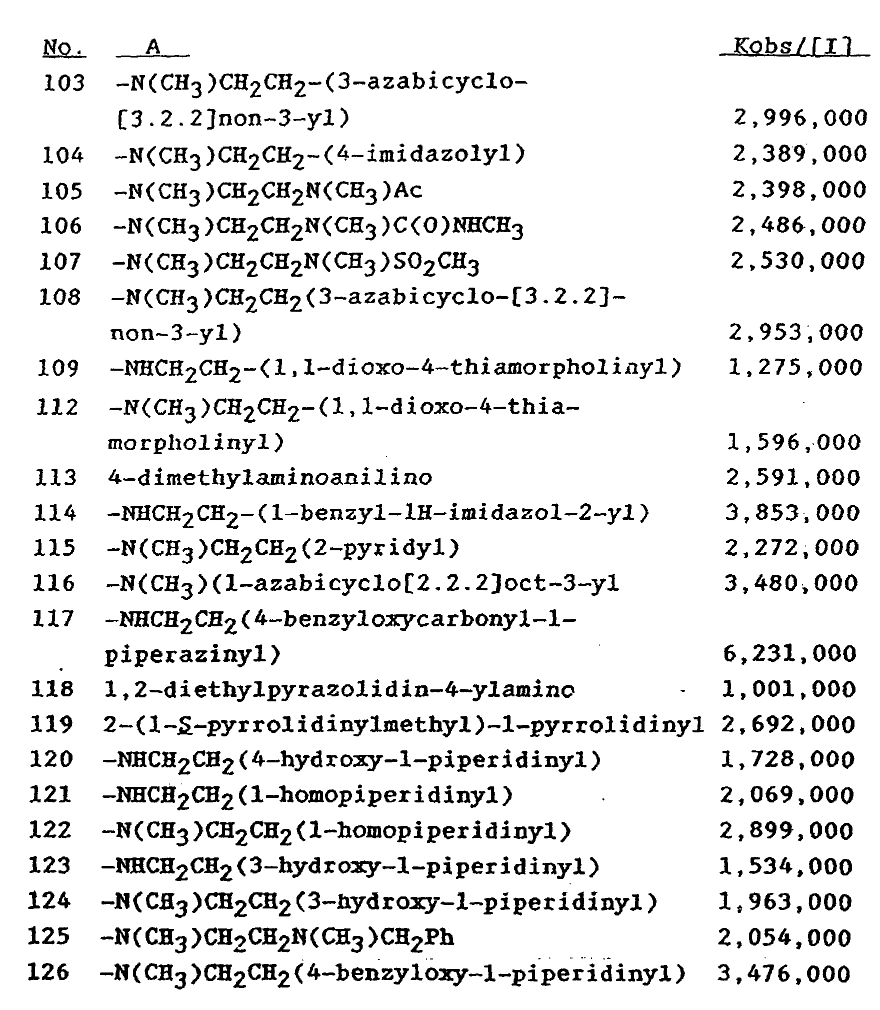

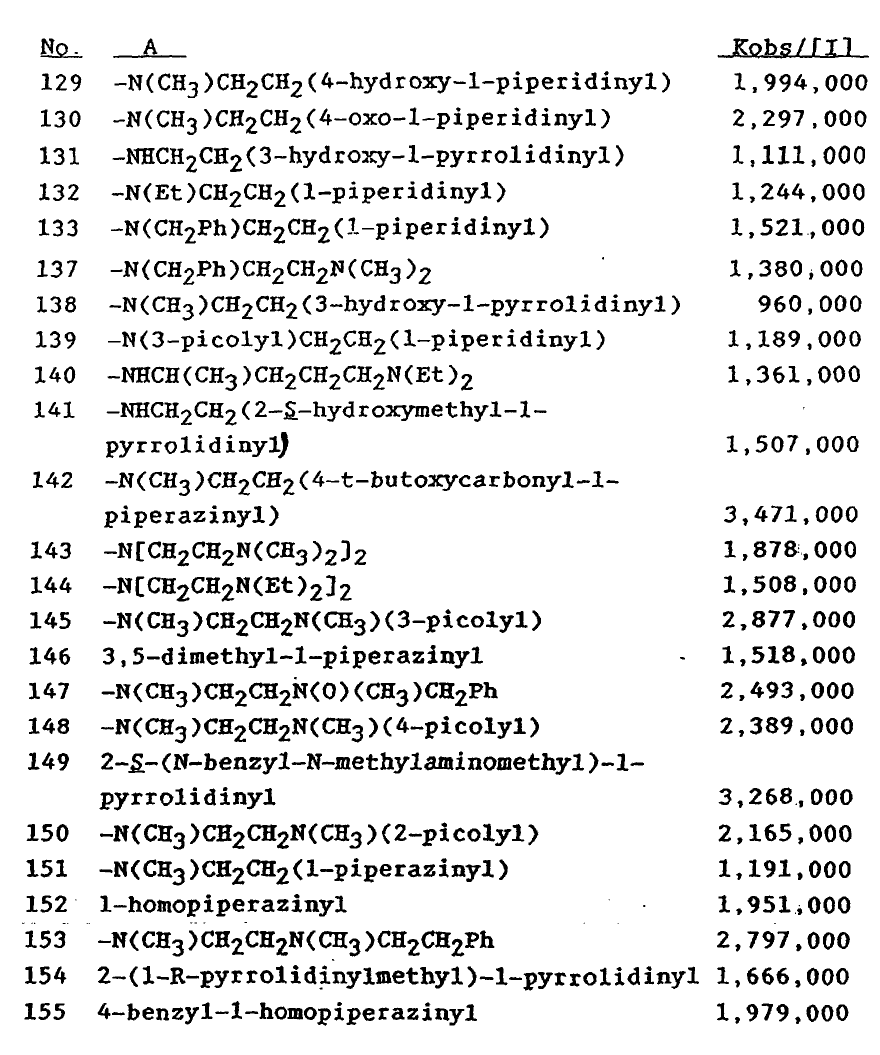

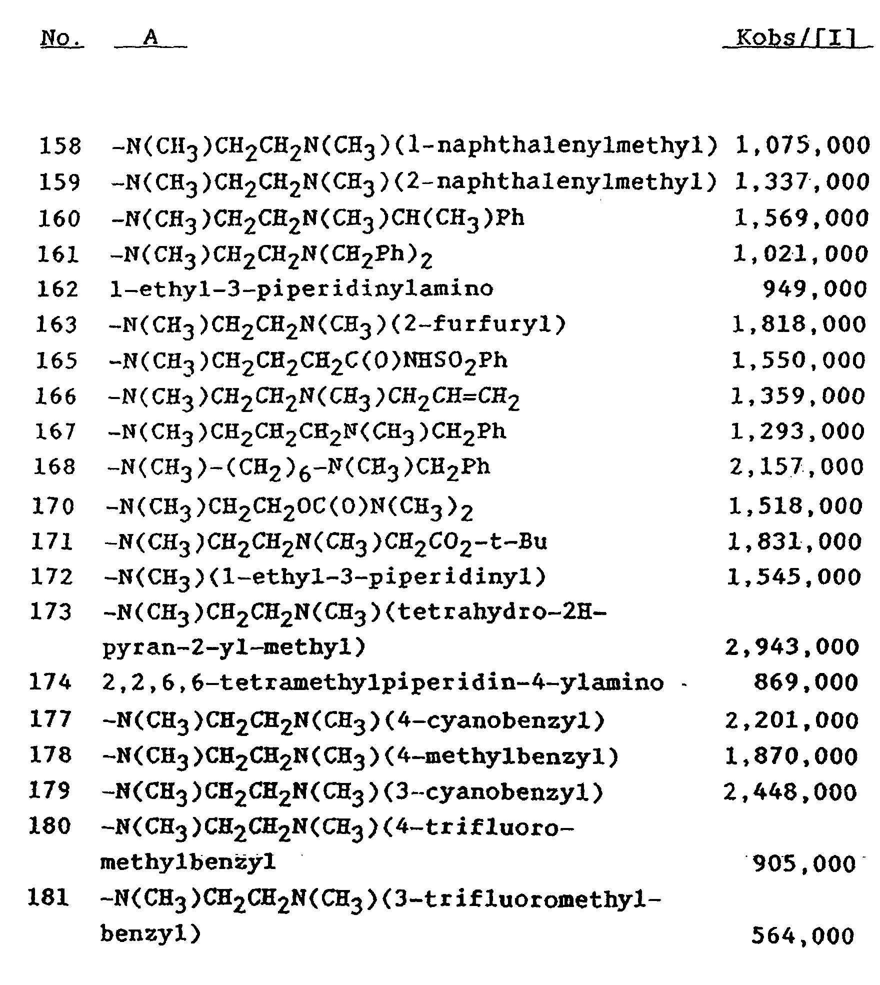

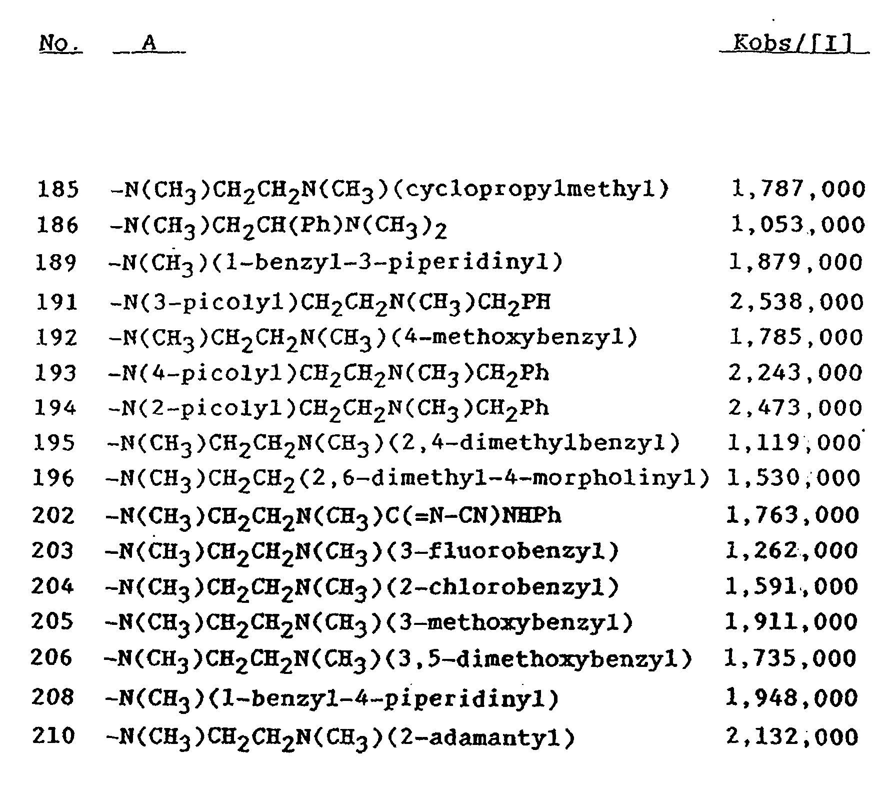

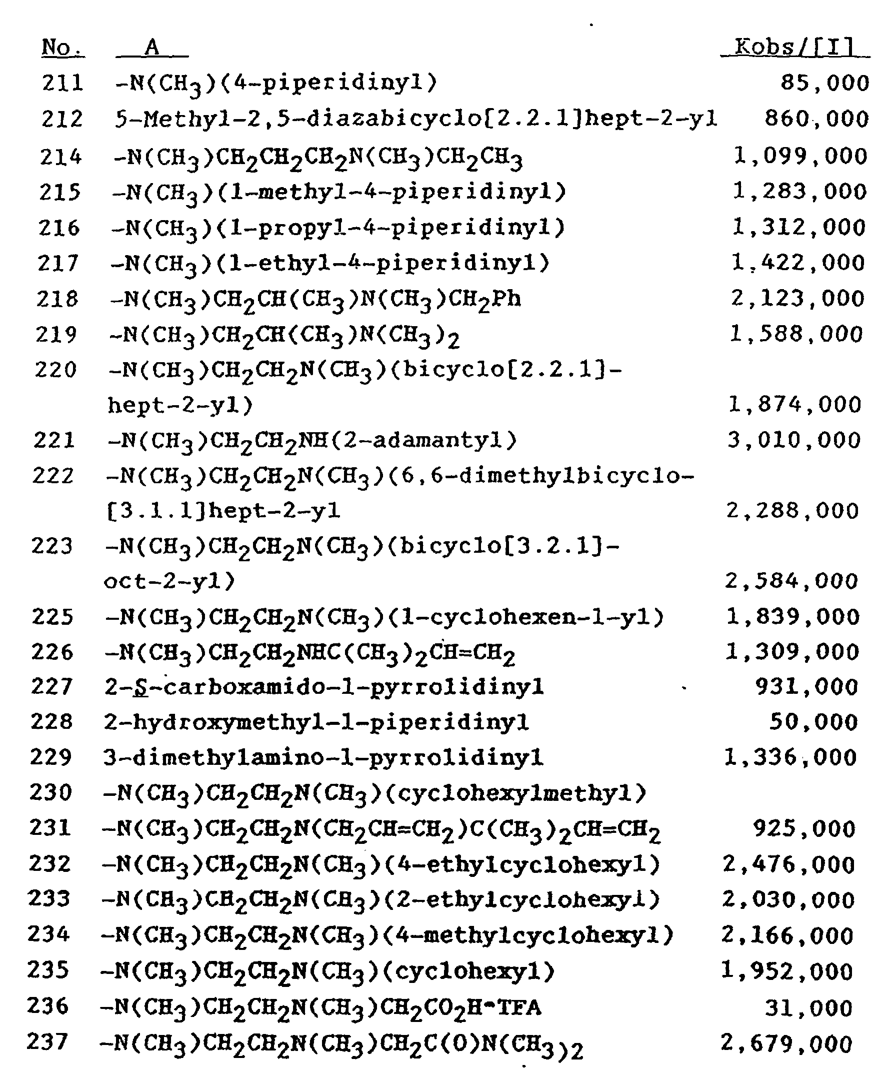

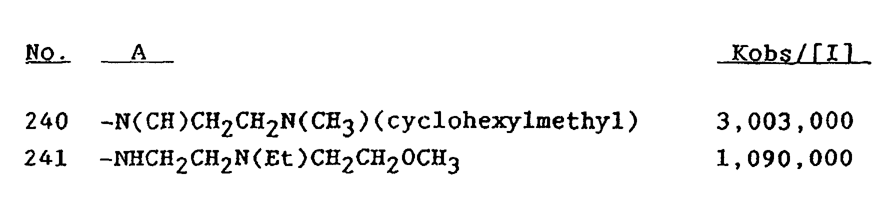

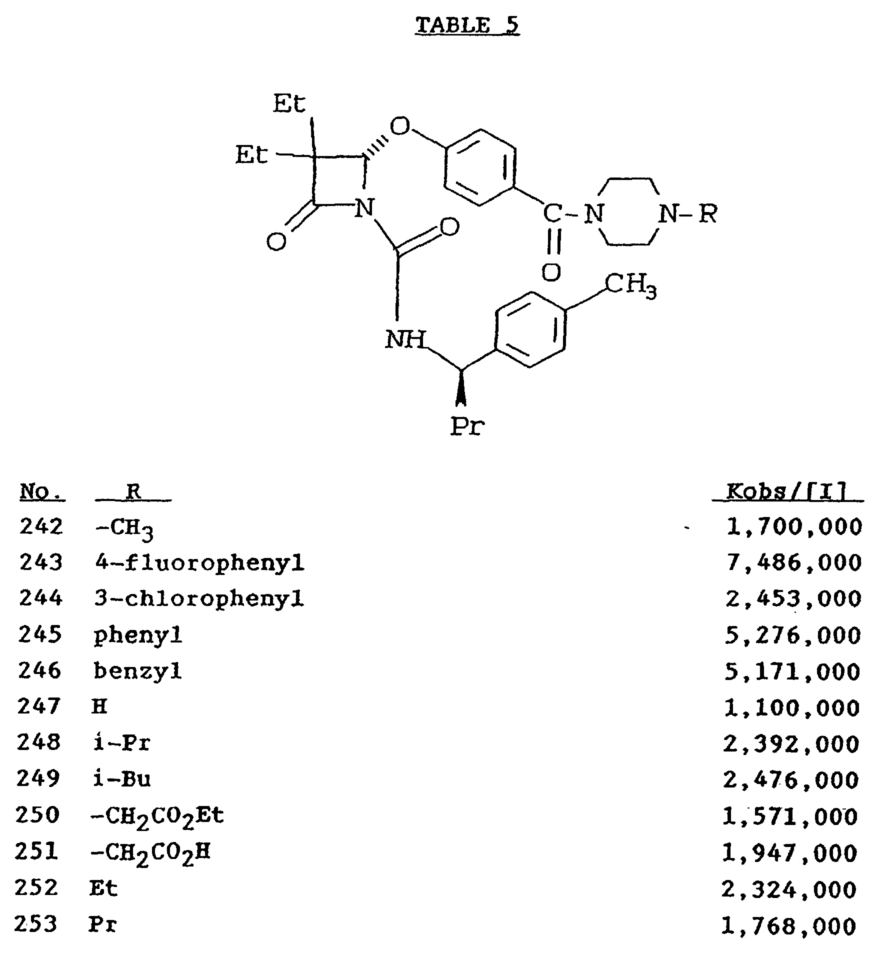

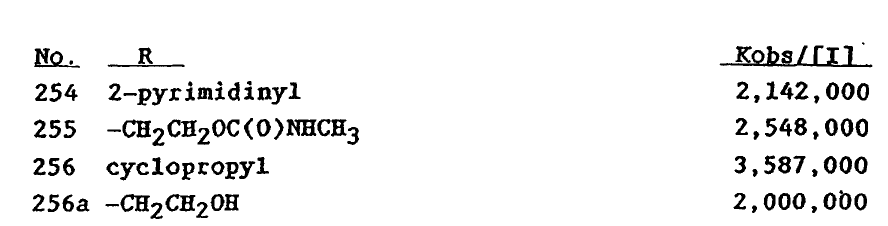

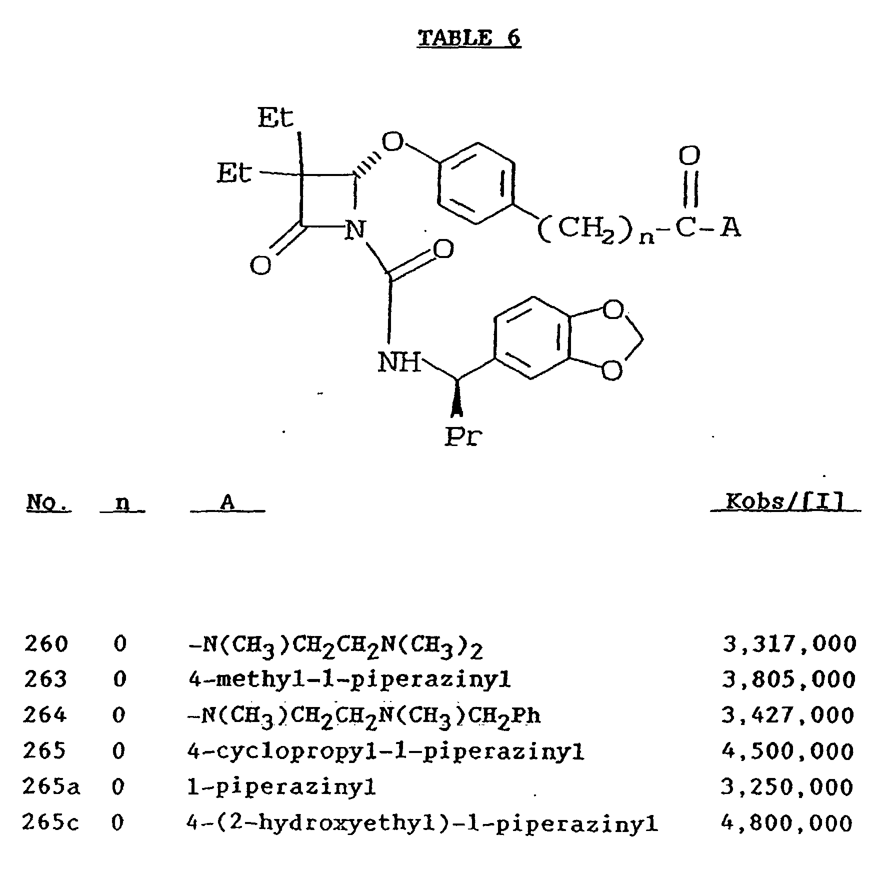

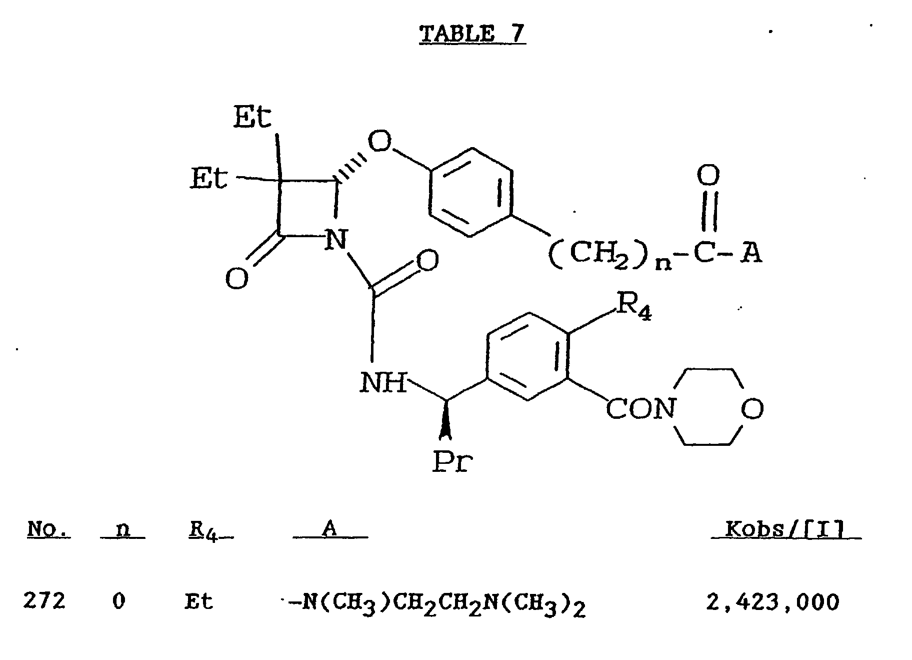

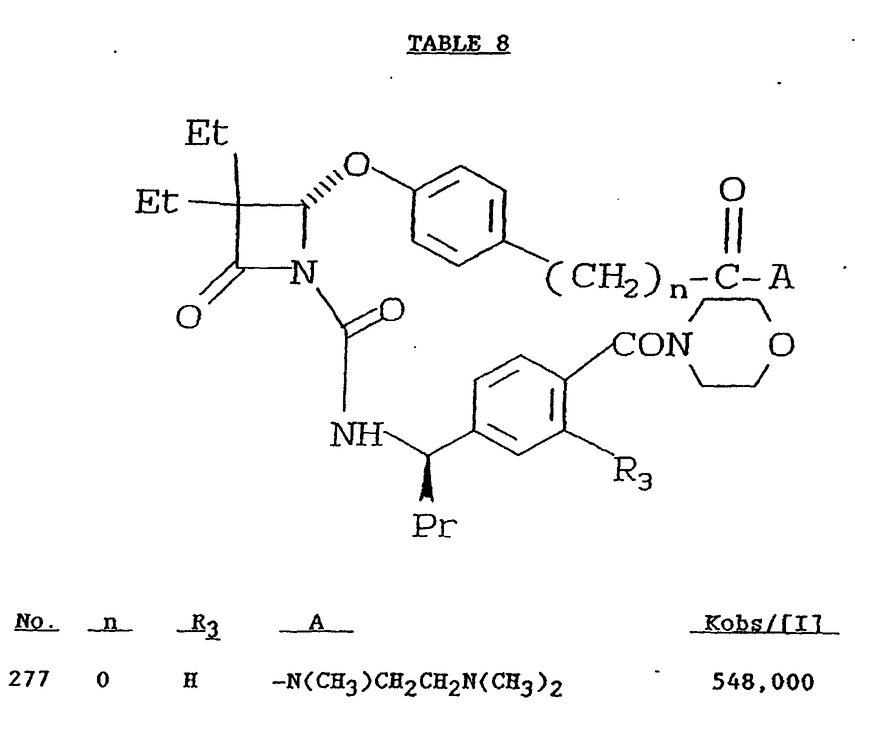

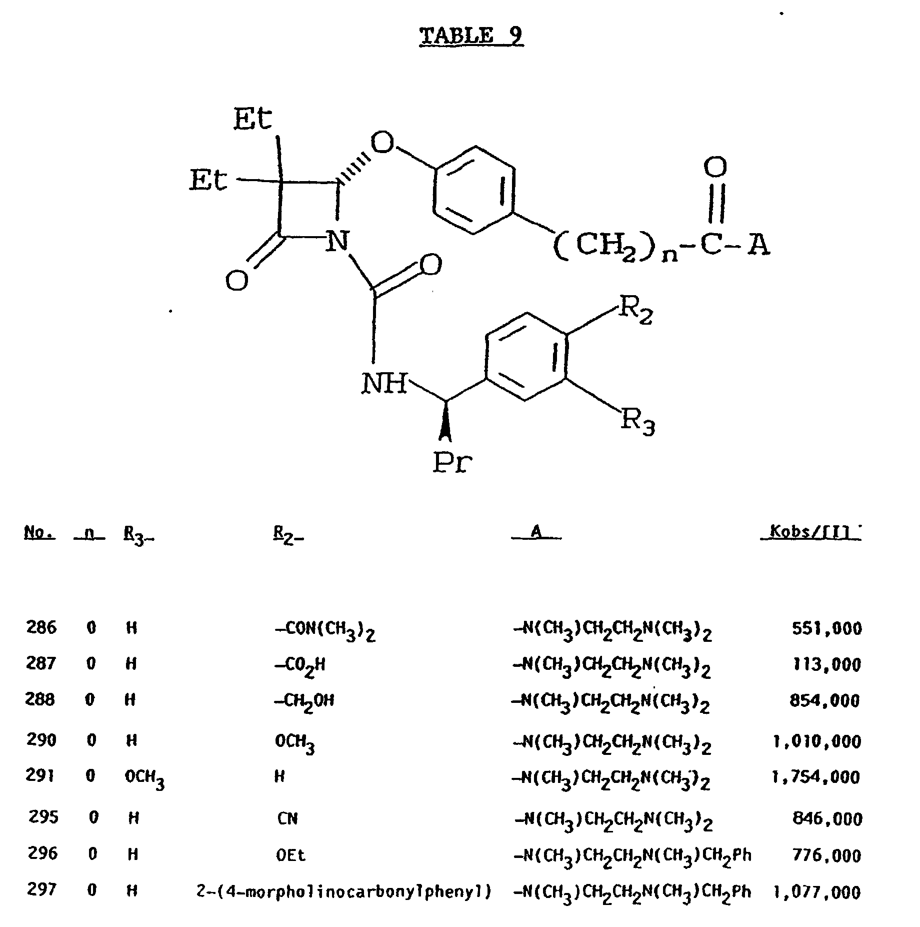

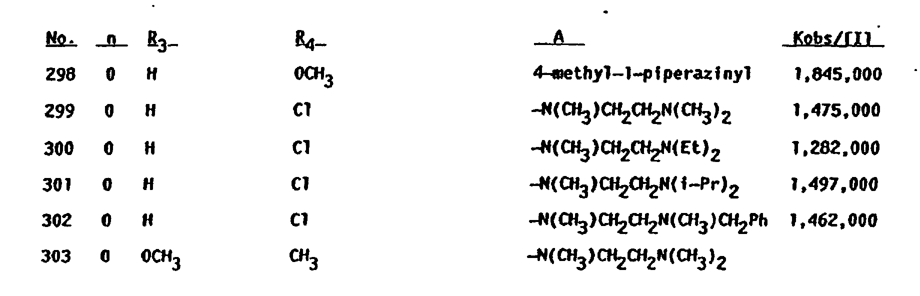

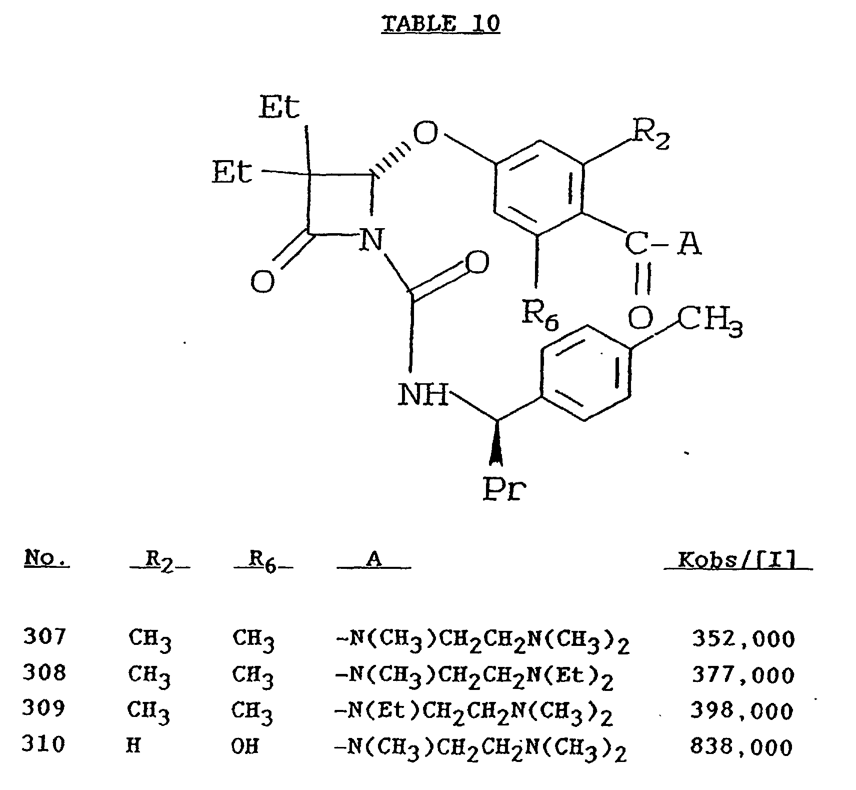

### Enzyme Assays for the Inhibition of Human Polymorphonuclear Leukocyte Elastase Via Hydrolysis of N-t-Boc-alanyl-alanyl-prolylalanine-p-nitroanilide (Boc-AAPAN) or N-t-Boc-alanyl-prolylvaline-p-nitro-anilide (Boc-AAPVN) Reagent:

0.05M TES (N-tris[hydroxymethyl]methyl-2-mino-ethanesulfonic acid) Buffer, pH 7.5.

0.2 mM Boc-AAPAN or Boc-AAPVN.

To prepare substrate, the solid was first dissolved in 10.0 ml DMSO. Buffer at pH 7.5 was then added to a final volume of 100 ml.

Crude extract of human polymorphonuclear leukocytes (PMN) containing elastase activity.

Inhibitors (azetidinones) to be tested dissolved in DMSO just before use.

To 1.0 ml of 0.2 mM Boc-AAPAN in a cuvette, 0.01-0.1 ml of DMSO with or without inhibitor was added. After mixing, a measurement was taken at 410 mµ to detect any spontaneous hydrolysis due to presence of test compound. 0.05 Milliliters of PMN extract was then added and the ΔOD/min at 410 mµ was measured and recorded. Beckman model 35 spectrophotometer was used.

Results were expressed to the nearest thousand ^{k}obs/I which is the second order rate constant in per mole per second for inactivation of the enzyme.

The elastase activity in the crude PMN extract may vary from one preparation to another. A control of each new batch is run, and the volume added in the assay procedure is adjusted according to activity.

This invention also relates to a method of treating inflammation in patients using a compound of Formula (I), particularly a preferred compound as the active constituent.

It has been found that the compounds of Formula (I) are effective inhibitors of the proteolytic function of human neutrophil elastase.

Accordingly, the compounds of Formula (I), can be used to reduce inflammation and/or relieve pain in diseases such as emphysema, rheumatoid arthritis, osteoarthritis, gout, bronchial inflammation, chronic or acute bronchitis, cystic fibrosis, adult respiratory distress syndrome, atherosclerosis, sepsis, septicemia, shock, periodontitis, glomerular nephritis or nephosis, myocardial infarction, reperfusion injury, infectious arthritis, rheumatic fever and the like, and may reduce hemorrhage in acute promyelocytic leukemia and the like.

In this capacity, and as appreciated by those of skill in the art, therapy comprising administration of compounds of Formula I may actually include co-administration of one or more additional active agents. Classes of active agents include, but are not limited to β₂-adrenergic agonists; anti-cholinergic agents; steroids; non-steroidal anti-inflammatory agents (NSAID's); mucolytic agents; most all stabilizers; and antibacterials.

For purposes of this specification, β₂-adrenergic agonists are intended to include, but are not limited to, metaproterenol, terbutaline, isoetharine, albuterol, and ritodrine, carbuterol, fenoterol, quinterenol, rimiterol, salmefamol, soterenol, and tretoquinol.

For purposes of this specification, anti-cholinergic agents are intended to include, but are not limited to, atropine, and iptratropium-bromide.

For purposes of this specification, mucolytic agents are intened to include, but are not limited to acetylcysteine and guattenesin.

For purposes of this specification, steroids are intended to include, but are not limited to, prednisone, beclomethasone, budesonide, solumedrol, triamcinolone, and methyl-prednisolone.

For purposes of this specification most cell stabilizers are intended to include, but are not limited to cromolyn and ketotafin.

For purposes of this specification, non-steroidal anti-inflammatory agents are intended to include, but are not limited to aspirin, diflunisal, naphthylsalicylate, phenylbutazolone, oxyphenbutazolone, indomethacin, sulindac, mefenamic acid, meclofenamate sodium, tolmetin, ibuprofen, naproxen, fenoprofen and piroxicam.

For the purposes of this specification, antibacterial agents are intended to include the broad classes of penicillins, cephalosporins and other beta-lactams, aminoglycosides, quinolones, macrolides, tetracyclines, sulfonamides, lincosamides and polymyxins. The penicillins, in turn, are intended to include, but are not limited to penicillin G, penicillin V, methicillin, nafcillin, oxacillin, cloxacillin, dicloxacillin, floxacillin, ampicillin, ampicillin/sulbactam, amoxicillin, amoxicillin/clavulanate, hetacillin, cyclacillin, bacampicillin, carbenicillin, carbenicillin indanyl, ticarcillin, ticarcillin/clavulanate, azlocillin, mezlocillin, peperacillin, and mecillinam. The cephalosporins and other beta-lactams are intended to include, but are not limited to cephalothin, cephapirin, cephalexin, cephradine, cefazolin, cefadroxil, cefaclor; cefamandole, cefotetan, cefoxitin, ceruroxime, cefonicid, ceforadine, cefixime, cefotaxime, moxalactam, ceftizoxime, cetriaxome, ceftizoxime, cetriaxone, cephoperazone, ceftazidime, imipenem and aztreonam. The aminoglycosides are intended to include, but are not limited to streptomycin, gentamicin, tobramycin, amikacin, netilmicin, kanamycin and neomycin. The quinolones are intended to include, but are not limited to nalidixic acid, norfloxacin, enoxacin, ciprofloxacin, ofloxacin, sparfloxacin and temafloxacin. The macrolides are intended to include, but are not limited to erythomycin, spiramycin and azithromycin. The tetracyclines are intended to include, but are not limited to doxycycline, minocycline and tetracycline. The sulfonamides are intended to include, but are not limited to sulfanilamide, sulfamethoxazole, sulfacetamide, sulfadiazine, sulfisoxazole and co-trimoxazole (trimethoprim/sulfamethoxazole). The lincosamides are intended to include, but are not limited to clindamycin and lincomycin. The polymyxins (polypeptides) are intended to include, but are not limited to polymyxin B and colistin.

Alternatively, compounds of Formula I are useful in the treatment of certain cancers including nonlymphoblastic leukemias, acute myelogenous leukemia (FAB M1 and FAB M2), acute promyelocytic leukemia (FAB M3), acute myelomonocytic leukemia (FAB M4), acute monocyte leukemia (FAB M5), erythroleukemia, chronic myelogenous leukemia, chronic myelomonocytic leukemia, chronic monocytic leukemia and conditions associated with leukemia involving activity of PMN neutral proteases e.g. disseminated intravascular coagulation.

Similarly, compounds of Formula I are useful for the inhibition of proteinase 3/myeloblastin, inhibition of elastase, inhibition of proliferation of leukemia cells, inducing differentiation of leukemia cells and remission of the disease state of leukemia.

Moreover, as described above, such treatment may optionally comprise the co-administration of an agent such as a compound selected from the group consisting of epsilon-aminocaproic acid, heparin, trasylol, prednisolone, cytosine arabinoside, b-mercaptopurine, cytarabine, an anthracycline and a vitamin A derivative such as retinoic acid.

For each of the uses, the compounds of Formula (I) and optional treatment agents, may be administered orally, topically, parenterally, by inhalation spray or rectally in dosage unit Formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

For treatment as described above the compounds of Formula (I) may be administered orally, topically, parenterally, by inhalation spray or rectally in dosage unit Formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol mono-oleate, or condensation products of ethylene oxide with partial esters derived from fatty acids and hexitol anhydrides, for example polyoxyethylene sorbitan monooleate. The said aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan mono-oleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution glucose in water and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the anti-inflammatory agents are employed.

The amount of active ingredient(s) that may be combined with the carrier materials to produce a single dosage form will vary depending upon the host treated and the particular mode of administration. For example, a formulation intended for the oral administration of humans may contain from 5 mg to 2000 mg or 5000 mg of each active agent(s) compounded with an appropriate and convenient amount of carrier material which may vary from about 5 to about 95 percent of the total composition. For purposes of this specification, this broad dosage range is specifically intended to include, but is not limited to, range of 5 mg to 2000 mg; 25 mg to 2000 mg; 5 mg to 1000 mg; 25 mg to 1000 mg; 5 mg to 500 mg; and 25 mg to 500 mg. Dosage unit forms will generally contain between from about 25 mg to about 500 mg of active ingredient(s).

Furthermore, it is also possible that most effective treatment may warrent administration of an initial dosage of one range (e.g. 1-5 mg of active agent per kg of patient weight) followed by administration of a second range (e.g. 0.1 to 1 mg of active agent per kg of pateint weight).

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following example illustrates the preparation of the compounds useful in the present invention, but does not limit the scope of the invention. Starting materials may be optionally prepared as disclosed in EPO 337 549 published October 18, 1989. Where appropriate, compounds may be produced and used in the form of pharmaceutically acceptable salts. For example, the basic coumpounds may be used in the form of a hydrochloride or mesylate or other acceptable salt. See Preformulation in Remington's Pharmaceutical Sciences, Mack Publishing, Easton PA.

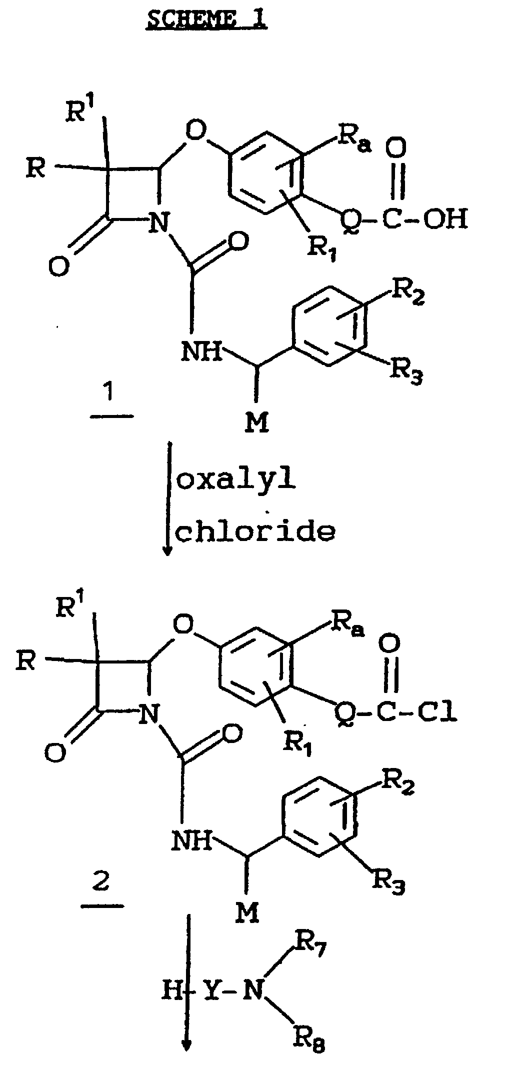

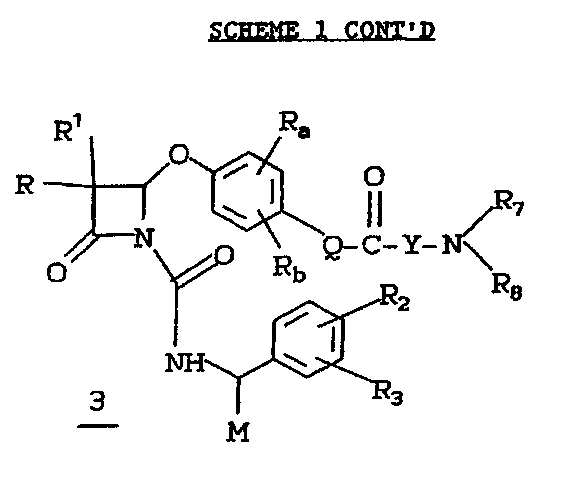

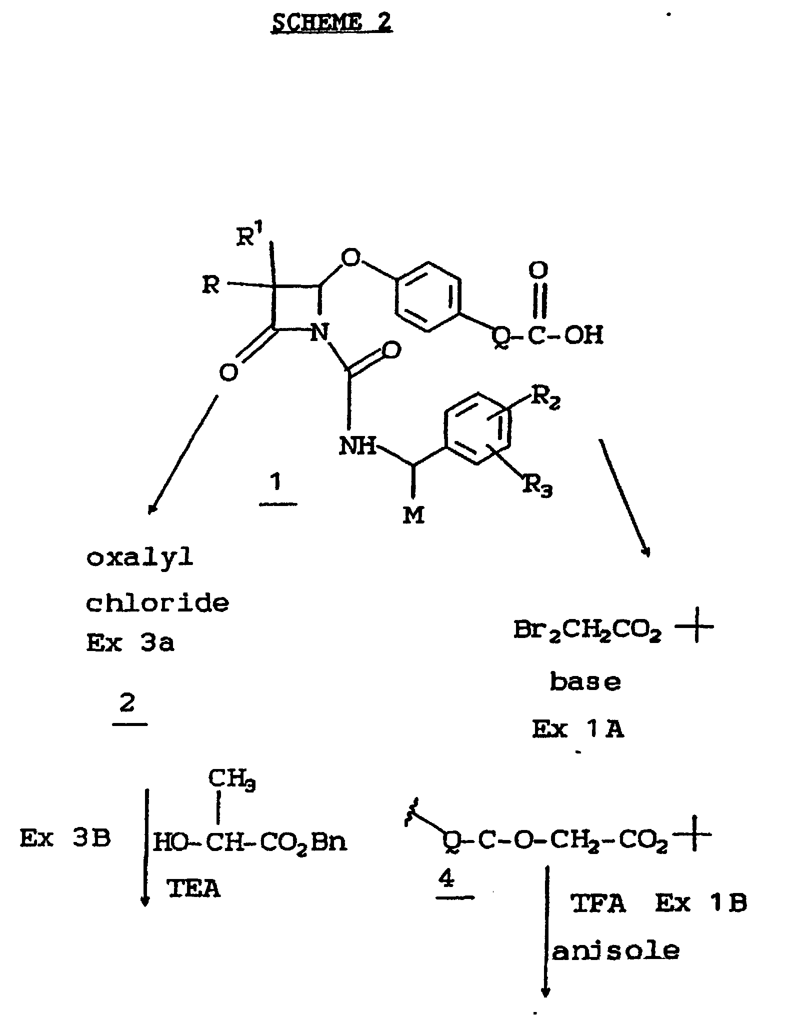

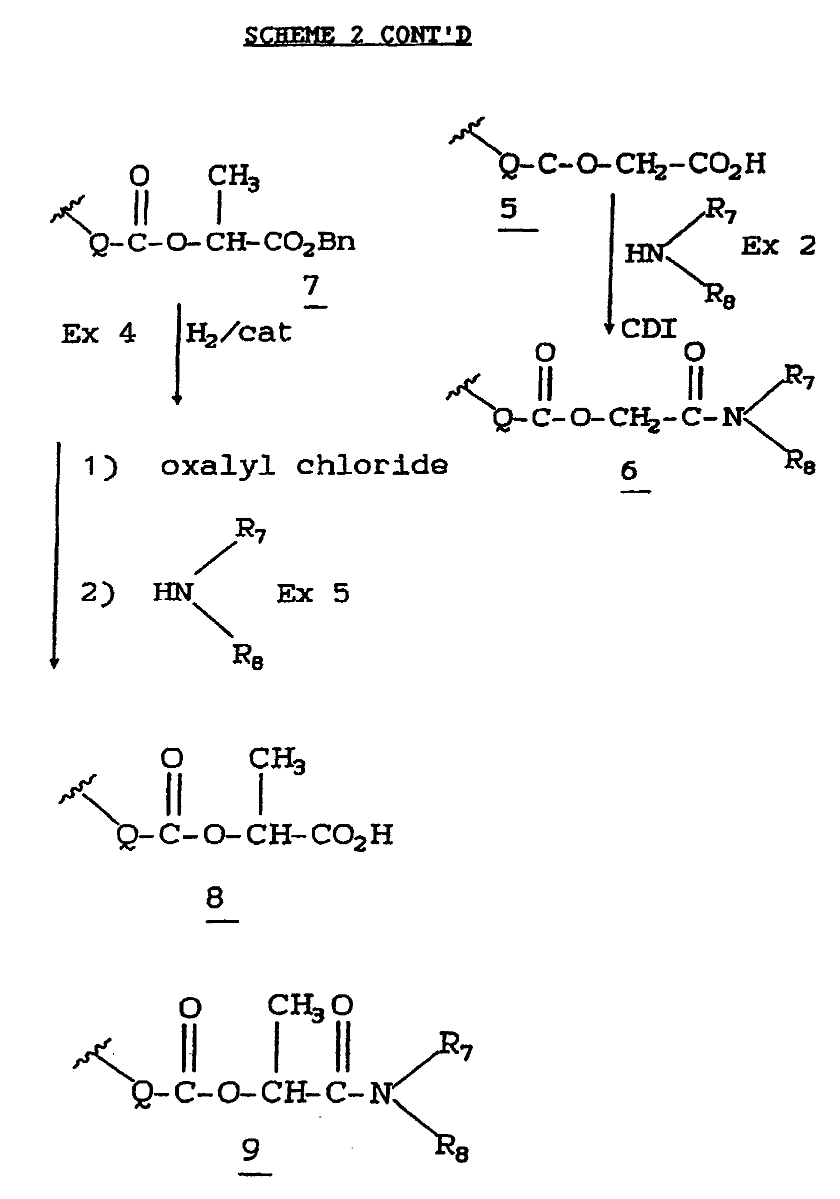

The glycolic acid derivatives described herein can be prepared according to the following scheme. The starting acid (as carboxylate anion) may be alkylated (Ex 1A) with a suitably protected α-halo acetic acid derivative to give the glycolate ester 4 which can be deprotected (Ex 1B) to the glycolic acid ester 5. Treatment of 5 with an amine utilizing a condensing agent such as dicyclohexylcarbodiimide or carbomyldiinidazole (Ex 2) affords the deserved amide 6. Alternately, the starting acid 1 may be converted to its acid chloride 2 (Ex 3A) and treated with a suitably protected α-hydroxyalkanoic acid (Ex 3B) in the presence of base to give the protected ester 7. Deprotection (Ex 4), followed by conversion to the acid chloride and treatment with the appropriate amine (Ex 5) affords the desired amide 9.

### Example 10

### [S-(R*,S*)] 2-[4-[[[2-(dimethylamino)-ethyl]amino]-carbonyl]phenoxy]-3,3-diethyl-N-[1-(4-methylphenyl)-butyl]-4-oxo-1-azetidinecarboxamide.

To a solution of 0.104 g carbonyldiimidazole in 2 ml methylene chloride is added a solution of 0.227 g of [S-(R*,S*)]-4-((3,3-diethyl-1-((-1-(4-methylphenyl)butylamino)carbonyl)-4-oxo-2-azetidinyl)-oxy)benzoic acid in 3 ml methylene chloride. The mixture is stirred at ambient temperature for 30 minutes at which time 0.100 g of N,N-dimethyl-ethylenediamine is added. After stirring overnight at room temperature the reaction mixture is poured into benzene (50 ml) and washed with water. The organic layer is separated, dried through sodium sulfate and concentrated in vacuo. Silica gel chromatography using 5% methanol in methylene chloride yields 0.160 g of 2-[4-[[[2-(dimethylamino)-ethyl]amino]carbonyl]phenoxy]-3,3-diethyl-N-[1-(4-methylphenyl)butyl]-4-oxo-1-azetidinecarboxamide.
(Compound 73).

| Analysis: C₃₀H₄₂N₄O₄+0.4H₂O | | | |
|---|---|---|---|
| Calc | C, 68.00; | H, 8.14; | N, 10.57. |
| Found | C, 68.01; | H, 8.18; | N, 10.62. |

### Example 11

### A. (S-(R*,S*))-4-((3,3-diethyl-1-((1-(4-methyl-phenyl)butylamino)catbonyl)-4-oxo-2-azetidinyl)-oxy)-benzoyl chloride.

To a solution of 0.150 g of [S-(R*,S*)] 4-((3,3-diethyl-1-(((1-(4-methylphenyl)butyl)amino)-carbonyl)-4-oxo-2-azetidinyl)oxy)benzoic acid in 5 ml methylene chloride containing a catalytic amount of dimethylformamide is added 0.5 ml of oxalyl chloride. The mixture is stirred at room temperature for 30 minutes and then concentrated in vacuo to yield (S-(R*,S*))-4-((3,3-diethyl-1-((1-(4-methyl-phenyl)-butylamino)carbonyl)-4-oxo-2-azetidinyl)oxy)-benzoyl chloride.

### B. [S-(R*,S*)]-2-[4-[[[2-(dimethylamino)ethyl]methyl-amino]carbonyl]-phenoxy]-3,3-diethyl-N-[1-(4-methylphenyl]butyl]-4-oxo-1-azetidinecarboxamide.

The above acid chloride is dissolved in 3 ml methylene chloride and a solution of 0.20 gm of N,N,N'-trimethylethylenediamine in 2 ml methylene chloride is added. The mixture is stirred overnight and then concentrated in vacuo. The residue is extracted between ethyl acetate and saturated sodium bicarbonate solution. The organic layer is dried through sodium sulfate and concentrated in vacuo. Silica gel chromatography of the residue (5% methanol in methylene chloride) affords 0.117 g of [S-(R*,S*)]-2-[4-[[[2-(dimethylamino)ethyl]methylamino]carbonyl]-phenoxy]-3,3-diethyl-N-[1-(4-methylphenyl]butyl]-4-oxo-1-azetidinecarboxamide. (Compound 75).

| Analysis: C₃₁H₄₄N₄O₄+0.5H₂O | | | |
|---|---|---|---|
| Calc | C, 68.23; | H, 8.31; | N, 10.27. |
| Found | C, 68.20; | H, 8.41; | N, 10.10. |

When N,N,N'-trimethylethylenediamine of Example 11b is replaced by the appropriate amines, there is obtained the corresponding amides.
1) Compound 76

| Analysis: C₃₂H₄₆N₄O₄ | | | |
|---|---|---|---|
| Calc | C, 69.79; | H, 8.42; | N, 10.17. |
| Found | C, 69.02; | H, 8.60; | N, 9.54. |

2) Compound 77

| Analysis: C₃₂H₄₆N₄O₄ +0.75 H₂O | | | |
|---|---|---|---|
| Calc | C, 68.12; | H, 8.49; | N, 9.93. |
| Found | C, 68.22; | H, 8.48; | N, 10.17. |

3) Compound 78

| Analysis: C₃₂H₄₄N₄O₅ | | | |
|---|---|---|---|
| Calc | C, 68.06; | H, 7.85; | N, 9.92. |
| Found | C, 67.84; | H, 8.07; | N, 9.62. |

4) Compound 79

| Analysis: C₃₃H₄₆N₄O₅ +H₂O | | | |
|---|---|---|---|
| Calc | C, 66.42; | H, 8.11; | N, 9.39. |
| Found | C, 66.73; | H, 8.19; | N, 9.23. |

5) Compound 80

| Analysis: C₃₅H₅₂N₄O₆ | | | |
|---|---|---|---|
| Calc | C, 67.28; | H, 8.39; | N, 8.97. |
| Found | C, 67.08; | H, 8.77; | N, 8.41. |

6) Compound 81

| Analysis: C₃₃H₄₈N₄O₄ +H₂O | | | |
|---|---|---|---|
| Calc | C, 68.01; | H, 8.65; | N, 9.61. |
| Found | C, 68.42; | H, 8.59; | N, 9.17. |

7) Compound 82

| Analysis: C₃₆H₄₆N₄O₄ +0.5H₂O | | | |
|---|---|---|---|
| Calc | C, 71.14; | H, 7.79; | N, 9.21. |
| Found | C, 71.41; | H, 7.68; | N, 9.10. |

8) Compound 83

| Analysis: C₃₂H₄₆N₄O₄ +1.2H₂O | | | |
|---|---|---|---|
| Calc | C, 67.15; | H, 8.52; | N, 9.79. |
| Found | C, 67.21; | H, 8.26; | N, 9.47. |

9) Compound 84
10) Compound 86

| Analysis: C₃₅H₅₂N₄O₄ | | | |
|---|---|---|---|
| Calc | C, 70.91; | H, 8.84; | N, 9.45. |
| Found | C, 70.37; | H, 8.84; | N, 8.77. |

11) Compound 89

| Analysis: C₃₁H₃₉N₅O₄ +0.7H₂O | | | |
|---|---|---|---|
| Calc | C, 66.71; | H, 7.29; | N, 12.54. |
| Found | C, 66.91; | H, 7.40; | N, 12.14. |

12) Compound 90

| Analysis: C₃₃H₄₆N₄O₄ +0.8H₂O | | | |
|---|---|---|---|
| Calc | C, 68.67; | H, 8.31; | N, 9.70. |
| Found | C, 68.82; | H, 8.11; | N, 9.70. |

13) Compound 91

| Analysis: C₃₄H₄₈N₄O₄ + 0.3H₂O | | | |
|---|---|---|---|
| Calc | C, 70.11; | H, 8.41; | N, 9.61. |
| Found | C, 70.17; | H, 8.64; | N, 9.33. |

14) Compound 92

| Analysis: C₃₀H₄₂N₄O₄ + 1.2H₂O | | | |
|---|---|---|---|
| Calc | C, 66.14; | H, 8.22; | N, 10.29. |
| Found | C, 66.18; | H, 8.12; | N, 10.31. |

15) Compound 93

| Analysis: C₃₂H₄₄N₄O₆ + 0.3H₂O | | | |
|---|---|---|---|
| Calc | C, 65.57; | H, 7.67; | N, 9.56. |
| Found | C, 65.72; | H, 7.50; | N, 9.34. |

16) Compound 94

| Analysis: C₃₂H₄₄N₄O₄ + 0.3H₂O | | | |
|---|---|---|---|
| Calc | C, 70.04; | H, 8.08; | N, 10.21. |
| Found | C, 70.34; | H, 8.90; | N, 8.93. |

17) Compound 95

| Analysis: C₃₃H₄₆N₄O₄ + .5H₂0 | | | |
|---|---|---|---|
| Calc | C, 69.32; | H, 8.28; | N, 9.80. |
| Found | C, 69.41; | H, 8.25; | N, 9.58. |

18) Compound 99

| Analysis: C₃₈H₅₄N₄O₄ + 1.5H₂O | | | |
|---|---|---|---|
| Calc | C, 69.37; | H, 8.72; | N, 8.51 |
| Found | C, 69.48; | H, 8.44; | N, 8.36 |

19) Compound 102

| Analysis: C₄₀H₄₉N₅O₆ + 1.0 H₂O | | | |
|---|---|---|---|
| Calc | C, 67.30; | H, 7.20; | N, 9.81 |
| Found | C, 67.50; | H, 7.24; | N, 9.53 |

20) Compound 104

| Analysis: C₃₂H₄₁N₅O₄ +0.75H₂O | | | |
|---|---|---|---|
| Calc | C, 67.04; | H, 7.47; | N, 12.21. |
| Found | C, 67.16; | H, 7.56; | N, 11.95. |

21) Compound 105

| Analysis: C₃₂H₄₄N₄O₅ +0.5H₂O | | | |
|---|---|---|---|
| Calc | C, 66.99; | H, 7.91; | N, 9.77. |
| Found | C, 67.00; | H, 8.25; | N, 9.50. |

22) Compound 106

| Analysis: C₃₂H₄₅N₅O₅ +0.8H₂O | | | |
|---|---|---|---|
| Calc | C, 64.69; | H, 7.90; | N, 11.78. |
| Found | C, 64.93; | H, 8.25; | N, 11.12. |

23) Compound 107

| Analysis: C₃₁H₄₄N₃O₆S +0.3H₂O | | | |
|---|---|---|---|
| Calc | C, 61.42; | H, 7.41; | N, 9.24. |
| Found | C, 61.43; | H, 7.54; | N, 9.05. |

24) Compound 110

| Analysis: C₃₅H₄₄N₄O₄ | | | |
|---|---|---|---|
| Calc | C, 71.89; | H, 7.58; | N, 9.58. |
| Found | C, 71.65; | H, 7.55; | N, 9.34. |

25) Compound 111

| Analysis: C₃₄H₄₂N₄O₄ +0.5H₂O | | | |
|---|---|---|---|
| Calc | C, 70.44; | H, 7.47, | N, 9.66. |
| Found | C, 70.82, | H, 7.46; | N, 9.20. |

26) Compound 113

| Analysis: C₃₄H₄₂N₄O₄ +0.3H₂O | | | |
|---|---|---|---|
| Calc | C, 70.88; | H, 7.45; | N, 9.72. |
| Found | C, 71.12; | H, 7.44; | N, 9.32. |

27) Compound 115

| Analysis: C₃₄H₄₂N₄O₄ +0.7H₂O | | | |
|---|---|---|---|
| Calc | C, 69.99; | H, 7.50; | N, 9.60. |
| Found | C, 70.14, | H, 7.63; | N, 9.25. |

28) Compound 116

| Analysis: C₃₄H₄₆N₄O₄ +1.4H₂O | | | |
|---|---|---|---|
| Calc | C, 68.06; | H, 8.19; | N, 9.33. |
| Found | C, 68.40; | H, 8.14; | N, 8.87. |

29) Compound 117

| Analysis: C₄₀H₅₁N₅O₆ +0.6H₂O | | | |
|---|---|---|---|
| Calc | C, 67.79; | H, 7.42; | N, 9.88. |
| Found | C, 67.81; | H, 7.58; | N, 9.76. |

30) Compound 118

| Analysis: C₃₃H₄₇N₅O₄ +0.7H₂O | | | |
|---|---|---|---|
| Calc | C, 67.14; | H, 8.26; | N, 11.86 |
| Found | C, 67.54; | H, 8.51; | N, 11.28. |

31) Compound 119

| Analysis: C₃₅H₄₈N₄O₄ +1.25H₂O | | | |
|---|---|---|---|
| Calc | C, 68.76; | H, 8.32; | N, 9.16. |
| Found | C, 69.07; | H, 8.19; | N, 8.75. |

32) Compound 121

| Analysis: C₃₄H₄₈N₄O₄ +1H₂O | | | |
|---|---|---|---|
| Calc | C, 68.66; | H, 8.47; | N, 9.42. |
| Found | C, 69.02; | H, 8.32; | N, 9.06. |

33) Compound 125

| Analysis: C₃₇H₄₈N₄O₄ +0.5H₂O | | | |
|---|---|---|---|
| Calc | C, 71.47; | H, 7.94; | N, 9.01 |
| Found | C, 71.65; | H, 7.91; | N, 8.73. |

34) Compound 126

| Analysis: C₄₁H₅₄N₄O₅ +2H₂O | | | |
|---|---|---|---|
| Calc | C, 68.83; | H, 8.25; | N, 7.64. |
| Found | C, 69.03; | H, 7.79; | N, 7.50. |

35) Compound 132

| Analysis: C₃₅H₅₀N₄O₄ | | | |
|---|---|---|---|
| Calc | C, 71.15; | H, 8.53; | N, 9.48. |
| Found | C, 70.90; | H, 8.74; | N, 9.12. |

36) Compound 133

| Analysis: C₄₀H₅₂N₄O₄ +0.9H₂O | | | |
|---|---|---|---|
| Calc | C, 71.80; | H, 8.10; | N, 8.37. |
| Found | C, 71.86; | H, 8.17; | N, 8.18. |

37) Compound 137

| Analysis: C₃₇H₄₈N₄O₄ +0.8H₂O | | | |
|---|---|---|---|
| Calc. | C, 70.85; | H, 7.97; | H, 8.93. |
| Found | C, 71.01; | H, 7.97; | N, 8.54. |

38) Compound 139

| Analysis: C₃₉H₅₁N₅O₄ +0.8H₂O | | | |
|---|---|---|---|
| Calc | C, 70.09; | H, 7.93; | N, 10.48. |
| Found | C, 70.18; | H, 7.79; | N, 10.42. |

39) Compound 142

| Analysis: C₃₈H₅₅N₅O₆ | | | |
|---|---|---|---|
| Calc | C, 67.33; | H, 8.18; | N, 10.33. |
| Found | C, 67.02; | H, 8.31; | N, 9.89. |

40) Compound 143

| Analysis: C₃₄H₅₁N₅O₄ | | | |
|---|---|---|---|
| Calc | C, 68.77; | H, 8.66; | N, 11.80. |
| Found | C, 68.57; | H, 8.50; | N, 11.53 |

41) Compound 144

| Analysis: C₃₈H₅₉N₅O₄ +0.4H₂O | | | |
|---|---|---|---|
| Calc | C, 69.45; | H, 9.17; | N, 10.65. |
| Found | C, 69.69; | H, 9.02; | N, 10 35. |

42) Compound 145

| Analysis: C₃₆H₄₇N₅O₄ +H₂O | | | |
|---|---|---|---|
| Calc | C, 68.44; | H, 7.82; | N, 11.08 |
| Found | C, 68.69; | H, 7.74; | N, 10.76. |

43) Compound 148

| Analysis: C₃₆H₄₇N₅O₄ +0.4H₂O | | | |
|---|---|---|---|
| Calc | C, 69.62; | H, 7.56; | N, 11.27. |
| Found | C, 69.79; | H, 7.70; | N, 11.12. |

44) Compound 149

| Analysis: C₃₉H₅₀N₄O₄ +0.4H₂O | | | |
|---|---|---|---|
| Calc | C, 72.50; | H, 7.93; | N, 8.67 |
| Found | C, 72.44; | H, 7.99; | N, 8.87. |

45) Compound 150

| Analysis: C₃₆H₄₇N₅O₄ +0.3H₂O | | | |
|---|---|---|---|
| Calc | C, 69.83; | H, 7.74; | N, 11.31. |
| Found | C, 69.93; | H, 7.65, | N, 11.10. |

46) Compound 151

| Analysis: C₃₃H₄₇N₅O₄ | | | |
|---|---|---|---|
| Calc | C, 68.60; | H, 8.20; | N, 12.12. |
| Found | C, 68.40; | H, 8.16; | N, 11.90. |

47) Compound 245

| Analysis: C₃₆H₄₄N₄O₄ | | | |
|---|---|---|---|
| Calc | C, 72.46; | H, 7.43; | N, 9.39 |
| Found | C, 72.49; | H, 7.49; | N, 9.25 |

48) Compound 246

| Analysis: C₃₇H₄₆N₄O₄ +0.25H₂O | | | |
|---|---|---|---|
| Calc | C, 72.26; | H, 7.61; | N, 9.10. |
| Found | C, 72.35; | H, 7.83; | N, 8.73. |

49) Compound 154

| Analysis: C₃₅H₄₈N₄O₄ +0.8H₂O | | | |
|---|---|---|---|
| Calc | C, 69.70; | H, 8.29; | N, 9.29 |
| Found | C, 69.65; | H, 8.27; | N, 9.35. |

50) Compound 158

| Analysis: C₃₅H₄₈N₄O₄ +0.5H₂O | | | |
|---|---|---|---|
| Calc | C, 73.29; | H, 7.65; | N, 8.33. |
| Found | C, 73.71, | H, 7.75, | N, 7.75. |

51) Compound 159

| Analysis: C₃₅H₄₈N₄O₄ | | | |
|---|---|---|---|
| Calc | C, 73.09; | H, 7.66; | N, 8.31. |
| Found | C, 73.40; | H, 7.75; | N, 7.80. |

52) Compound 160

| Analysis: C₃₈H₄₈N₄O₄ +1.0H₂O | | | |
|---|---|---|---|
| Calc | C, 70.78; | H, 8.12; | N, 8.68. |
| Found | C, 71.00; | H, 8.05; | N, 8.59. |

53) Compound 161

| Analysis: C₄₃H₅₂N₄O₄ +1H₂O | | | |
|---|---|---|---|
| Calc | C, 73.05; | H, 7.70; | N, 7.92. |
| Found | C, 73.29; | H, 7.95; | N, 7.37. |

54) Compound 166

| Analysis: C₃₃H₄₆N₄O₄ +1.5H₂O | | | |
|---|---|---|---|
| Calc. | C, 67.20; | H, 8.37; | N, 9.50 |
| Found | C, 67.38; | H, 7.98; | N, 9.41. |

55) Compound 171

| Analysis: C₃₆H₅₂N₄O₆ +1.6H₂O | | | |
|---|---|---|---|
| Calc | C, 64.95; | H, 8.36; | N, 8.41. |
| Found | C, 65.26; | H, 8.15; | N, 8.07. |

56) Compound 177

| Analysis: C₃₈H₄₇N₅O₄ | | | |
|---|---|---|---|
| Calc | C, 71.56; | H, 7.43; | N, 10,98. |
| Found | C, 71.64; | H, 7.62, | N, 10.93. |

57) Compound 178

| Analysis: C₃₈H₅₀N₄O₄ | | | |
|---|---|---|---|
| Calc | C, 72.81; | H, 8.04; | N, 8.94. |
| Found | C, 72.96; | H, 8.17; | N, 8.83. |

58) Compound 179

| Analysis: C₃₈H₄₇N₅O₄ | | | |
|---|---|---|---|
| Calc | C, 71.56; | H, 7.43; | N, 10.98. |
| Found | C, 72.00; | H, 7.55; | N, 10.87. |

59) Compound 180

| Analysis: C₃₈H₄₇F₃N₄O₄ | | | |
|---|---|---|---|
| Calc | C, 67.04; | H, 6.96; | N, 8.23. |
| Found | C, 67.02; | H, 7.25; | N, 8.23. |

60) Compound 181

| Analysis: C₃₈H₄₇F₃N₄O₄ | | | |
|---|---|---|---|
| Calc | C, 67.04; | H, 6.96; | N, 8.23 |
| Found | C, 66.63; | H, 6.98; | N, 7.94. |

62) Compound 185

| Analysis: C₃₄H₄₈N₄O₄ | | | |
|---|---|---|---|
| Calc | C, 70.80; | H, 8.39; | N, 9.71 |
| Found | C, 70.44; | H, 8.45; | N, 9.51. |

63) Compound 186

| Analysis: C₃₇H₄₈N₄O₄ +0.8H₂O | | | |
|---|---|---|---|
| Calc | C, 70.85; | E, 7.97; | N, 8.93 |
| Found | C, 71.12; | H, 8.25; | N, 8.45. |

64) Compound 191

| Analysis: C₄₂H₅₁N₅O₄ +.5CH₂Cl₂ | | | |
|---|---|---|---|
| Calc | C, 69.78; | H, 7.16; | N, 9.58 |
| Found | C, 69.75; | H, 7.31; | N, 9.68. |

65) Compound 203

| Analysis: C₃₇H₄₇N₄C₄ +1.1H₂O | | | |
|---|---|---|---|
| Calc | C, 68.31; | H, 7.62; | N, 8.61 |
| Found | C, 68.32; | H, 7.57; | N, 8.53. |

66) Compound 204

| Analysis: C₃₇H₄₇ClN₄O₄ | | | |
|---|---|---|---|
| Calc | C, 68.66; | H, 7.32; | N, 8.66. |
| Found | C, 68.32, | H, 7.48; | N, 8.42. |

67) Compound 205

| Analysis: C₃₈H₅₀N₄O₅ +0.7H₂O | | | |
|---|---|---|---|
| Calc | C, 69.63; | H, 7.90; | N, 8.54. |
| Found | C, 69.72; | N, 7.91; | N, 8.54. |

68) Compound 206

| Analysis: C₃₉H₅₂N₄O₆ +0.8H₂O | | | |
|---|---|---|---|
| Calc | C, 68.15; | H, 7.86; | N, 8.15 |
| Found | C, 68.01; | H, 8.02; | N, 8.15. |

### Example 12

### A) [S-(R*,S*)]3,3-Diethyl-2-[4-[[[2-(4-hydroxy-1-piperidinyl)ethyl]amino]carbonyl]phenoxy]-N-[1-(4-methylphenyl)butyl]-4-oxo-1-azetidinecarboxamide

When 1-(2-aminoethyl)-4-benzyloxypiperidine is used in place of N,N,N'-trimethylethylene diamine in the procedure of Example 11b there is obtained [S-(R*,S*)] 3,3-diethyl-2-[4-[[[2-(4-benzyloxy-1-piperidinyl]ethyl]amino]carbonyl]phenoxy]-N-[1-(4-methylphenyl)butyl]-4-oxo-1-azetidinecarboxamide.

### B) [S-(R+,S*)]-3,3-diethyl-2-[4-[[[2-(4-hydroxy-1-piperidinyl)ethyl]amino]carbonyl]phenoxy]-N-[1-(4-methyl-phenyl)butyl]-4-oxo-1-azetidine-carboxamide

A solution of the amide from step A above in 10 ml of glacial acetic acid containing 22 mg of 10% Pd/C is hydrogenated under 42 lb hydrogen pressure. When TLC indicate completion of the reaction, the mixture is filtered and concentrated in vacuo after the addition of 50 ml toluene. The residue is dissolved in ethyl acetate, washed with saturated sodium bicarbonate solution. The organic layer is dried with sodium sulfate and concentrated in vacuo. The residue is chromatographed on 15 g silica gel using 5% methanol in methylene chloride and yields 96 mg of [S-(R+,S*)]-3,3-diethyl-2-[4-[[[2-(4-hydroxy-1-piperidinyl)ethyl]amino]carbonyl]phenoxy]-N-[1-(4-methyl-phenyl)butyl]-4-oxo-1-azetidine-carboxamide

| Analysis: C₃₃H₄₆N₄O₅ +1.3H₂O | | | |
|---|---|---|---|
| Calc | C, 65.83; | H, 8.13; | N, 9.30 |
| Found | C, 66.10; | H, 8.06; | N, 8.91 |

When 1-(2-aminoethyl)-4-benzyloxypiperidine is replaced in the procedure of Example 12 by the appropriate amines, the following compounds 123, 124, 129, 131 and 138 are obtained, for example:
1) Compound 129

| Analysis: C₃₄H₄₈N₄O₅ | | | |
|---|---|---|---|
| Calc | C, 68.89; | H, 8.16; | N, 9.45 |
| Found | C, 68.68; | H, 8.18; | N, 8.65 |

2) Compound 131

| Analysis: C₃₂H₄₄N₄O₅ +1H₂O | | | |
|---|---|---|---|
| Calc | C, 65.92; | H, 7.96; | N, 9.61 |
| Found | C, 66.07; | H, 7.86; | N, 9.45 |

3) Compound 138

| Analysis: C₃₃H₄₆N₄O₅ +0.5H₂O | | | |
|---|---|---|---|
| Calc | C, 67.43; | H, 8.06; | N, 9.53 |
| Found | C, 67.61; | H, 8.06; | N, 9.37 |

### Diamine Intermediates

The diamines used to prepare the amino amides described herein were commercially available or prepared according to the following routes

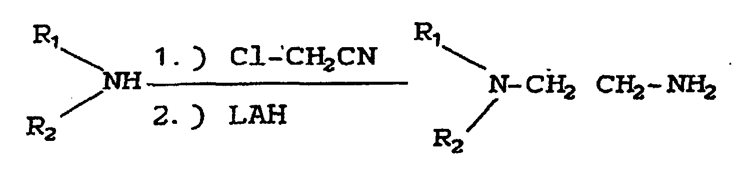

### EXAMPLE 13

### A N-cyanomethylhomopiperazine.

To a solution of 1.98 g homopiperazine in 50 ml acetone is added 4.25 g of powdered anhydrous sodium carbonate and 1.3 ml of chloroacetonitrite. After 24 hrs the reaction mixture is filtered and the filter cake washed with 100 ml acetone. The combined filtrates are concentrated in vacuo and the residue chromatographed on silica gel using methylene chloride as the eluent. The yield of N-cyanomethyl homopiperazine is 2.69 g.

### B. N-(2-aminoethyl)homopiperazine.

To a suspension of 1.02 g lithium aluminum hydride in 50 ml of ether is slowly added a solution of 2.65 gm N-cyanomethyl homopiperazine in 25 ml ether. After the addition in complete the mixture is heated at reflux for 1 hour, then cooled to room temperature and quenched carefully with 1 ml water, 1 ml of 15% sodium hydroxide solution and 3 ml water. The mixture is filtered through sodium sulfate, the filter cake washed well with ether and the combined filtrates concentrated in vacuo to yield 2.60 g. N-(2-aminoethyl)homopiperazine.

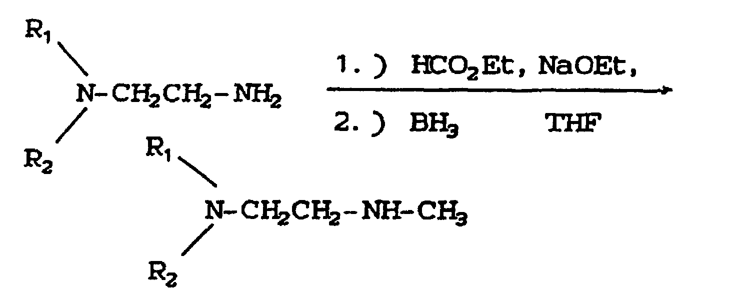

### EXAMPLE 14

### N-(2-methylaminoethyl)homopiperazine.

### A). N-(2-formamidoethyl)homopiperazine.

To a carefully prepared solution of 0.718 g of 60% sodium hydride dispension in 75 ml of absolute ethanol which has been cooled to 0°C is added 7.3 ml of ethyl formate. After 5 minutes there is added a solution of 2.55 gm of N-(2-aminoethyl)homopiperazine in 25 ml absolute ethanol. The mixture is stirred at room temperature overnight. Saturated sodium bicarbonate solution (15 ml) is then added and the reaction mixture is stirred with 150 ml ethyl acetate, filtered through MgSO₄ and the filtrate concentrated in vacuo. Chromatography of the residue on 150 gm silica gel using an eluent of methylene chloride/methanol/conc. ammonium hydroxide (95/5/0.5) gives 2.78 g of N-(2-formamidoethyl)homopiperazine.

### B). N-(2-methylaminoethyl)homopiperazine.

To a solution of 2.75 gm of N-(2-formamidoethyl)homopiperazine in 20 ml of dry THF under N₂ is added carefully 60 ml of borane THF solution. After the addition is complete the reaction mixture is heated to reflux for 5 hours then stirred at room temperature overnight. The reaction mixture is quenched by the careful addition of 20 ml of 6N HCl followed by refluxing for 1 hour. The reaction mixture is cooled, 50 ml of water added and solid KOH added carefully to alkaline pH. Extraction with ether gives the desired product N-(2-methylamino) homopiperazine.

### EXAMPLE 15

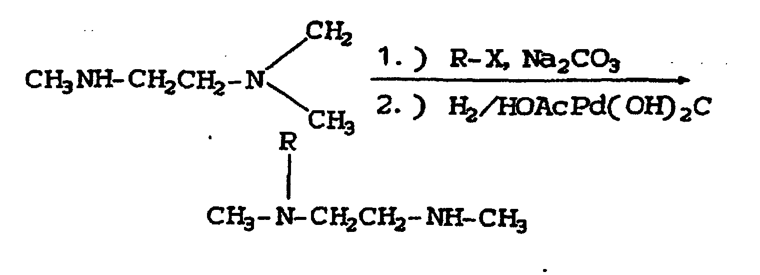

### A) N-benzyl-N,N'-dimethyl-N'-(2-phenylethyl)-ethylenediamine

A mixture of 0.900 gm N-benzyl-N,N'-dimethylethylenediamine, 1.10 gm powdered sodium carbonate and 0.75 ml of 2-phenylethylbromide is refluxed for 5 hours. An additional 0.25 ml of bromide is added during this time. The reaction mixture is then cooled and filtered. The filterate is concentrated in vacuo and the residue chromatographed on silica gel using an eluent of CH₂Cl₂/CH₃OH/NH₄OH (97/3/0.3) to yield 0.875 gm of N-benzyl-N,N'-dimethyl-N'-(2-phenylethyl)ethylenediamine.

### B) N,N'-dimethyl-N-(2-phenylethyl)ethylenediamine.

To a solution 0.870 gm N-benzyl-N-N'-dimethyl-N'-(2-phenylethyl)ethylenediamine in 10 ml ethanol and 5 ml acetic acid is added 0.18 gm Pd (OH)₂/C. The mixture is hydrogenated at 40 psi for 3.5 hours, then filtered and concentrated in vacuo. The residue is made akaline with 1N NaOH and extracted well with ethyl acetate (5 X 25 ml). The combined extracts are filtered through sodium sulfate and concentrated to yield N,N'-dimethyl-N-(2-phenylethyl)ethylenediamine.

### EXAMPLE 16

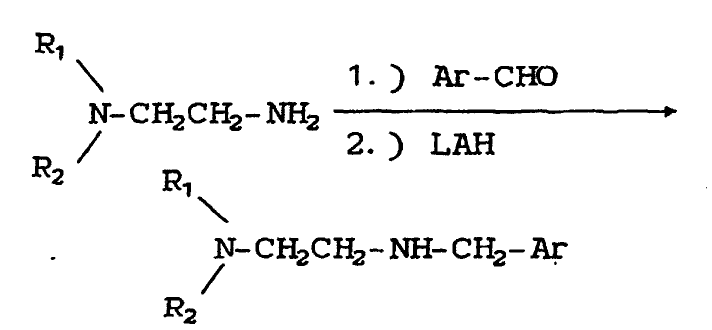

### A)

A solution of 1.28 gm 1-(2-amino-ethyl)-piperdine and 1.07 gm pyridine-3-carboxaldehyde in 40 ml of toluene is heated to reflux under a Dean Stark trap. After 10 ml toluene distilled over the NMR of an aliquot indicated no aldehyde left. The reaction mixture was concentrated and the imine used directly in the next step.

### B)

To a suspension of 0.380 gm of lithium aluminum hydride in 30 ml of dry THF which has been cooled to -10°C is added dropwise a solution of the above imine in 20 ml of dry THF. After about 1 hour the cold reaction mixture is quenched by the addition of 5 ml of 5N NaOH, then diluted with 100 ml ether and 20 ml of water. The organic layer is separated, washed with brine, filtered thru sodium sulfate and concentrated to give 2.17 gm of 1-[2-(3-pyridylmethylamino)ethyl]-piperidine suitable for use in subsequent reactions.

### EXAMPLE 17

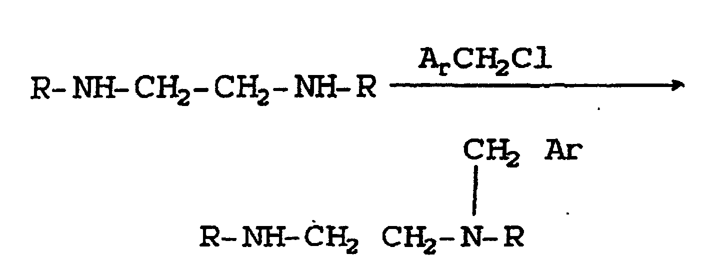

To 7.50 gm of N,N'-dimethylethylenediamine which has been cooled in an ice-ethanol bath is added portionwise over a 30 minute period 1.40 gm of 3-picolyl chloride. After stirring cold for 1 hour after the addition is completed, the reaction mixture is concentrated in vacuo and the residue partitioned between 50 ml of ether and 10 ml of 5N NaOH solution. The organic layer is separated and the aqueous layer extracted 2 times with 50 ml of ether. The combined organic extracts are dried through sodium sulfate and concentrated in vacuo. Chromatography on 150 gm silica gel using CH₂Cl₂/CH₃OH/NH₄OH (90/10/1) as eluent gives 0.930 g of N,N'-dimethyl-N-(3-pyridylmethyl)ethylenediamine.

### EXAMPLE 18

### Amino Acid → diamine

A) To an ice cooled solution of 2.29 N-CBZ-D-Proline in 50 ml of CHCl₂ is added 1.35 gm 1-hydroxybenzotriazole hydrate followed by 2.06 gm of dicyclohexylcarbodiimide. After 20 minutes, 0.85 ml of pyrrolidine is added and the reaction mixture stirred overnight after which time it is filtered and the filtrate concentrated in vacuo. The residue is partitioned between 100 ml ethyl acetate and 50 ml of 2N hydrochloric acid. The organic layer is separated, washed with 50 ml of 1.0N sodium hydroxide solution, dried through sodium sulfate and concentrated in vacuo. Chromatography on 150 gm of silica gel using ethylacetate in hexanes (30 - 100%) as eluent gives 2.04 gm of the desired pyrrolidine amide
B) To a solution of 1.519 gm of the amide (prepared in A) in 20 ml absolute ethanol is added 75 mg of 10% Pd on carbon catalyst. The mixture is hydrogenated at 40 psi for about an hour then filtrate and the filtrate concentrated to yield D-proline pyrrolidine amide.
C) To a suspension of 0.380 gm of lithium aluminum hydride in 15 ml of dry tetrahydrofuran is carefully added a solution of the D-proline amide (prepared in B above) in 10 ml tetrahydrofuran. The mixture is refluxed for 2 hrs then cooled and quenched with 2 ml of 2.5 N sodium hydroxide. The mixture is filtered through a pad of sodium sulfate and the filter cake washed with 2 x 50 ml of ether. The combined filtrates are concentrated in vacuo to yield 0.80 gm of desired 2-(1-pyrrolidinylmethyl) pyrrolidine.

### EXAMPLE 19

### [S-(R*,S*)]-2-[4-[[(4-Methyl)piperazin-1-yl]carbonyl] phenoxy]-((3,3-diethyl-N-[1-(methylphenyl)butyl]-4-oxo-1-azetidinecarboxamide

A solution of S-(R*,S*)]-4-(((3,3-diethyl-1-((4-methylphenyl)butylamino)carbonyl)-4-oxo-2-azetidinyl)oxy)benzoyl chloride (3.8 mmol), prepared as in Example 11A, in 50 ml of methylene chloride was cooled in an ice bath and a solution of 0.70 gm of N-methylpiperazine in 10 ml of methylene chloride was added over 5 min. The reaction was stirred for 1 hr and was then poured into a mixture of ice water and 10% potassium carbonate. The product was extracted with two portions of methylene chloride and each methylene chloride layer was washed with a portion of brine. The methylene chloride layers were combined, dried over sodium sulfate and evaporated. The residue was purified with flash chromatography using ethyl acetate, then 2% triethylamine/10% methanol/88% ethyl acetate to afford 2.1 gm of the title compound as a white solid.

| Analysis: C₃₀H₄₂N₄O₄ | | | |
|---|---|---|---|
| Calc | C, 69.64; | H, 7.92; | N, 10.48 |
| Found | C, 69.62; | H, 8.23; | N, 10.46 |

### Example 20

### [S-(R*,S*)]-2-[4-[[(4-Methyl)piperazin-1-yl]carbonyl] phenoxy]-((3,3-diethyl-N-[1-(3,4-methylenedioxyphenyl) butyl]-4-oxo-1-azetidinecarboxamide

When [S-(R*,S*)]-4-(((3,3-diethyl-1-((3,4-methylenedioxyphenyl)butylamino)carbonyl)-4-oxo-2-azetidinyl)oxy)benzoyl chloride (3.1 mmol), prepared as in Example 11A, was reacted with N-methylpiperazine as in Example 19, there was obtained 1.75 gm of the title compound.

| Analysis: C₃₁H₄₀N₄O₆ | | | |
|---|---|---|---|
| Calc | C, 65.94; | H, 7.14; | N, 9.92 |
| Found | C, 65.80; | H, 7.31; | N, 10.05 |

### Example 21

### [S-(R*,S*)]-2-[4-[[(4-Hydroxyethyl)piperazin-1-yl] carbonyl]phenoxy]-((3,3-diethyl-N-[1-(methylphenyl) butyl]-4-oxo-1-azetidinecarboxamide

When [S-(R*,S*)]-4-(((3,3-diethyl-1-((4-methylphenyl)butylamino)carbonyl)-4-oxo-2-azetidinyl)oxy)benzoyl chloride (3.8 mmol), prepared as in Example 11A, was reacted with N-(2-hydroxyethyl)piperazine (7.6 mmol) and diisopropylethylamine (3.8 mmol) as in Example 19, there was obtained 2.1 gm of the title compound.

| Analysis: C₃₂H₄₄N₄O₅ | | | |
|---|---|---|---|
| Calc | C, 68.06; | H, 7.85; | N, 9.92 |
| Found | C, 67.88; | H, 7.87; | N, 10.17 |

### Example 22

### [S-(R*,S*)]-2-[4-[[(4-Hydroxyethyl)piperazin-1-yl] carbonyl]phenoxy]-((3,3-diethyl-N-[1-(3,4-methylene dioxyphenyl)butyl]-4-oxo-1-azetidinecarboxamide

When [S-(R*,S*)]-4-(((3,3-diethyl-1-((3,4-methylenedioxyphenyl)butylamino)carbonyl)-4-oxo-2-azetidinyl)oxy)benzoyl chloride (3.1 mmol), prepared as in Example 11A, was reacted with N-(2-hydroxyethyl)piperazine (6.2 mmol) and diisopropylethylamine (3.1 mmol) as in Example 19, there was obtained 1.50 gm of the title compound.

| Analysis: C₃₂H₄₂N₄O₇ ·1.5H20 | | | |
|---|---|---|---|
| Calc | C, 61.94; | H, 6.89; | N, 9.06 |
| Found | C, 61.95; | H, 6.92; | N, 8.96 |

### Example 23

### [S-(R*,S*)]-2-[4-[[(4-Cyclopropyl)piperazin-1-yl] carbonyl]phenoxy]-((3,3-diethyl-N-[1-(4-methylphenyl)b utyl]-4-oxo-1-azetidinecarboxamide

To a solution of [S-(R*,S*)]-4-(((3,3-diethyl-1-((4-methylphenyl)butylamino)carbonyl)-4-oxo-2-azetidinyl)oxy)benzoyl chloride (3.8 mmol), prepared as in Example 11A, in 50 ml of methylene chlorine was added N-(cyclopropyl)piperazine dihydrochloride (5.7 mmol) and then a solution of diisopropylethylamine (15.8 mmol) in 10 ml of methylene chloride was added over 5 min with ice-bath cooling. The reaction was stirred for 1 hr at 0 C and then poured into ice water. The product was extracted with two portions of methylene chloride and each methylene chloride layer was washed with a portion of brine. The methylene chloride layers were combined, dried over sodium sulfate and ethyl acetate/50% hexanes, then 70% ethyl acetate/30% hexanes to afford 2.1 gm of the title compound as a white solid.

| Analysis: C₃₂H₄₂N₄O₄ | | | |
|---|---|---|---|
| Calc | C, 70.69; | H, 7.91; | N, 9.99 |
| Found | C, 70.62; | H, 8.04; | N, 9.95 |

### Example 24

### [S-(R*,S*)]-2-[4-[[(4-Cyclopropyl)piperazin-1-yl] carbonyl]phenoxy]-((3,3-diethyl-N-[1-(3,4-methylene dioxyphenyl)butyl]-4-oxo-1-azetidinecarboxamide

When [S-(R*,S*)]-4-(((3,3-diethyl-1-((3,4-methylenedioxyphenyl)butylamino)carbonyl)-4-oxo-2-azetidinyl)oxy)benzoyl chloride (3.1 mmol), prepared as in Example 11A, was reacted with N-(cyclopropyl)piperazine (4.6 mmol) and diisopropylethylamine (9.3 mmol) as in Example 23, there was obtained 1.80 gm of the title compound.

| Analysis: C₃₂H₄₂N₄O₇ | | | |
|---|---|---|---|
| Calc | C, 67.10; | H, 7.17; | N, 9.49 |
| Found | C, 67.03; | H, 7.31; | N, 9.47 |

### Example 25

### [S-(R*,S*)]-2-[4-[[(4-Piperazin-1-yl)carbonyl]phenoxy] - ((3,3-diethyl-N-[1-(4-methylphenyl)butyl]-4-oxo-1-azetidinecarboxamide

### Step A: [S-R*,S*)]-2-[4-[[(4-(t-Butoxycarbonyl))piper azin-1-yl]carbonyl]phenoxy]-((3,3-diethyl-N-[ 1-(4-methylphenyl)butyl]-4-oxo-1-azetidinecarboxamide

[S-(R*,S*)]-4-(((3,3-Diethyl-1-((4-methylphenyl)butyla mino)carbonyl)-4-oxo-2-azetidinyl)oxy)benzoyl chloride (0.4 mmol), prepared as in Example 11A, was reacted with N-(t-butoxycarbonyl)piperazine (0.6 mmol) and triethylamine (1.2 mmol) as in Example 23. The crude, title product so obtained was used directly in the following Step B.

### Step B: [S-R*,S*)]-2-[4-[(Piperazin-1-yl)carbonyl]phe noxy]-((3,3-diethyl-N-[1-(4-methylphenyl)buty 1-4-oxo-1-azetidinecarboxamide

The product from Step A was dissolved in 0.5 ml of anisole and 2 ml of cold TFA was added. The reaction was stirred at 0 C for 1 hr and was then diluted with methylene chloride and evaporated. The residue was taken up in methylene chloride, washed with 10% sodium carbonate and brine, dried over sodium sulfate and concentrated. The residue was purified by flash chromatography using 5, then 10% methanol/methylene chloride to afford 0.212 gm of title product.

| Analysis: C₃₀H₄₀N₄O₅ ·1H20 | | | |
|---|---|---|---|
| Calc | C, 66.89; | H, 7.85; | N, 10.40 |
| Found | C, 67.06; | H, 7.55; | N, 10.30 |

### Example 26

### [S-(R*,S*)]-2-[4-[[(4-Piperazin-1-yl)carbonyl]phenoxy] - ((3,3-diethyl-N-[1-(3,4-methylenedioxyphenyl)butyl]-4 - oxo-1- azetidinecarboxamide

### Step A: [S-R*,S*)]-2-[4-[[(4-Benzyloxycarbonyl) Piperazin-1-yl]carbonyl]phenoxy]-((3,3-diethy 1-N-[1-3,4-methylenedioxyphenyl)butyl]-4-oxo-1-azetidinecarboxamide

When [S-(R*,S*)]-4-(((3,3-diethyl-1-((3,4-methylenedioxyphenyl)butylamino)carbonyl)-4-oxo-2-azet idinyl)oxy)benzoyl chloride (0.41 mmol), prepared as in Example 11A, was reacted with N-(benzyloxycarbonyl)piperazine (0.77 mmol) and diisopropylethylamine (1.6 mmol) as in Example 23, there was obtained 290 mg of the title compound.

### Step B: [S-R*,S*)]-2-[4-[(Piperazin-1-yl)carbonyl]phe noxy]-((3,3-diethyl-N-[1-(3,4-methylenedioxyp henyl)butyl-4-oxo-1-azetidinecarboxamide

A solution of 250 mg of material from Example 26, Step A in 10 ml of ethanol was hydrogenated at 40 p.s.i. over 50 mg of 10% Pd/C for 16 hrs. The reaction was filtered and evaporated. The residue was purified by preparative TLC eluting with 2% TEA/10% methanol/88% ethyl acetate to afford 150 mg of title product.

| Analysis: C₃₀H₃₈N₄O₆ ·3H2O | | | |
|---|---|---|---|
| Calc | C, 59.59; | H, 7.33; | N, 9.29 |
| Found | C, 59.66; | H, 7.65; | N, 9.61 |

## Claims

1. A compound of Formula (I): 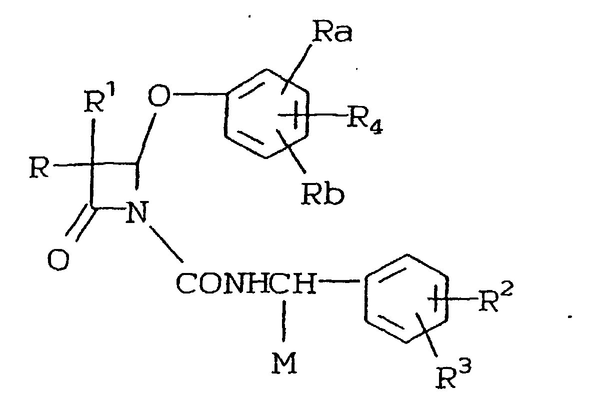 or a pharmaceutically acceptable salt thereof wherein:
R is C₁₋₆alkyl;
R¹ is C₁₋₆alkyl or C₁₋₆alkoxy-C₁₋₆alkyl;
M is
(1) hydrogen,
(2) C₁₋₆alkyl,
(3) hydroxy C₁₋₆alkyl,
(4) halo C₁₋₆alkyl,
(5) C₂₋₆alkenyl, or
(6) C₁₋₆alkoxy-C₁₋₆alkyl;
Ra and Rb are each individually
(1) hydrogen,
(3) halo,
(4) carboxy,
(5) C₁₋₆alkoxy,
(6) phenyl,
(7) C₁₋₆alkylcarbonyl,
(8) di-(C₁₋₆alkyl)amino;
(9) hydroxy;
R² and R³ are each independently
(1) hydrogen,
(2) C₁₋₆alkyl,
(3) halo,
(4) carboxy,
(5) C₁₋₆alkoxy,
(6) phenyl,
(7) C₁₋₆alkylcarbonyl,
(8) aminoC₂₋₃alkyloxy carbonyl wherein the amino is optionally mono or di substituted with C₁₋₆alkyl,
(9) aminoC₂₋₃alkylamino carbonyl wherein the amino is optionally mono or di substituted with C₁₋₆alkyl,
(10) hydroxy,
(11) aminocarbonyl wherein the amino is optionally mono or di substituted with C₁₋₆alkyl,
(12) hydroxymethyl,
(13) aminocarbonyloxy C₁₋₃alkyl, wherein the amino is optionally mono or di substituted with C₁₋₆alkyl,
(14) cyano,
(15) morpholinocarbonylphenyl,
(16) amino wherein the amino is optionally mono or di substituted with C₁₋₆alkyl, or R² and R³ may be joined together to form a methylenedioxy group or a furan ring,
(17) morpholinocarbonyl;
R₄ is 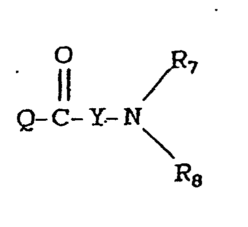
wherein
Q is a covalent bond and
Y is 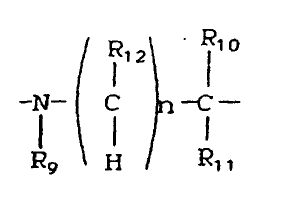
R₁₂ is hydrogen or C₁₋₃alkyl;
R₇ and R₈ are each individually
(a) hydrogen,
(b) C₁₋₆alkyl,
(c) C₁₋₆alkyloxy C₂₋₃alkyl,
(d) hydroxy C₂₋₆alkyl,
(e) polyhydroxyC₂₋₆alkyl,
(f) carboxamido C₁₋₆alkyl,
(h) C₁₋₆alkanoyl,
(i) substituted phenyl or phenyl C₁₋₆alkyl, wherein the substituents are X₁ and X₂ as defined immediately below,
(j) C₂₋₆alkenyl,
(k) C₆₋₁₀cycloalkenyl,
(l) heteroaryl C₁₋₆alkyl wherein the hetero aryl is pyridinyl, imidazolyl, triazolyl, benzylimidazolyl, or furyl,
(m) carboxy C₁₋₆alkyl,
(n) carbo C₁₋₆alkoxy C₁₋₃alkyl,
(o) phenylsulfonyl,
(p) C₁₋₆alkylsulfonyl,
(q) benzyloxy,
(r) morpholinyl C₁₋₃alkylsulfonyl,
(s) tetrahydropyranyl,
(t) aminoC₁₋₃alkylsulfonyl wherein the amino is optionally mono or di substituted with C₁₋₆alkyl,
(u) aminocarbonyl wherein the amino is optionally mono or di substituted with C₁₋₆alkyl,
(v) aminocarbonyloxyC₂₋₆alkyl wherein the amino is optionally mono or di substituted with C₁₋₆alkyl,
(w) azabicyclo of 7 to 12 atoms,
(x) di C₁₋₃alkylamino C₂₋₆alkyl wherein the amino is optionally mono or di substituted with C₁₋₆alkyl,
(y) bicycloalkyl of 7 to 12 atoms,
(z) C₃₋₁₀cycloalkyl optionally substituted with C₁₋₆alkyl,
(aa) pyrazolidinyl,
(bb) substituted piperidinyl or prrrolidinyl wherein the substituent is hydrogen, C₁₋₃alkyl, hydroxyC₁₋₃alkylbenzyl, carboxamido or amino wherein the amino is optionally mono or di substituted with C₁₋₆alkyl,
(cc) substituted pyrrolidinyl wherein the substituent is carboxamido or amino wherein the amino is optionally mono or di substituted with C₁₋₆alkyl,
(dd) pyrimidinyl,
(ee) N-cyano-N'-phenylamidino,
(ff) phosphonoC₁₋₆alkyl, or
(gg) α-C₁₋₃alkyl benzyl or mono or di substituted benzyl or mono or di substituted pyridylmethyl, wherein the substituents are X₁ and X₂,
wherein
X₁ is
(1) hydrogen,
(2) halo,
(3) C₁₋₆alkyl,
(4) halo-C₁₋₆alkyl,
(5) C₂₋₆alkenyl,
(6) hydroxy-C₁₋₆alkyl,
(7) C₁₋₆alkylcarbonyl,
(8) C₁₋₆alkylcarbonylamino;
(9) CN,
(10) CF₃,
(11) CH₃O,
(12) amino wherein the amino is optionally mono or di substituted with C₁₋₆alkyl;
(13) carboxy, or
(14) phenylsulfonylaminocarbonyl;
X₂ is hydrogen, halo or C₁₋₆alkyl;
n is 1, 2, 3, 4 or 5;
R₉ is selected from hydrogen, C₁₋₄ alkyl, and C₁₋₃alkoxyC₁₋₃alkyl; or phenyl, phenyl C₁₋₃alkyl, pyridyl, and pyridyl C₁₋₃alkyl;
R₁₀ and R₁₁ are each independently selected from hydrogen, C₁₋₄alkyl, and C₁₋₃alkoxy C₁₋₃alkyl, or are together 0=; or
R₇ and R₈ are joined together to form mono or di substituted ring of 4, 5, 6, or 7 atoms or 7 to 12 atoms such as
(1) piperidinyl or homopiperidinyl,
(2) piperazinyl,
(3) morpholinyl, thiomorpholinyl or 1,1-dioxo-4-thiomorpholinyl,
(4) pyrrolidinyl,
(5) pyrryl,
(6) imidazolyl,
(7) triazolyl,
(8) saturated azabicyclo of 7 to 12 atoms,
(9) azaspiro having 3 to 9 carbon atoms, said ring being saturated,
(10) tetrazolyl,
(11) pyrazolidinyl,
(12) dihydrodimethoxyisoquinolyl,
(13) azetidinyl, or
(14)diazabicyclo ring of 7-12 atoms,
wherein the substituents are each selected from the group consisting of hydrogen and C₁₋₃alkyl, benzyloxycarbonyl, carboxy, phenyl C₁₋₃alkyl amino carbonyl, pyrrolidinylmethyl, hydroxy C₁₋₃alkyl, C₁₋₆alkyloxy, C₁₋₄alkyloxy carbonyl, aminocarbonyl wherein the amino is optionally mono or di substituted with C₁₋₆alkyl, and oxo; or -N(R7)R8 may be a lysing residue; or
R₈ and R₉ are joined together to form a mono or di substituted saturated monocyclic ring of 6 to 7 atoms and having two hetero atoms which are the nitrogens to which R₈ and R₉ are attached; said rings to include piperazinyl and homopiperazinyl; or R₉ and R₁₀ are joined together to form a mono or di substituted monocyclic saturated ring of 5 to 7 atoms and having one hetero atom which is the nitrogen to which R₉ is attached; or
R₉ and R₁₂ are joined together to form a mono or di substituted saturated monocyclic ring of 5, 6, or 7 atoms, said ring having one hetero atom which is the nitrogen to which R₉ is attached; or
R₁₀ and R₁₂ are joined together to form a mono or di substituted saturated monocyclic ring of 5, 6, or 7 carbon atoms; or
R₈ and R₁₁ are joined together to form a mono or di substituted saturated monocyclic ring of 5, 6, or 7 atoms, said ring having one hetero atom which is the nitrogen to which R₈ is attached; and the substituents are independently selected from hydrogen and C₁₋₃alkyl.

2. A compound according to Claim 1 wherein
R is C₁₋₆alkyl;
R¹ is C₁₋₆alkyl or C₁₋₆alkoxy-C₁₋₆alkyl:
M is
(1) hydrogen,
(2) C₁₋₆alkyl,
(3) hydroxy C₁₋₆alkyl,
(4) halo C₁₋₆alkyl,
(5) C₂₋₆alkenyl, or
(6) C₁₋₆alkoxy-C₁₋₆alkyl;
Ra is
(1) hydrogen,
(2) C₁₋₆alkyl,
(3) halo,
(4) carboxy,
(5) C₁₋₆alkoxy,
(6) phenyl,
(7) C₁₋₆alkylcarbonyl,
(8) di-(C₁₋₆alkyl)amino;
Rb is hydrogen or C₁₋₆alkyl,
R² and R³ are each independently
(1) hydrogen,
(2) C₁₋₆alkyl,
(3) halo,
(4) carboxy,
(5) C₁₋₆alkoxy,
(6) phenyl,
(7) C₁₋₆alkylcarbonyl,
(8) di-(C₁₋₆alkyl)amino, or
R² and R³ may be joined together to form a methylenedioxy group or a furan ring;
R₄ is 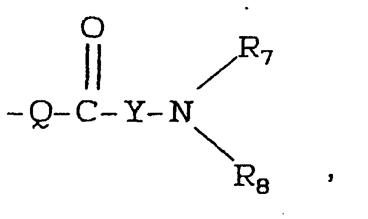 wherein
Q is a covalent bond,
Y is 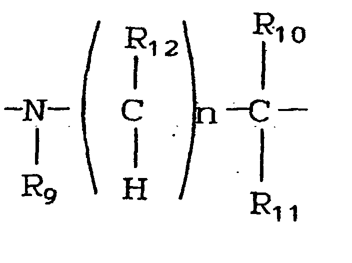
R₁₂ is hydrogen or C₁₋₃alkyl;
R₇ and R₈ are each individually
(a) hydrogen,
(b) C₁₋₆alkyl,
(c) C₁₋₆alkyloxy C₂₋₃alkyl,
(d) hydroxy C₂₋₆alkyl,
(e) carboxamido C₁₋₆alkyl,
(f) C₁₋₆alkanoyl,
(g) phenyl or phenyl C₁₋₆alkyl,
(h) C₂₋₆alkenyl,
(i) C₆₋₁₀cycloalkenyl,
(j) heteroaryl C₁₋₆alkyl wherein the heteroaryl is pyridinyl, imidazolyl, triazolyl, benzylimidazolyl, or furyl,
(k) carboxy C₁₋₆alkyl or carboC₁₋₆alkoxyC₁₋₆alkyl,
(l) C₁₋₆alkylsulfonyl,
(m) benzyloxy,
(n) morpholinyl C₁₋₃alkylsulfonyl,
(o) aminoC₁₋₃alkylsulfonyl wherein the amino may be optionally substituted with C₁₋₆alkyl,
(p) aminocarbonyl wherein the amino may be optionally substituted with C₁₋₆alkyl,
(q) aminocarbonyloxyC₁₋₆alkyl wherein the amino may be optionally substituted with C₁₋₆alkyl,
(r) di C₁₋₃alkyl amino C₁₋₆alkyl wherein the amino may be optionally substituted with C₁₋₆alkyl,
(s) pyrazolidinyl,
(t) piperidinyl,
(u) pyrrolidinyl
(v) pyrimidinyl,
(w) C₃₋₁₀cycloalkyl,
(x) α-C₁₋₃alkyl benzyl or mono or di substituted benzyl or mono or di substituted pyridylmethyl, wherein the substitutents are X₁ and X₂.
wherein
X₁ is
(1) hydrogen,
(2) halo,
(3) C₁₋₆alkyl,
(4) halo-C₁₋₆alkyl,
(5) C₂₋₆alkenyl,
(6) hydroxy-C₁₋₆alkyl,
(7) C₁₋₆alkylcarbonyl,
(8) C₁₋₆alkylcarbonylamino;
(9) di-C₁₋₃alkylamino; or
(10) carboxy,
X₂ is hydrogen, halo or C₁₋₆alkyl;
n is 1, 2, 3 or 4;
R₉ is selected from hydrogen, C₁₋₄ alkyl, and C₁₋₃alkoxyC₁₋₃alkyl; R₁₀ and R₁₁ are each independently selected from hydrogen, C₁₋₄alkyl, and C₁₋₃alkoxy C₁₋₃alkyl; or
R₇ and R₈ are joined together to form mono or di substituted ring selected from
(1) piperidinyl,
(2) piperazinyl,
(3) morpholinyl,
(4) pyrroylidinyl,
(5) pyrryl,
(6) imidazolyl,
(7) triazolyl,
(8) tetrayolyl,
(9) pyrazolidinyl, or
(10) azetidinyl,
wherein the substituents are each selected from the group consisting of hydrogen and C₁₋₃alkyl, benzyloxycarbonyl, carboxy, phenyl C₁₋₃alkyl amino carbonyl, pyrrolidinyl, methyl, hydroxy C₁₋₃alkyl, C₁₋₆alkyloxy, C₁₋₄alkyloxy carbonyl, and oxo; or R₈ and R₉ are joined together to form a mono or di substituted saturated monocyclic ring of 6 to 7 atoms and having two hetero atoms which are the nitrogens to which R₈ and R₉ are attached; said rings to include piperazinyl and homopiperazinyl; or R₉ and R₁₀ are joined together to form a mono or di substituted monocyclic saturated ring of 5 to 7 atoms and having one hetero atom which is the nitrogen to which R₉ is attached; or
R₉ and R₁₂ are joined together to form a mono or di substituted saturated monocyclic ring of 5, 6, or 7 atoms, said ring having one hetero atom which is the nitrogen to which R₉ is attached; or
R₁₀ and R₁₂ are joined together to form a mono or di substituted saturated monocyclic ring of 5, 6, or 7 carbon atoms; or
R₈ and R₁₁ are joined together to form a mono or di substituted saturated monocyclic ring of 5, 6, or 7 atoms, said ring having one hetero atom which is the nitrogen to which R₈ is attached; and the substituents are independently selected from hydrogen and C₁₋₃alkyl.

3. A compound according to Claim 2 wherein
R is C₁₋₃ alkyl;
R₁ is C₁₋₃ alkyl;
M is
(a) C₁₋₆ alkyl, or
(b) C₂₋₆ alkenyl;
R² is
(a) hydrogen
(b) C₁₋₆ alkyl, or C₁₋₆ alkoxy, and
R³ is hydrogen, or
R² and R³ are joined together to form a methylenedioxy group or a furan ring;
R₇ and R₈ are each independently selected from
(a) hydrogen,
(b) C₁₋₃ alkyl,
(c) C₁₋₃ alkoxy C₂₋₃ alkyl,
(d) C₃₋₇ cycloalkyl,
(e) hydroxyC₂₋₃ alkyl,
(f) carboC₁₋₄alkoxymethyl,
(g) substituted benzyl wherein the substituents are X₁ and X₂
wherein X₁ is hydrogen and X₂ is
(1) hydrogen,
(2) halo, or
(3) C₁₋₃ alkyl;
n is 1, 2 or 3, and
R₉, R₁₀ and R₁₁ are each independently selected from hydrogen, C₁₋₄ alkyl, and C₁₋₃ alkoxy C₁₋₃alkyl; or
R₇ and R₈ are joined together to form a substituted ring selected from
(a) piperidinyl,
(b) piperazinyl, and
(c) morpholinyl;
or
R₈ and R₉ are joined together to form a mono or di substituted saturated monocyclic ring of 6 to 7 atoms and having two hetero atoms which are the nitrogens to which R₈ and R₉ are attached.

4. A compound according to claim 1 of formula: 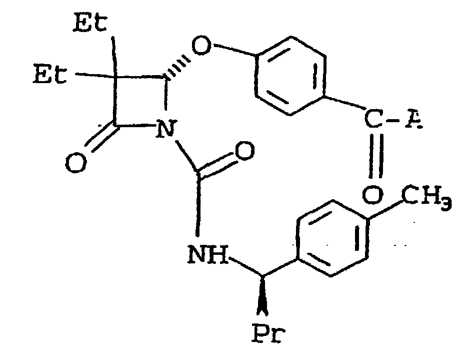 wherein A is:
-NHCH₂CH₂N(CH₃)₂
-N(CH₃)CH₂CH₂N(CH₃)₂
-N(Et)CH₂CH₂N(CH₃)₂
-NHCH₂CH₂N(Et)₂
-NHCH₂CH₂-(4-morpholinyl)
-N(CH₃)CH₂CH₂-(4-morpholinyl)
-N(CH₃)CH₂CH₂N(CH₂CH₂OCH₃)₂
-N(CH₃)CH₂CH₂N(Et)₂
-N(Ph)CH₂CH₂N(CH₃)₂
-N(CH₃)CH₂CH₂CH₂N(CH₃)₂
-NHCH₂CH₂N(i-Pr)₂
-N(CH₃)CH₂CH₂N(O)(CH₃)₂
-N(CH₃)CH₂CH₂N(i-Pr)₂
-NH-SO₂CH₂CH₂-(4-morpholinyl)
-NH-SO₂CH₂CH₂N(CH₃)₂
-NHCH₂CH₂-(4-imidazolyl)
-NHCH₂CH₂-(1-piperidinyl)
-N(CH₃)CH₂CH₂-(1-piperidinyl)
-N(CH₃)CH₂CH₂NHCH₃
-N(CH₃)CH₂CH₂N(CH₃)Ac
-NHCH₂CH₂-(1-pyrrolidinyl)
-N(CH₃)CH₂CH₂-(1-pyrrolidinyl)
-NHCH₂CH₂-(1H-1,2,4-triazol-1-yl)
-NH-CH₂CH₂-(1-imidazolyl)
-NH-CH₂CH₂-(3-azabicyclo-[3.2.2-non-3-yl)
-NH-CH₂CH₂-(3-azaspiro[5.5]-undec-3-yl)
-NH-CH₂CH₂-(2H-tetrazol-2-yl)
-NH-CH₂CH₂-(1H-tetrazol-1-yl)
-NHCH₂C(O)-Pro-NHCH₂Ph
-N(CH₃)CH₂CH₂-(3-azabicyclo-[3.2.2]non-3-yl)
-N(CH₃)CH₂CH₂-(4-imidazolyl)
-N(CH₃)CH₂CH₂N(CH₃)Ac
-N(CH₃)CH₂CH₂N(CH₃)C(O)NHCH₃
-N(CH₃)CH₂CH₂N(CH₃)SO₂CH₃
-N(CH₃)CH₂CH₂(3-azabicyclo-[3.2.2]-non-3-yl)
-NHCH₂CH₂-(1,1-dioxo-4-thiamorpholinyl)
-N(CH₃)CH₂CH₂-(1,1-dioxo-4-thiamorpholinyl)
4-dimethylaminoanilino
-NHCH₂CH₂-(1-benzyl-1H-imidazol-2-yl)
-N(CH₃)CH₂CH₂(2-pyridyl)
-N(CH₃)(1-azabicyclo[2.2.2]oct-3-yl
-NHCH₂CH₂(4-benzyloxycarbonyl-1-piperazinyl)
1,2-diethylpyrazolidin-4-ylamino
2-(1-S-pyrrolidinylmethyl)-1-pyrrolidinyl
-NHCH₂CH₂(4-hydroxy-1-piperidinyl)
-NHCH₂CH₂(1-homopiperidinyl)
-N(CH₃)CH₂CH₂(1-homopiperidinyl)
-NHCH₂CH₂(3-hydroxy-1-piperidinyl)
-N(CH₃)CH₂CH₂(3-hydroxy-1-piperidinyl)
-N(CH₃)CH₂CH₂N(CH₃)CH₂Ph
-N(CH₃)CH₂CH₂(4-benzyloxy-1-piperidinyl)
-N(CH₃)CH₂CH₂(4-hydroxy-1-piperidinyl)
-N(CH₃)CH₂CH₂(4-oxo-1-piperidinyl)
-NHCH₂CH₂(3-hydroxy-1-pyrrolidinyl)
-N(Et)CH₂CH₂(1-piperidinyl)
-N(CH₂Ph)CH₂CH₂(1-piperidinyl)
-N(CH₂Ph)CH₂CH₂N(CH₃)₂
-N(CH₃)CH₂CH₂(3-hydroxy-1-pyrrolidinyl)
-N(3-picolyl)CH₂CH₂(1-piperidinyl)
-NHCH(CH₃)CH₂CH₂CH₂N(Et)₂
-NHCH₂CH₂(2-S-hydroxymethyl-1-pyrrolidinyl)
-N(CH₃)CH₂CH₂(4-t-butoxycarbonyl-1-piperazinyl)
-N[CH₂CH₂N(CH₃)₂]₂
-N[CH₂CH₂N(Et)₂]₂
-N(CH₃)CH₂CH₂N(CH₃)(3-picolyl)
3,5-dimethyl-1-piperazinyl
-N(CH₃)CH₂CH₂N(O)(CH₃)CH₂Ph
-N(CH₃)CH₂CH₂N(CH₃)(4-picolyl)
2-S-(N-benzyl-N-methylaminomethyl)-1-pyrrolidinyl
-N(CH₃)CH₂CH₂N(CH₃)(2-picolyl)
-N(CH₃)CH₂CH₂(1-piperazinyl)
1-homopiperazinyl
-N(CH₃)CH₂CH₂N(CH₃)CH₂CH₂Ph
2-(1-R-pyrrolidinylmethyl)-1-pyrrolidinyl
4-benzyl-1-homopiperazinyl
-N(CH₃)CH₂CH₂N(CH₃)(1-naphthalenylmethyl)
-N(CH₃)CH₂CH₂N(CH₃)(2-naphthalenylmethyl)
-N(CH₃)CH₂CH₂N(CH₃)CH(CH₃)Ph
-N(CH₃)CH₂CH₂N(CH₂Ph)₂
1-ethyl-3-piperidinylamino
-N(CH₃)CH₂CH₂N(CH₃)(2-furfuryl)
-N(CH₃)CH₂CH₂CH₂C(O)NHSO₂Ph
-N(CH₃)CH₂CH₂N(CH₃)CH₂CH=CH₂
-N(CH₃)CH₂CH₂CH₂N(CH₃)CH₂Ph
-N(CH₃)-(CH₂)₆-N(CH₃)CH₂Ph
-N(CH₃)CH₂CH₂OC(O)N(CH₃)₂
-N(CH₃)CH₂CH₂N(CH₃)CH₂CO₂-t-Bu
-N(CH₃)(1-ethyl-3-piperidinyl)
-N(CH₃)CH₂CH₂N(CH₃)(tetrahydro-2H-pyran-2-yl-methyl)
2,2,6,6-tetramethylpiperidin-4-ylamino
-N(CH₃)CH₂CH₂N(CH₃)(4-cyanobenzyl)
-N(CH₃)CH₂CH₂N(CH₃)(4-methylbenzyl)
-N(CH₃)CH₂CH₂N(CH₃)(3-cyanobenzyl)
-N(CH₃)CH₂CH₂N(CH₃)(4-trifluoromethylbenzyl
-N(CH₃)CH₂CH₂N(CH₃)(3-trifluoromethylbenzyl)
-N(CH₃)CH₂CH₂N(CH₃)(cyclopropylmethyl)
-N(CH₃)CH₂CH(Ph)N(CH₃)₂
-N(CH₃)(1-benzyl-3-piperidinyl)
-N(3-picolyl)CH₂CH₂N(CH₃)CH₂PH
-N(CH₃)CH₂CH₂N(CH₃)(4-methoxybenzyl)
-N(4-picolyl)CH₂CH₂N(CH₃)CH₂Ph
-N(2-picolyl)CH₂CH₂N(CH₃)CH₂Ph
-N(CH₃)CH₂CH₂N(CH₃)(2,4-dimethylbenzyl)
-N(CH₃)CH₂CH₂(2,6-dimethyl-4-morpholinyl)
-N(CH₃)CH₂CH₂N(CH₃)C(=N-CN)NHPh
-N(CH₃)CH₂CH₂N(CH₃)(3-fluorobenzyl)
-N(CH₃)CH₂CH₂N(CH₃)(2-chlorobenzyl)
-N(CH₃)CH₂CH₂N(CH₃)(3-methoxybenzyl)
-N(CH₃)CH₂CH₂N(CH₃)(3,5-dimethoxybenzyl)
-N(CH₃)(1-benzyl-4-piperidinyl)
-N(CH₃)CH₂CH₂N(CH₃)(2-adamantyl)
-N(CH₃)(4-piperidinyl)
5-Methyl-2,5-diazabicyclo[2.2.1]hept-2-yl
-N(CH₃)CH₂CH₂CH₂N(CH₃)CH₂CH₃
-N(CH₃)(1-methyl-4-piperidinyl)
-N(CH₃)(1-propyl-4-piperidinyl)
-N(CH₃)(1-ethyl-4-piperidinyl)
-N(CH₃)CH₂CH(CH₃)N(CH₃)CH₂Ph
-N(CH₃)CH₂CH(CH₃)N(CH₃)₂
-N(CH₃)CH₂CH₂N(CH₃)(bicyclo[2.2.1]-hept-2-yl)
-N(CH₃)CH₂CH₂NH(2-adamantyl)
-N(CH₃)CH₂CH₂N(CH₃)(6,6-dimethylbicyclo-[3.1.1]hept-2-yl
-N(CH₃)CH₂CH₂N(CH₃)(bicyclo[3.2.1]-oct-2-yl)
-N(CH₃)CH₂CH₂N(CH₃)(1-cyclohexen-1-yl)
-N(CH₃)CH₂CH₂NHC(CH₃)₂CH=CH₂
2-S-carboxamido-1-pyrrolidinyl
2-hydroxymethyl-1-piperidinyl
3-dimethylamino-1-pyrrolidinyl
-N(CH₃)CH₂CH₂N(CH₃)(cyclohexylmethyl)
-N(CH₃)CH₂CH₂N(CH₂CH=CH₂)C(CH₃)₂CH=CH₂
-N(CH₃)CH₂CH₂N(CH₃)(4-ethylcyclohexyl)
-N(CH₃)CH₂CH₂N(CH₃)(2-ethylcyclohexyl)
-N(CH₃)CH₂CH₂N(CH₃)(4-methylcyclohexyl)
-N(CH₃)CH₂CH₂N(CH₃)(cyclohexyl)
-N(CH₃)CH₂CH₂N(CH₃)CH₂CO₂H-TFA
-N(CH₃)CH₂CH₂N(CH₃)CH₂C(O)N(CH₃₎₂
-N(CH)CH₂CH₂N(CH₃)(cyclohexylmethyl) or
-NHCH₂CH₂N(Et)CH₂CH₂OCH₃

5. A compound according to claim 1 of formula: 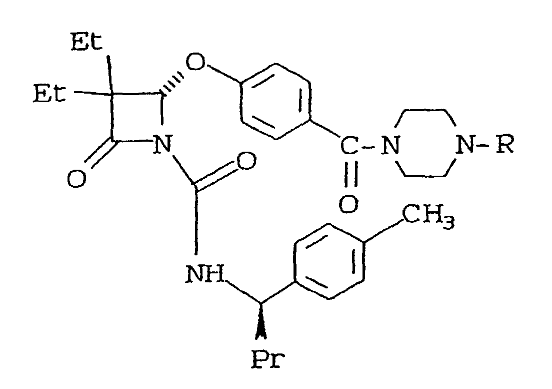 wherein R is:
-CH₃
4-fluorophenyl
3-chlorophenyl
phenyl
benzyl
H
i-Pr
i-Bu
-CH₂CO₂Et
-CH₂CO₂H
Et
Pr
2-pyrimidinyl
-CH₂CH₂OC(O)NHCH₃
cyclopropyl or
-CH₂CH₂OH

6. A compound according to claim 1 of formula: 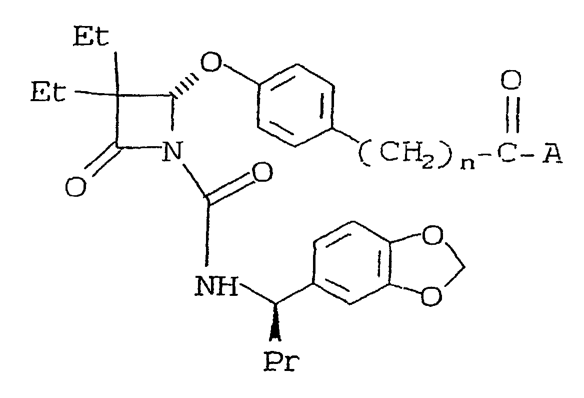 wherein n is 0 and A is:
-N(CH₃)CH₂CH₂N(CH₃)₂
4-methyl-1-piperazinyl
-N(CH₃)CH₂CH₂N(CH₃)CH₂Ph
4-cyclopropyl-1-piperazinyl
1-piperazinyl or
4-(2-hydroxyethyl)-1-piperazinyl

7. A compound according to claim 1 of formula: 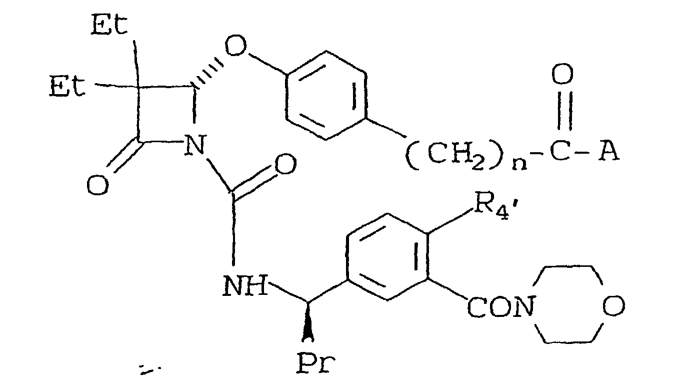 wherein n is 0 and R₄' and A are as shown below:
| R₄' | A |
|---|---|
| Et | -N(CH₃)CH₂CH₂N(CH₃)₂ |

8. A compound according to claim 1 of formula: 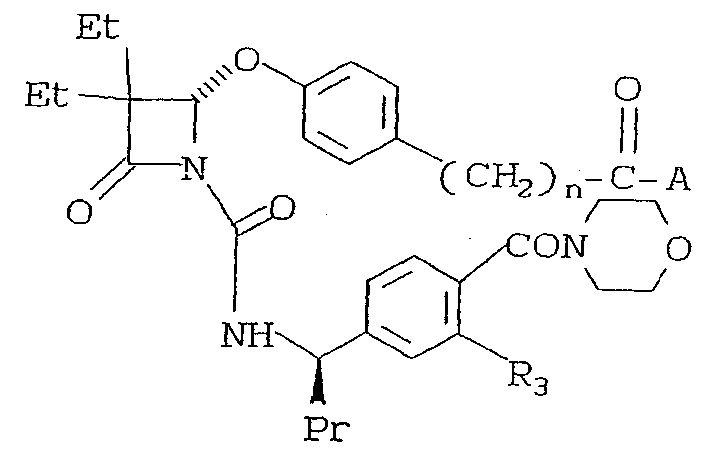 wherein n is 0 and R₃ and A are as shown below:
| R₃ | A |
|---|---|
| H | -N(CH₃)CH₂CH₂N(CH₃)₂ |

9. A compound according to claim I of formula: 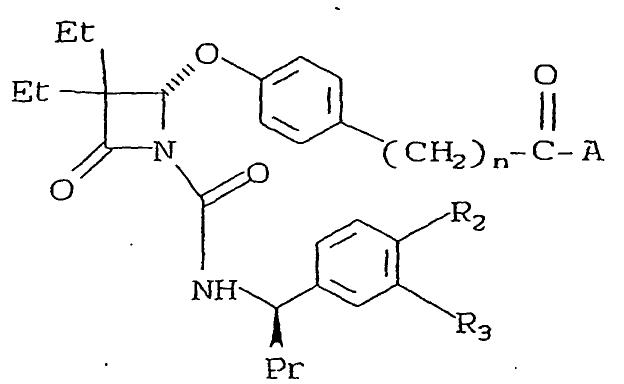 wherein n is 0 and R₂, R₃ and A are as shown below:
| R₃ | R₂ | A |
|---|---|---|
| H | -CON(CH₃)₂ | -N(CH₃)CH₂CH₂N(CH₃)₂ |
| H | -CO₂H | -N(CH₃)CH₂CH₂N(CH₃)₂ |
| H | -CH₂OH | -N(CH₃)CH₂CH₂N(CH₃)₂ |
| H | OCH₃ | -N(CH₃)CH₂CH₂N(CH₃)₂ |
| OCH₃ | H | -N(CH₃)CH₂CH₂N(CH₃)₂ |
| H | CN | -N(CH₃)CH₂CH₂N(CH₃)₂ |
| H | OEt | -N(CH₃)CH₂CH₂N(CH₃)CH₂Ph |
| H | 2-(4-morpholinocarbonylphenyl) | -N(CH₃)CH₂CH₂N(CH₃)CH₂Ph |
| H | OCH₃ | 4-methyl-1-piperazinyl |
| H | Cl | -N(CH₃)CH₂CH₂N(CH₃)₂ |
| H | Cl | -N(CH₃)CH₂CH₂N(Et)₂ |
| H | Cl | -N(CH₃)CH₂CH₂N(i-Pr)₂ |
| H | Cl | -N(CH₃)CH₂CH₂N(CH₃)CH₂Ph or |
| OCH₃ | CH₃ | -N(CH₃)CH₂CH₂N(CH₃)₂ |

10. A compound according to claim 1 of formula: 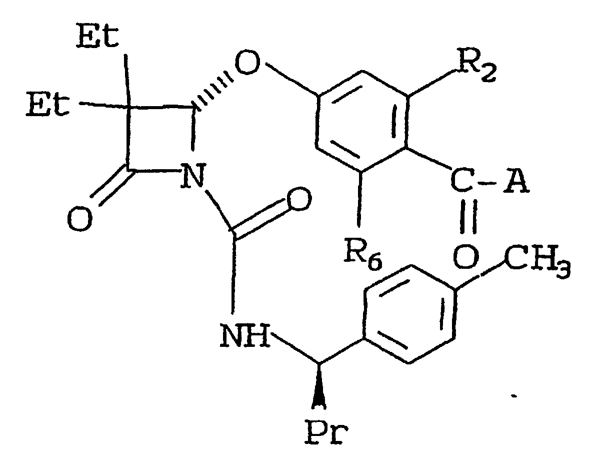 wherein R₂, R₆ and A are as shown below:
| R₂ | R₆ | A |
|---|---|---|
| CH₃ | CH₃ | -N(CH₃)CH₂CH₂N(CH₃)₂ |
| CH₃ | CH₃ | -N(CH₃)CH₂CH₂N(Et)₂ |
| CH₃ | CH₃ | -N(Et)CH₂CH₂N(CH₃)₂ or |
| H | OH | -N(CH₃)CH₂CH₂N(CH₃)₂ |

11. [S-(R*,S*)]-2-[4-[[(4-Methyl)piperazin-1-yl]carbonyl]phenoxy]-3,3-diethyl-N-[1-(3,4-methylenedioxyphenyl)butyl]-4-oxo-1-azetidinecarboxamide or a pharmaceutically acceptable salt thereof.

12. A pharmaceutical composition comprising a compound according to any previous claim and a pharmaceutically acceptable carrier.

13. The use of a compound according to any of claims 1-11 for the manufacture of a medicament for the inhibition of human leukocyte elastase.

## Patentansprüche

1. Eine Verbindung der Formel (I): 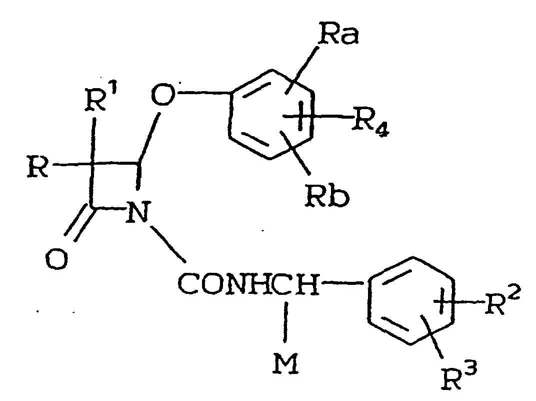 oder ein pharmazeutisch annehmbares Salz davon, wobei:
R C₁₋₆-Alkyl ist,
R¹ C₁₋₆-Alkyl oder C₁₋₆-Alkoxy-C₁₋₆-alkyl ist,
M ist
(1) Wasserstoff,
(2) C₁₋₆-Alkyl,
(3) Hydroxy-C₁₋₆-alkyl,
(4) Halogen-C₁₋₆-alkyl,
(S) C₂₋₆-Alkenyl oder
(6) C₁₋₆-Alkoxy-C₁₋₆-alkyl,
Ra und Rb jeweils unabhängig sind
(1) Wasserstoff,
(2) C₁₋₆-Alkyl,
(3) Halogen,
(4) Carboxy,
(5) C₁₋₆-Alkoxy,
(6) Phenyl,
(7) C₁₋₆-Alkylcarbonyl,
(8) Di-(C₁₋₆-alkyl)amino,
(9) Hydroxy,
R² und R³ jeweils unabhängig sind
(1) Wasserstoff,
(2) C₁₋₆-Alkyl,
(3) Halogen,
(4) Carboxy,
(5) C₁₋₆-Alkoxy,
(6) Phenyl,
(7) C₁₋₆-Alkylcarbonyl,
(8) Amino-C₂₋₃-alkyloxycarbonyl, wobei das Amino gegebenenfalls mit C₁₋₆-Alkyl mono- oder disubstituiert ist,
(9) Amino-C₂₋₃-alkylaminocarbonyl, wobei das Amino gegebenenfalls mit C₁₋₆-Alkyl mono- oder disubstituiert ist,
(10) Hydroxy,
(11) Aminocarbonyl, wobei das Amino gegebenenfalls mit C₁₋₆-Alkyl mono- oder disubstituiert ist,
(12) Hydroxymethyl,
(13) Aminocarbonyloxy-C₁₋₃-alkyl, wobei das Amino gegebenenfalls mit C₁₋₆-Alkyl mono- oder disubstituiert ist,
(14) Cyano,
(15) Morpholinocarbonylphenyl,
(16) Amino, wobei das Amino gegebenenfalls mit C₁₋₆-Alkyl mono- oder disubstituiert ist, oder R² und R³ miteinander verbunden sein können, um eine Methylendioxygruppe oder einen Furanring zu bilden,
(17) Morpholinocarbonyl,
R₄ ist 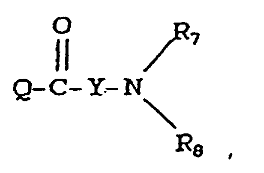
wobei
Q eine kovalente Bindung ist und
Y 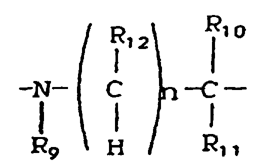 ist,
R₁₂ Wasserstoff oder C₁₋₃-Alkyl ist,
R₇ und R₈ jeweils unabhängig sind
(a) Wasserstoff,
(b) C₁₋₆-Alkyl,
(c) C₁₋₆-Alkyloxy-C₂₋₃-alkyl,
(d) Hydroxy-C₂₋₆-alkyl,
(e) Polyhydroxy-C₂₋₆-alkyl,
(f) Carboxamido-C₁₋₆-alkyl,
(h) C₁₋₆-Alkanoyl,
(i) substituiertes Phenyl oder Phenyl-C₁₋₆-alkyl, wobei die Substituenten X₁ und X₂ sind, wie sie unmittelbar nachstehend definiert sind,
(j) C₂₋₆-Alkenyl,
(k) C₆₋₁₀-Cycloalkenyl,
(l) Heteroaryl-C₁₋₆-alkyl, wobei das Heteroaryl Pyridinyl, Imidazolyl, Triazolyl, Benzylimidazolyl oder Furyl ist,
(m) Carboxy-C₁₋₆-alkyl,
(n) Carbo-C₁₋₆-alkoxy-C₁₋₃-alkyl,
(o) Phenylsulfonyl,
(p) C₁₋₆-Alkylsulfonyl,
(q) Benzyloxy,
(r) Morpholinyl-C₁₋₃-alkylsulfonyl,
(s) Tetrahydropyranyl,
(t) Amino-C₁₋₃-alkylsulfonyl, wobei das Amino gegebenenfalls mit C₁₋₆-Alkyl mono- oder disubstituiert ist,
(u) Aminocarbonyl, wobei das Amino gegebenenfalls mit C₁₋₆-Alkyl mono- oder disubstituiert ist,
(v) Aminocarbonyloxy-C₂₋₆-alkyl, wobei das Amino gegebenenfalls mit C₁₋₆-Alkyl mono- oder disubstituiert ist,
(w) Azabicyclo mit 7 bis 12 Atomen,
(x) Di-C₁₋₃-alkylamino-C₂₋₆-alkyl, wobei das Amino gegebenenfalls mit C₁₋₆-Alkyl mono- oder disubstituiert ist,
(y) Bicycloalkyl mit 7 bis 12 Atomen,
(z) C₃₋₁₀-Cycloalkyl, gegebenenfalls mit C₁₋₆-Alkyl substituiert,
(aa) Pyrazolidinyl,
(bb) substituiertes Piperidinyl oder Pyrrolidinyl, wobei der Substituent Wasserstoff, C₁₋₃-Alkyl, Hydroxy-C₁₋₃-alkylbenzyl, Carboxamido oder Amino ist, wobei das Amino gegebenenfalls mit C₁₋₆-Alkyl mono- oder disubstituiert ist,
(cc) substituiertes Pyrrolidinyl, wobei der Substituent Carboxamido oder Amino ist, wobei das Amino gegebenenfalls mit C₁₋₆-Alkyl mono- oder disubstituiert ist,
(dd) Pyrimidinyl,
(ee) N-Cyano-N'-phenylamidino,
(ff) Phosphono-C₁₋₆-alkyl oder
(gg) α-C₁₋₃-Alkylbenzyl oder mono- oder disubstituiertes Benzyl oder mono- oder disubstituiertes Pyridylmethyl,
wobei die Substituenten X₁ und X₂ sind,
wobei
X₁ ist
(1) Wasserstoff,
(2) Halogen,
(3) C₁₋₆-Alkyl,
(4) Halogen-C₁₋₆-alkyl,
(5) C₂₋₆-Alkenyl,
(6) Hydroxy-C₁₋₆-alkyl,
(7) C₁₋₆-Alkylcarbonyl,
(8) C₁₋₆-Alkylcarbonylamino,
(9) CN,
(10) CF₃,
(11) CH₃O,
(12) Amino, wobei das Amino gegebenenfalls mit C₁₋₆-Alkyl mono- oder disubstituiert ist,
(13) Carboxy oder
(14) Phenylsulfonylaminocarbonyl,
X₂ Wasserstoff, Halogen oder C₁₋₆-Alkyl ist,
n 1, 2, 3, 4 oder 5 ist,
R₉ ausgewählt ist aus Wasserstoff, C₁₋₄-Alkyl und C₁₋₃-Alkoxy-C₁₋₃-alkyl oder Phenyl, Phenyl-C₁₋₃-alkyl, Pyridyl und Pyridyl-C₁₋₃-alkyl,
R₁₀ und R₁₁ jeweils unabhängig ausgewählt sind aus Wasserstoff, C₁₋₄-Alkyl und C₁₋₃-Alkoxy-C₁₋₃-alkyl oder zusammen O= sind, oder
R₇ und R₈ miteinander verbunden sind, um einen mono- oder disubstituierten Ring mit 4, 5, 6 oder 7 Atomen oder 7 bis 12 Atomen zu bilden, wie z.B.
(1) Piperidinyl oder Homopiperidinyl,
(2) Piperazinyl,
(3) Morpholinyl, Thiomorpholinyl oder 1,1-Dioxo-4-thiomorpholinyl,
(4) Pyrrolidinyl,
(5) Pyrryl,
(6) Imidazolyl,
(7) Triazolyl,
(8) gesättigtes Azabicyclo mit 7 bis 12 Atomen,
(9) Azaspiro mit 3 bis 9 Kohlenstoffatomen, wobei der Ring gesättigt ist,
(10) Tetrazolyl,
(11) Pyrazolidinyl,
(12) Dihydrodimethoxyisochinolinyl,
(13) Azetidinyl oder
(14) Diazabicycloring mit 7-12 Atomen,
wobei die Substituenten jeweils ausgewählt sind aus der Gruppe, bestehend aus Wasserstoff und C₁₋₃-Alkyl, Benzyloxycarbonyl, Carboxy, Phenyl-C₁₋₃alkylaminocarbonyl, Pyrrolidinylmethyl, Hydroxy-C₁₋₃-alkyl, C₁₋₆-Alkyloxy, C₁₋₄-Alkyloxycarbonyl, Aminocarbonyl, wobei das Amino gegebenenfalls mit C₁₋₆-Alkyl mono- oder disubstituiert ist, und Oxo, oder -N(R7)R8 ein Lysinrest sein kann oder
R₈ und R₉ miteinander verbunden sind, um einen mono- oder disubstituierten gesättigten monocyclischen Ring mit 6 bis 7 Atomen und mit zwei Heteroatomen, welche die Stickstoffatome sind, an die R₈ und R₉ gebunden sind, zu bilden, wobei die Ringe Piperazinyl und Homopiperazinyl umfassen, oder
R₉ und R₁₀ miteinander verbunden sind, um einen mono- oder disubstituierten monocyclischen gesättigten Ring mit 5 bis 7 Atomen und mit 1 Heteroatom, welches das Stickstoffatom ist, an das R, gebunden ist, zu bilden, oder
R₉ und R₁₂ miteinander verbunden sind, um einen mono- oder disubstituierten gesättigten monocyclischen Ring mit 5, 6 oder 7 Atomen zu bilden, wobei der Ring 1 Heteroatom besitzt, welches das Stickstoffatom ist, an das R, gebunden ist, oder
R₁₀ und R₁₂ miteinander verbunden sind, um einen mono- oder disubstituierten gesättigten monocyclischen Ring mit 5, 6 oder 7 Kohlenstoffatomen zu bilden, oder
R₈ und R₁₁ miteinander verbunden sind, um einen mono- oder disubstituierten gesättigten monocyclischen Ring mit 5, 6 oder 7 Atomen zu bilden, wobei der Ring 1 Heteroatom besitzt, welches das Stickstoffatom ist, an das R₈ gebunden ist, und wobei die Substituenten unabhängig ausgewählt sind aus Wasserstoff und C₁₋₃-Alkyl.

2. Eine Verbindung gemäß Anspruch 1, wobei
R C₁₋₆-Alkyl ist,
R¹ C₁₋₆-Alkyl oder C₁₋₆-Alkoxy-C₁₋₆-alkyl ist,
M ist
(1) Wasserstoff,
(2) C₁₋₆-Alkyl,
(3) Hydroxy-C₁₋₆-alkyl,
(4) Halogen-C₁₋₆-alkyl,
(5) C₂₋₆-Alkenyl oder
(6) C₁₋₆-Alkoxy-C₁₋₆-alkyl,
Ra ist
(1) Wasserstoff,
(2) C₁₋₆-Alkyl,
(3) Halogen,
(4) Carboxy,
(5) C₁₋₆-Alkoxy,
(6) Phenyl,
(7) C₁₋₆-Alkylcarbonyl,
(8) Di-(C₁₋₆-alkyl)amino,
Rb Wasserstoff oder C₁₋₆-Alkyl ist,
R² und R³ jeweils unabhängig sind
(1) Wasserstoff,
(2) C₁₋₆-Alkyl,
(3) Halogen,
(4) Carboxy,
(5) C₁₋₆-Alkoxy,
(6) Phenyl,
(7) C₁₋₆-Alkylcarbonyl,
(8) Di- (C₁₋₆-alkyl)amino oder
R₂ und R₃ miteinander verbunden sein können, um eine Methylendioxygruppe oder einen Furanring zu bilden,
R₄ ist 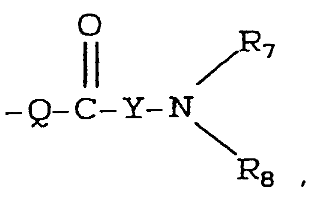
wobei
Q eine kovalente Bindung ist,
Y 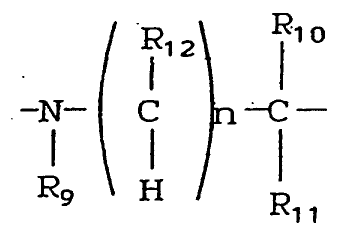 ist,
R₁₂ Wasserstoff oder C₁₋₃-Alkyl ist,
R₇ und R₈ jeweils unabhängig sind
(a) Wasserstoff,
(b) C₁₋₆-Alkyl,
(c) C₁₋₆-Alkyloxy-C₂₋₃-alkyl,
(d) Hydroxy-C₂₋₆-alkyl,
(e) Carboxamido-C₁₋₆-alkyl,
(f) C₁₋₆-Alkanoyl,
(g) Phenyl oder Phenyl-C₁₋₆-alkyl,
(h) C₂₋₆-Alkenyl,
(i) C₆₋₁₀-Cycloalkenyl,
(j) Heteroaryl-C₁₋₆-alkyl, wobei das Heteroaryl Pyridinyl, Imidazolyl, Triazolyl, Benzylimidazolyl oder Furyl ist,
(k) Carboxy-C₁₋₆-alkyl oder Carbo-C₁₋₆-alkoxy-C₁₋₆-alkyl,
(l) C₁₋₆-Alkylsulfonyl,
(m) Benzyloxy,
(n) Morpholinyl-C₁₋₃-alkylsulfonyl,
(o) Amino-C₁₋₃-alkylsulfonyl, wobei das Amino gegebenenfalls mit C₁₋₆-Alkyl substituiert sein kann,
(p) Aminocarbonyl, wobei das Amino gegebenenfalls mit C₁₋₆-Alkyl substituiert sein kann,
(q) Aminocarbonyloxy-C₁₋₆-alkyl, wobei das Amino gegebenenfalls mit C₁₋₆-Alkyl substituiert sein kann,
(r) Di-C₁₋₃-alkylamino-C₁₋₆-alkyl, wobei das Amino gegebenenfalls mit C₁₋₆-Alkyl substituiert sein kann,
(s) Pyrazolidinyl,
(t) Piperidinyl,
(u) Pyrrolidinyl,
(v) Pyrimidinyl,
(w) C₃₋₁₀-Cycloalkyl,
(x) α-C₁₋₃-Alkylbenzyl oder mono- oder disubstituiertes Benzyl oder mono- oder disubstituiertes Pyridylmethyl, wobei die Substituenten X₁ und X₂ sind,
wobei
X₁ ist
(1) Wasserstoff,
(2) Halogen,
(3) C₁₋₆-Alkyl,
(4) Halogen-C₁₋₆-alkyl,
(5) C₂₋₆-Alkenyl,
(6) Hydroxy-C₁₋₆-alkyl,
(7) C₁₋₆-Alkylcarbonyl,
(8) C₁₋₆-Alkylcarbonylamino,
(9) Di-C₁₋₃-alkylamino oder
(10) Carboxy,
X₂ Wasserstoff, Halogen oder C₁₋₆-Alkyl ist,
n 1, 2, 3 oder 4 ist,
R₉ ausgewählt ist aus Wasserstoff, C₁₋₄-Alkyl und C₁₋₃-Alkoxy-C₁₋₃-alkyl,
R₁₀ und R₁₁ jeweils unabhängig ausgewählt sind aus Wasserstoff, C₁₋₄-Alkyl und C₁₋₃-Alkoxy-C₁₋₃-alkyl, oder
R₇ und R₈ miteinander verbunden sind, um einen mono- oder disubstituierten Ring zu bilden, ausgewählt aus
(1) Piperidinyl,
(2) Piperazinyl,
(3) Morpholinyl,
(4) Pyrrolidinyl,
(5) Pyrryl,
(6) Imidazolyl,
(7) Triazolyl,
(8) Tetrazolyl,
(9) Pyrazolidinyl oder
(10) Azetidinyl,
wobei die Substituenten jeweils ausgewählt sind aus der Gruppe, bestehend aus Wasserstoff und C₁₋₃-Alkyl, Benzyloxycarbonyl, Carboxy, Phenyl-C₁₋₃alkylaminocarbonyl, Pyrrolidinyl, Methyl, Hydroxy-C₁₋₃-alkyl, C₁₋₆-Alkyloxy, C₁₋₄-Alkyloxycarbonyl und Oxo, oder
R₈ und R₉ miteinander verbunden sind, um einen mono- oder disubstituierten gesättigten monocyclischen Ring mit 6 bis 7 Atomen und mit zwei Heteroatomen, welche die Stickstoffatome sind, an die R₈ und R₉ gebunden sind, zu bilden, wobei die Ringe Piperazinyl und Homopiperazinyl umfassen, oder
R₉ und R₁₀ miteinander verbunden sind, um einen mono- oder disubstituierten monocyclischen gesättigten Ring mit 5 bis 7 Atomen und mit 1 Heteroatom, welches das Stickstoffatom ist, an das R₉ gebunden ist, zu bilden, oder
R₉ und R₁₂ miteinander verbunden sind, um einen mono- oder disubstituierten gesättigten monocyclischen Ring mit 5, 6 oder 7 Atomen zu bilden, wobei der Ring 1 Heteroatom besitzt, welches das Stickstoffatom ist, an das R₉ gebunden ist, oder
R₁₀ und R₁₂ miteinander verbunden sind, um einen mono- oder disubstituierten gesättigten monocyclischen Ring mit 5, 6 oder 7 Kohlenstoffatomen zu bilden, oder
R₈ und R₁₁ miteinander verbunden sind, um einen mono- oder disubstituierten gesättigten monocyclischen Ring mit 5, 6 oder 7 Atomen zu bilden, wobei der Ring 1 Heteroatom besitzt, welches das Stickstoffatom ist, an das R₈ gebunden ist, und wobei die Substituenten unabhängig ausgewählt sind aus Wasserstoff und C₁₋₃-Alkyl.

3. Eine Verbindung gemäß Anspruch 2, wobei
R C₁₋₃-Alkyl ist,
R₁ C₁₋₃-Alkyl ist,
M ist
(a) C₁₋₆-Alkyl oder
(b) C₂₋₆-Alkenyl,
R² ist
(a) Wasserstoff,
(b) C₁₋₆-Alkyl oder C₁₋₆-Alkoxy und
R³ Wasserstoff ist oder
R² und R³ miteinander verbunden sind, um eine Methylendioxygruppe oder einen Furanring zu bilden,
R₇ und R₈ jeweils unabhängig ausgewählt sind aus
(a) Wasserstoff,
(b) C₁₋₃-Alkyl,
(c) C₁₋₃-Alkoxy-C₂₋₃-alkyl,
(d) C₃₋₇-Cycloalkyl,
(e) Hydroxy-C₂₋₃-alkyl,
(f) Carbo-C₁₋₄-alkoxymethyl,
(g) substituiertem Benzyl, wobei die Substituenten X₁ und X₂ sind,
wobei X₁ Wasserstoff ist und X₂ ist
(1) Wasserstoff,
(2) Halogen oder
(3) C₁₋₃-Alkyl,
n 1, 2 oder 3 ist und
R₉, R₁₀ und R₁₁ jeweils unabhängig ausgewählt sind aus Wasserstoff, C₁₋₄-Alkyl und C₁₋₃-Alkoxy-C₁₋₃-alkyl, oder
R₇ und R₈ miteinander verbunden sind, um einen substituierten Ring zu bilden, ausgewählt aus
(a) Piperidinyl,
(b) Piperazinyl und
(c) Morpholinyl,
oder
R₈ und R₉ miteinander verbunden sind, um einen mono- oder disubstituierten gesättigten monocyclischen Ring mit 6 bis 7 Atomen und mit zwei Heteroatomen, welche die Stickstoffatome sind, an die R₈ und R₉ gebunden sind, zu bilden.

4. Eine Verbindung gemäß Anspruch 1 der Formel: 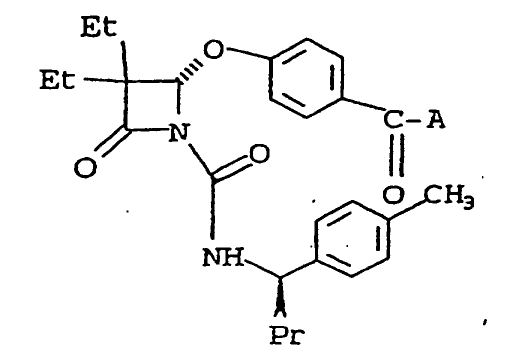 wobei A ist:
-NHCH₂CH₂N(CH₃)₂
-N(CH₃)CH₂CH₂N(CH₃)₂
-N(Et)CH₂CH₂N(CH₃)₂
-NHCH₂CH₂N(Et)₂
-NHCH₂CH₂-(4-Morpholinyl)
-N(CH₃)CH₂CH₂-(4-Morpholinyl)
-N(CH₃)CH₂CH₂N(CH₂CH₂OCH₃)₂
-N(CH₃)CH₂CH₂N(Et)₂
-N(Ph)CH₂CH₂N(CH₃)₂
-N(CH₃)CH₂CH₂CH₂N(CH₃)₂
-NHCH₂CH₂N(i-Pr)₂
-N(CH₃)CH₂CH₂N(O)(CH₃)₂
-N(CH₃)CH₂CH₂N(i-Pr)₂
-NH-SO₂CH₂CH₂-(4-Morpholinyl)
-NH-SO₂CH₂CH₂N(CH₃)₂
-NHCH₂CH₂-(4-Imidazolyl)
-NHCH₂CH₂-(1-Piperidinyl)
-N(CH₃)CH₂CH₂-(1-Piperidinyl)
-N(CH₃)CH₂CH₂NHCH₃
-N(CH₃)CH₂CH₂N(CH₃)Ac
-NHCH₂CH₂-(1-Pyrrolidinyl)
-N(CH₃)CH₂CH₂-(1-Pyrrolidinyl)
-NHCH₂CH₂-(1H-1,2,4-Triazol-1-yl)
-NH-CH₂CH₂-(1-Imidazolyl)
-NH-CH₂CH₂-(3-Azabicyclo-[3.2.2]-non-3-yl)
-NH-CH₂CH₂-(3-Azaspiro[5.5]undec-3-yl)
-NH-CH₂CH₂-(2H-Tetrazol-2-yl)
-NH-CH₂CH₂-(1H-Tetrazol-1-yl)
-NHCH₂C(O)-Pro-NHCH₂Ph
-N(CH₃)CH₂CH₂-(3-Azabicyclo[3.2.2]non-3-yl)
-N(CH₃)CH₂CH₂-(4-Imidazolyl)
-N(CH₃)CH₂CH₂N(CH₃)Ac
-N(CH₃)CH₂CH₂N(CH₃)C (O)NHCH₃
-N(CH₃)CH₂CH₂N(CH₃)SO₂CH₃
-N(CH₃)CH₂CH₂(3-Azabicyclo[3.2.2]non-3-yl)
-NHCH₂CH₂-(1,1-Dioxo-4-thiamorpholinyl)
-N(CH₃)CH₂CH₂-(1,1-Dioxo-4-thiamorpholinyl)
4-Dimethylaminoanilino
-NHCH₂CH₂-(1-Benzyl-1H-imidazol-2-yl)
-N(CH₃)CH₂CH₂(2-Pyridyl)
-N(CH₃)(1-Azabicyclo[2.2.2]oct-3-yl
-NHCH₂CH₂(4-Benzyloxycarbonyl-1-piperazinyl)
1,2-Diethylpyrazolidin-4-ylamino
2-(1-S-Pyrrolidinylmethyl)-1-pyrrolidinyl
-NHCH₂CH₂(4-Hydroxy-1-piperidinyl)
-NHCH₂CH₂(1-Homopiperidinyl)
-N(CH₃)CH₂CH₂(1-Homopiperidinyl)
-NHCH₂CH₂(3-Hydroxy-1-piperidinyl)
-N(CH₃)CH₂CH₂(3-Hydroxy-1-piperidinyl)
-N(CH₃)CH₂CH₂N(CH₃)CH₂Ph
-N(CH₃)CH₂CH₂(4-Benzyloxy-1-piperidinyl)
-N(CH₃)CH₂CH₂(4-Hydroxy-1-piperidinyl)
-N(CH₃)CH₂CH₂(4-Oxo-1-piperidinyl)
-NHCH₂CH₂(3-Hydroxy-1-pyrrolidinyl)
-N(Et)CH₂CH₂(1-Piperidinyl)
-N(CH₂Ph)CH₂CH₂(1-Piperidinyl)
-N(CH₂Ph)CH₂CH₂N(CH₃)₂
-N(CH₃)CH₂CH₂(3-Hydroxy-1-pyrrolidinyl)
-N(3-Picolyl)CH₂CH₂(1-piperidinyl)
-NHCH(CH₃)CH₂CH₂CH₂N(Et)₂
-NHCH₂CH₂(2-S-Hydroxymethyl-1-pyrrolidinyl)
-N(CH₃)CH₂CH₂(4-t-Butoxycarbonyl-1-piperazinyl)
-N[CH₂CH₂N(CH₃)₂]₂
-N[CH₂CH₂N(Et)₂]₂
-N(CH₃)CH₂CH₂N(CH₃)(3-Picolyl)
3,5-Dimethyl-1-piperazinyl
-N(CH₃)CH₂CH₂N(O)(CH₃)CH₂Ph
-N(CH₃)CH₂CH₂N(CH₃)(4-Picolyl)
2-S-(N-Benzyl-N-methylaminomethyl)-1-pyrrolidinyl
-N(CH₃)CH₂CH₂N(CH₃)(2-Picolyl)
-N(CH₃)CH₂CH₂(1-Piperazinyl)
1-Homopiperazinyl
-N(CH₃)CH₂CH₂N(CH₃)CH₂CH₂Ph
2-(1-R-Pyrrolidinylmethyl)-1-pyrrolidinyl
4-Benzyl-1-homopiperazinyl
-N(CH₃)CH₂CH₂N(CH₃)(1-Naphthalinylmethyl)
-N(CH₃)CH₂CH₂N(CH₃)(2-Naphthalinylmethyl)
-N(CH₃)CH₂CH₂N(CH₃)CH(CH₃)Ph
-N(CH₃)CH₂CH₂N(CH₂Ph)₂
1-Ethyl-3-piperidinylamino
-N(CH₃)CH₂CH₂N(CH₃)(2-Furfuryl)
-N(CH₃)CH₂CH₂CH₂C(O)NHSO₂Ph
-N(CH₃)CH₂CH₂N(CH₃)CH₂CH=CH₂
-N(CH₃)CH₂CH₂CH₂N(CH₃)CH₂Ph
-N(CH₃)-(CH₂)₆-N(CH₃)CH₂Ph
-N(CH₃)CH₂CH₂OC(O)N(CH₃)₂
-N(CH₃)CH₂CH₂N(CH₃)CH₂CO₂-t-Bu
-N(CH₃)(1-Ethyl-3-piperidinyl)
-N(CH₃)CH₂CH₂N(CH₃)(Tetrahydro-2H-pyran-2-ylmethyl)
2,2,6,6-Tetramethylpiperidin-4-ylamino
-N(CH₃)CH₂CH₂N(CH₃)(4-Cyanobenzyl)
-N(CH₃)CH₂CH₂N(CH₃)(4-Methylbenzyl)
-N(CH₃)CH₂CH₂N(CH₃)(3-Cyanobenzyl)
-N(CH₃)CH₂CH₂N(CH₃)(4-Trifluormethylbenzyl)
-N(CH₃)CH₂CH₂N(CH₃)(3-Trifluormethylbenzyl)
-N(CH₃)CH₂CH₂N(CH₃)(Cyclopropylmethyl)
-N-(CH₃)CH₂CH(Ph)N(CH₃)₂
-N(CH₃)(1-Benzyl-3-piperidinyl)
-N(3-Picolyl)CH₂CH₂N(CH₃)CH₂Ph
-N(CH₃)CH₂CH₂N(CH₃)(4-methoxybenzyl)
-N(4-Picolyl)CH₂CH₂N(CH₃)CH₂Ph
-N(2-Picolyl)CH₂CH₂N(CH₃)CH₂Ph
-N(CH₃)CH₂CH₂N(CH₃)(2,4-Dimethylbenzyl)
-N(CH₃)CH₂CH₂(2,6-Dimethyl-4-morpholinyl)
-N(CH₃)CH₂CH₂N(CH₃)C(=N-CN)NHPh
-N(CH₃)CH₂CH₂N(CH₃)(3-Fluorbenzyl)
-N(CH₃)CH₂CH₂N(CH₃)(2-Chlorbenzyl)
-N(CH₃)CH₂CH₂N(CH₃)(3-Methoxybenzyl)
-N(CH₃)CH₂CH₂N(CH₃)(3,5-Dimethoxybenzyl)
-N(CH₃)(1-Benzyl-4-piperidinyl)
-N(CH₃)CH₂CH₂N(CH₃)(2-Adamantyl)
-N(CH₃)(4-Piperidinyl)
5-Methyl-2,5-diazabicyclo[2.2.1]hept-2-yl
-N(CH₃)CH₂CH₂CH₂N(CH₃)CH₂CH₃
-N(CH₃)(1-Methyl-4-piperidinyl)
-N(CH₃)(1-Propyl-4-piperidinyl)
-N(CH₃)(1-Ethyl-4-piperidinyl)
-N(CH₃)CH₂CH(CH₃)N(CH₃)CH₂Ph
-N(CH₃)CH₂CH(CH₃)N(CH₃)₂
-N(CH₃)CH₂CH₂N(CH₃)(Bicyclo[2.2.1]hept-2-yl)
-N(CH₃)CH₂CH₂NH(2-Adamantyl)
-N(CH₃)CH₂CH₂N(CH₃)(6,6-dimethylbicyclo[3.1.1]hept-2-yl
-N(CH₃)CH₂CH₂N(CH₃)(Bicyclo[3.2.1]oct-2-yl)
-N(CH₃)CH₂CH₂N(CH₃)(1-Cyclohexen-1-yl)
-N(CH₃)CH₂CH₂NHC(CH₃)₂CH=CH₂
2-S-Carboxamido-1-pyrrolidinyl
2-Hydroxymethyl-1-piperidinyl
3-Dimethylamino-1-pyrrolidinyl
-N(CH₃)CH₂CH₂N(CH₃)(Cyclohexylmethyl)
-N(CH₃)CH₂CH₂N(CH₂CH=CH₂)C(CH₃)₂CH=CH₂
-N(CH₃)CH₂CH₂N(CH₃)(4-Ethylcyclohexyl)
-N(CH₃)CH₂CH₂N(CH₃)(2-Ethylcyclohexyl)
-N(CH₃)CH₂CH₂N(CH₃)(4-Methylcyclohexyl)
-N(CH₃)CH₂CH₂N(CH₃)(Cyclohexyl)
-N(CH₃)CH₂CH₂N(CH₃)CH₂CO₂H·TFA
-N(CH₃)CH₂CH₂N(CH₃)CH₂C(O)N(CH₃)₂
-N(CH)CH₂CH₂N(CH₃)(Cyclohexylmethyl) oder
-NHCH₂CH₂N(Et)CH₂CH₂OCH₃.

5. Eine Verbindung gemäß Anspruch 1 der Formel: 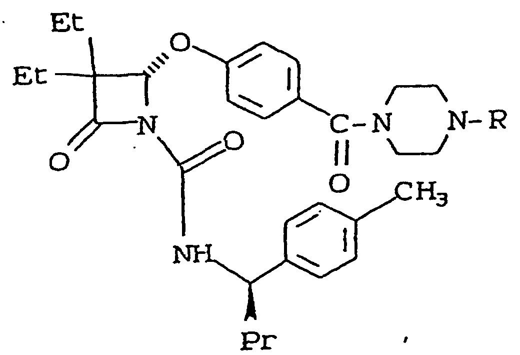 wobei R ist:
-CH₃
4-Fluorphenyl
3-Fluorphenyl
Phenyl
Benzyl
H
i-Pr
i-Bu
-CH₂CO₂Et
-CH₂CO₂H
Et
Pr
2-Pyrimidinyl
-CH₂CH₂OC(O)NHCH₃
Cyclopropyl oder
- CH₂CH₂OH.

6. Eine Verbindung gemäß Anspruch 1 der Formel: 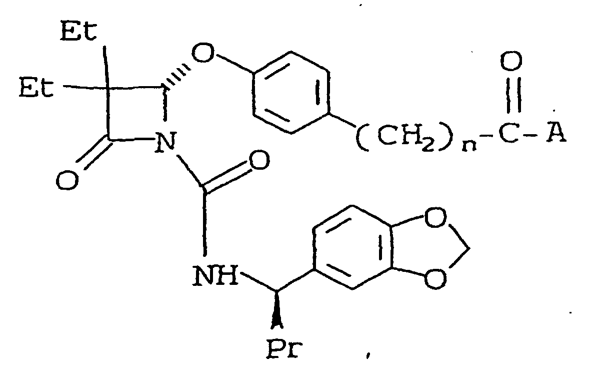 wobei n 0 ist und A ist:
-N(CH₃)CH₂CH₂N(CH₃)₂
4-Methyl-1-piperazinyl
-N(CH₃)CH₂CH₂N(CH₃)CH₂Ph
4-Cyclopropyl-1-piperazinyl
1-Piperazinyl oder
4-(2-Hydroxyethyl)-1-piperazinyl.

7. Ein Verbindung gemäß Anspruch 1 der Formel: 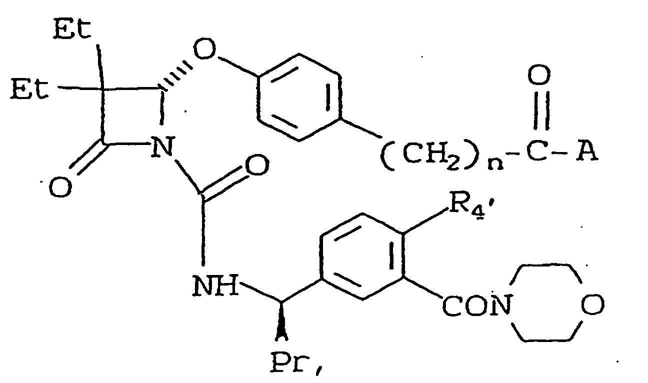 wobei n 0 ist und R₄' und A wie nachstehend gezeigt sind:
| R₄' | A |
|---|---|
| Et | -N (CH₃)CH₂CH₂N(CH₃)₂. |

8. Eine Verbindung gemäß Anspruch 1 der Formel: 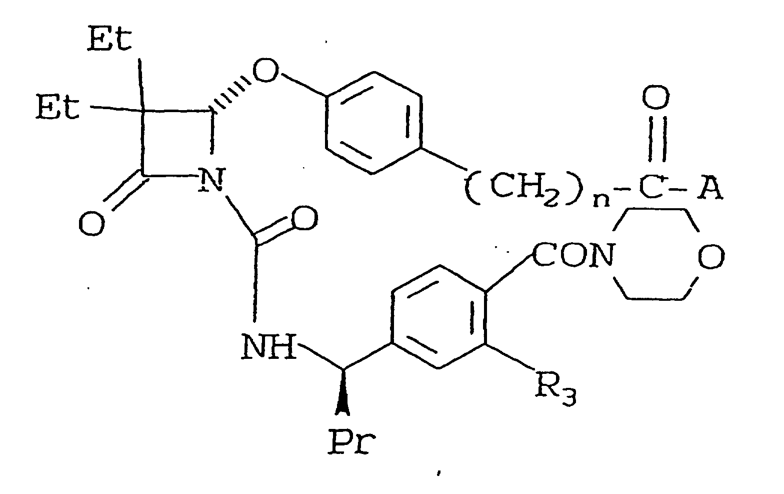 wobei n 0 ist und R₃ und A wie nachstehend gezeigt sind:
| R₃ | A |
|---|---|
| H | -N (CH₃)CH₂CH₂N(CH₃)₂. |

9. Eine Verbindung gemäß Anspruch 1 der Formel: 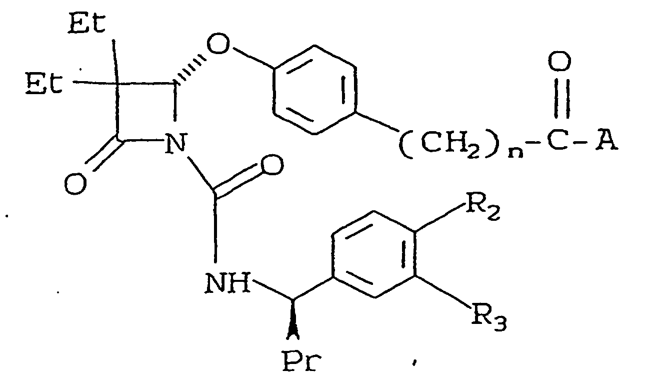 wobei n 0 ist und R₂, R₃ und A wie nachstehend gezeigt sind:
| R₃ | R₂ | A |
|---|---|---|
| H | -CON(CH₃)₂ | -N(CH₃)CH₂CH₂N(CH₃)₂ |
| H | -CO₂H | -N(CH₃)CH₂CH₂N(CH₃)₂ |
| H | -CH₂OH | -N(CH₃)CH₂CH₂N(CH₃)₂ |
| H | OCH₃ | -N(CH₃)CH₂CH₂N(CH₃)₂ |
| OCH₃ | H | -N(CH₃)CH₂CH₂N(CH₃)₂ |
| H | CN | -N(CH₃)CH₂CH₂N(CH₃)₂ |
| H | OEt | -N(CH₃)CH₂CH₂N(CH₃)CH₂Ph |
| H | 2-(4-Morpholinocarbonylphenyl) | -N(CH₃)CH₂CH₂N(CH₃)CH₂Ph |
| H | OCH₃ | 4-Methyl-1-piperazinyl |
| H | Cl | -N(CH₃)CH₂CH₂N(CH₃)₂ |
| H | Cl | -N(CH₃)CH₂CH₂N(Et)₂ |
| H | Cl | -N(CH₃)CH₂CH₂N(i-Pr)₂ |
| H | Cl | -N(CH₃)CH₂CH₂N(CH₃)CH₂Ph oder |
| OCH₃ | CH₃ | -N(CH₃)CH₂CH₂N(CH₃)₂. |

10. Eine Verbindung gemäß Anspruch 1 der Formel: 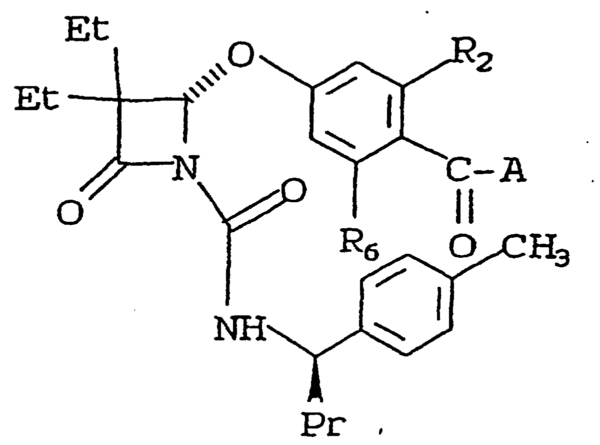 wobei R₂, R₆ und A wie nachstehend gezeigt sind:
| R₂ | R₆ | A |
|---|---|---|
| CH₃ | CH₃ | -N(CH₃)CH₂CH₂N(CH₃)₂ |
| CH₃ | CH₃ | -N(CH₃)CH₂CH₂N(Et)₂ |
| CH₃ | CH₃ | -N(Et)CH₂CH₂N(CH₃)₂ oder |
| H | OH | -N(CH₃)CH₂CH₂N(CH₃)₂. |

11. [S-(R*,S*)]-2-[4-[[(4-Methyl)piperazin-1-yl]carbonyl]phenoxy]-3,3-diethyl-N-[1-(3,4-methylendioxyphenyl)butyl]-4-oxo-1-azetidincarboxamid oder ein pharmazeutisch annehmbares Salz davon.

12. Eine pharmazeutische Zusammensetzung, die eine Verbindung gemäß irgendeinem vorhergehenden Anspruch und einen pharmazeutisch annehmbaren Träger enthält.

13. Die Verwendung einer Verbindung gemäß irgendeinem der Ansprüche 1-11 zur Herstellung eines Medikaments zur Inhibierung humaner Leukozytenelastase.

## Revendications

1. Composé de formule (I) : 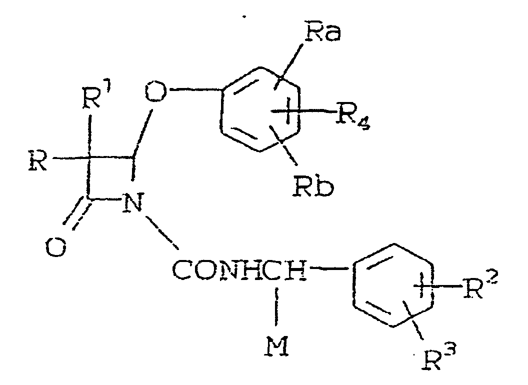 ou un sel pharmaceutiquement acceptable de celui-ci, dans lequel :
R est un alkyle en C₁ à C₆ ;
R¹ est un alkyle en C₁ à C₆ ou un alcoxy en C₁ à C₆-alkyle en C₁ à C₆ ;
M est
(1) un hydrogène,
(2) un alkyle en C₁ à C₆,
(3) un hydroxy alkyle en C₁ à C₆,
(4) un halogéno alkyle en C₁ à C₆,
(5) un alcényle en C₂ à C₆, ou
(6) un alcoxy en C₁ à C₆-alkyle en C₁ à C₆ ;
Ra et Rb sont chacun individuellement
(1) un hydrogène,
(2) un alkyle en C₁ à C₆,
(3) un halogéno,
(4) un carboxy,
(5) un alcoxy en C₁ à C₆,
(6) un phényle,
(7) un alkylcarbonyle en C₁ à C₆,
(8) un di-(alkyle en C₁ à C₆)amino ;
(9) un hydroxy ;
R² et R³ sont chacun indépendamment
(1) un hydrogène,
(2) un alkyle en C₁ à C₆,
(3) un halogéno,
(4) un carboxy,
(5) un alcoxy en C₁ à C₆,
(6) un phényle,
(7) un alkylcarbonyle en C₁ à C₆,
(8) un amino C₂ à C₃ alkyloxy carbonyle dans lequel l'amino est éventuellement mono ou di substitué avec un alkyle en C₁ à C₆,
(9) un amino en C₂ à C₃ alkylamino carbonyle dans lequel l'amino est éventuellement mono ou disubstitué avec un alkyle en C₁ à C₆
(10) un hydroxy,
(11) un aminocarbonyle dans lequel l'amino est éventuellement mono ou di substitué avec un alkyle en C₁ à C₆,
(12) un hydroxyméthyle,
(13) un aminocarbonyloxy alkyle en C₁ à C₃, dans lequel l'amino est éventuellement mono ou di substitué avec un alkyle en C₁ à C₆
(14) un cyano,
(15) un morpholinocarbonylphényle,
(16) un amino dans lequel l'amino est éventuellement mono ou di substitué avec un alkyle en C₁ à C₆, ou R² et R³ peuvent être joints ensemble pour former un groupe méthylènedioxy ou un cycle furane,
(17) un morpholinocarbonyle ;
R₄ est 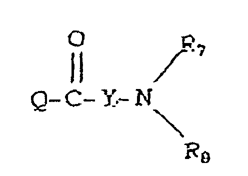
dans laquelle
Q est une liaison covalente et
Y est 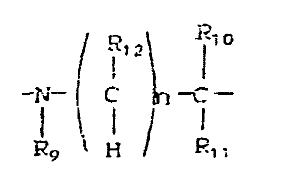
R₁₂ est un hydrogène ou un alkyle en C₁ à C₃ ;
R₇ et R₈ sont chacun individuellement
(a) un hydrogène,
(b) un alkyle en C₁ à C₆,
(c) un alkyloxy en C₁ à C₆ alkyle en C₂ à C₃,
(d) un hydroxy alkyle en C₂ à C₆,
(e) un polyhydroxy alkyle en C₂ à C₆,
(f) un carboxamido alkyle en C₁ à C₆,
(h) un alcanoyle en C₁ à C₆,
(i) un phényle ou un phényle alkyle en C₁ à C₆ substitué, dans lequel les substituants sont X₁ et X₂ tels que définis immédiatement ci-dessous,
(j) un alcényle en C₂ à C₆,
(k) un cycloalcényle en C₆ à C₁₀,
(l) un hétéroaryle alkyle en C₁ à C₆ dans lequel l'hétéroaryle est le pyridinyle, l'imidazolyle, le triazolyle, le benzylimidazolyle, ou le furyle,
(m) un carboxy alkyle en C₁ à C₆,
(n) un carbo alcoxy en C₁ à C₆ alkyle en C₁ à C₃,
(o) un phénylsulfonyle,
(p) un alkylsulfonyle en C₁ à C₆,
(q) un benzyloxy,
(r) un morpholinyl alkylsulfonyle en C₁ à C₃,
(s) un tétrahydropyranyle,
(t) un amino alkylsulfonyle en C₁ à C₃, dans lequel l'amino est éventuellement mono ou di substitué avec un alkyle en C₁ à C₆,
(u) un aminocarbonyle, dans lequel l'amino et éventuellement mono ou di substitué avec un alkyle en C₁ à C₆,
(v) un aminocarbonyloxy alkyle en C₂ à C₆, dans lequel l'amino est éventuellement mono ou di substitué avec un alkyle en C₁ à C₆,
(w) un azabicyclo de 7 à 12 atomes,
(x) un di alkylamino en C₁ à C₃ alkyle en C₂ à C₆, dans lequel l'amino est éventuellement mono ou di substitué avec un alkyle en C₁ à C₆,
(y) un bicycloalkyle de 7 à 12 atomes,
(z) un cycloalkyle en C₃ à C₁₀ éventuellement substitué avec un alkyle en C₁ à C₆,
(aa) un pyrazolidinyle,
(bb) un pipéridinyle ou un pyrrolidinyle substitué, dans lequel le substituant est un hydrogène, un alkyle en C₁ à C₃, un hydroxy alkylbenzyle en C₁ à C₃, un carboxamido ou un amino dans lequel l'amino est éventuellement mono ou di substitué avec un alkyle en C₁ à C₆,
(cc) un pyrrolidinyle substitué dans lequel le substituant est un carboxamido ou un amino dans lequel l'amino est éventuellement mono ou di substitué avec un alkyle en C₁ à C₆,
(dd) un pyrimidinyle,
(ee) un N-cyano-N'-phénylamidino,
(ff) un phosphono alkyle en C₁ à C₆, ou
(gg) un α-alkyle en C₁ à C₃ benzyle ou un benzyle mono ou di substitué ou un pyridylméthyle mono ou di substitué, dans lequel les substituants sont X₁ et X₂,
dans lequel
X₁ est
(1) un hydrogène,
(2) un halogéno,
(3) un alkyle en C₁ à C₆,
(4) un halogéno-alkyle en C₁ à C₆,
(5) un alcényle en C₂ à C₆,
(6) un hydroxy-alkyle en C₁ à C₆,
(7) un alkylcarbonyle en C₁ à C₆,
(8) un alkylcarbonylamino en C₁ à C₆,
(9) CN,
(10) CF₃,
(11) CH₃O,
(12) un amino dans lequel l'amino est éventuellement mono ou di substitué avec un alkyle en C₁ à C₆ ;
(13) un carboxy, ou
(14) un phénylsulfonylaminocarbonyle ;
X₂ est un hydrogène, un halogéno ou un alkyle en C₁ à C₆ ;
n vaut 1, 2, 3, 4 ou 5 ;
R⁹ est choisi parmi l'hydrogène, un alkyle en C₁ à C₄, et un alcoxy en C₁ à C₃-alkyle en C₁ à C₃ ; ou un phényle, un phényl alkyle en C₁ à C₃, un pyridyle, et un pyridyle alkyle en C₁ à C₃ ;
R¹⁰ et R¹¹ sont chacun choisis indépendamment parmi l'hydrogène, un alkyle en C₁ à C₄, et un alcoxy en C₁ à C₃ alkyle en C₁ à C₃, ou sont ensemble O= ; ou
R⁷ et R⁸ sont joints ensemble pour former un cycle mono ou di substitué de 4, 5, 6 ou 7 atomes ou 7 à 12 atomes tels que
(1) le pipéridinyle ou l'homopipéridinyle,
(2) le pipérazinyle,
(3) le morpholinyle, le thiomorpholinyle ou le 1,1-dioxo-4-thiomorpholinyle,
(4) le pyrrolidinyle,
(5) le pyrryle,
(6) l'imidazolyle,
(7) le triazolyle,
(8) l'azabicyclo saturé de 7 à 12 atomes,
(9) l'azaspiro ayant de 3 à 9 atomes de carbone, ledit cycle étant saturé,
(10) le tétrazolyle,
(11) le pyrazolidinyle,
(12) le dihydrodiméthoxyisoquinolyle,
(13) l'azétidinyle, ou
(14) un cycle azabicyclo de 7 à 12 atomes, dans lequel les substituants sont chacun choisis dans le groupe comprenant l'hydrogène et un alkyle en C₁ à C₃, le benzyloxycarbonyle, un carboxy, un phényl alkyle en C₁ à C₃ amino carbonyle, le pyrrolidinylméthyle, un hydroxy alkyle en C₁ à C₃, un alkyloxy en C₁ à C₆, un alkyloxy en C₁ à C₄ carbonyle, un aminocarbonyle dans lequel l'amino est éventuellement mono ou disubstitué avec un alkyle en C₁ à C₆, et un oxo ; ou
-N(R7)R8 peut être un résidu lysine ; ou
R₈ et R₉ sont joints ensemble pour former un cycle monocyclique saturé mono ou di substitué de 6 à 7 atomes et ayant deux atomes hétéro qui sont les azotes auxquels R₈ et R₉ sont attachés ; lesdits cycles comprennent le pipérazinyle et l'homopipérazinyle ; ou R₉ et R₁₀ sont joints ensemble pour former un cycle saturé monocyclique mono ou di substitué de 5 à 7 atomes et ayant un atome hétéro qui est l'azote auquel R₉ est attaché ; ou
R₉ et R₁₂ sont joints ensemble pour former un cycle monocyclique saturé mono ou di substitué de 5, 6, ou 7 atomes de carbone, ledit cycle ayant un atome hétéro qui est l'azote auquel R₉ est attaché ; ou
R₁₀ et R₁₂ sont joints ensemble pour former un cycle monocyclique saturé mono ou di substitué de 5, 6 ou 7 atomes de carbone ; ou
R₈ et R₁₁ sont joints ensemble pour former un cycle monocyclique saturé mono ou di substitué de 5, 6 ou 7 atomes, ledit cycle ayant un atome hétéro qui est l'azote auquel R₈ est attaché ; et les substituants sont choisis indépendamment parmi l'hydrogène et un alkyle en C₁ à C₃.

2. Composé selon la revendication 1, dans lequel
R est un alkyle en C₁ à C₆ ;
R¹ est un alkyle en C₁ à C₆ ou un alcoxy en C₁ à C₆-alkyle en C₁ à C₆ ;
M est
(1) un hydrogène,
(2) un alkyle en C₁ à C₆,
(3) un hydroxy alkyle en C₁ à C₆,
(4) un halogéno alkyle en C₁ à C₆,
(5) un alcényle en C₂ à C₆, ou
(6) un alcoxy en C₁ à C₆-alkyle en C₁ à C₆ ;
Ra est
(1) un hydrogène,
(2) un alkyle en C₁ à C₆,
(3) un halogéno,
(4) un carboxy,
(5) un alcoxy en C₁ à C₆,
(6) un phényle,
(7) un alkylcarbonyle en C₁ à C₆,
(8) un di-(alkyle en C₁ à C₆)amino ;
Rb est un hydrogène ou un alkyle en C₁ à C₆,
R² et R³ sont chacun indépendamment
(1) un hydrogène,
(2) un alkyle en C₁ à C₆,
(3) un halogéno,
(4) un carboxy,
(5) un alcoxy en C₁ à C₆,
(6) un phényle,
(7) un alkylcarbonyle en C₁ à C₆,
(8) un di-(alkyle en C₁ à C₆)amino, ou
R² et R³ peuvent être joints ensemble pour former un groupe méthylènedioxy ou un cycle furane ;
R₄ est 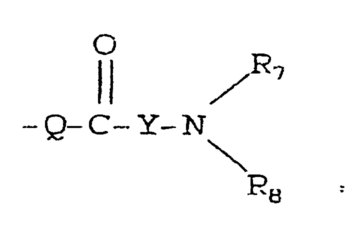
dans laquelle
Q est une liaison covalente,
Y est 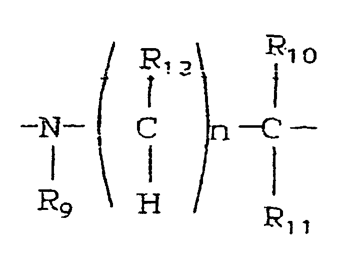
R₁₂ est un hydrogène ou un alkyle en C₁ à C₃ ;
R₇ et R₈ sont chacun individuellement
(a) un hydrogène,
(b) un alkyle en C₁ à C₆,
(c) un alkyloxy en C₁ à C₆ alkyle en C₂ à C₃,
(d) un hydroxy alkyle en C₂ à C₆,
(e) un carboxamido alkyle en C₁ à C₆,
(f) un alcanoyle en C₁ à C₆,
(g) un phényle ou un phényle alkyle en C₁ à C₆,
(h) un alcényle en C₂ à C₆,
(i) un cycloalcényle en C₆ à C₁₀,
(j) un hétéroaryl alkyle en C₁ à C₆ dans lequel l'hétéroaryle est le pyridinyle, l'imidazolyle, le triazolyle, le benzylimidazolyle, ou le furyle,
(k) un carboxy alkyle en C₁ à C₆ ou un carbo alcoxy en C₁ à C₆ alkyle en C₁ à C₆,
(l) un alkylsulfonyle en C₁ à C₆,
(m) un benzyloxy,
(n) un morpholinyl alkylsulfonyle en C₁ à C₃,
(o) un amino alkylsuflonyle en C₁ à C₃ dans lequel l'amino peut être éventuellement substitué avec un alkyle en C₁ à C₆,
(p) un aminocarbonyle dans lequel l'amino peut être éventuellement substitué avec un alkyle en C₁ à C₆,
(q) un aminocarbonyloxy alkyle en C₁ à C₆ dans lequel l'amino peut être éventuellement substitué avec un alkyle en C₁ à C₆,
(r) un di alkyle en C₁ à C₃ amino alkyle en C₁ à C₆ dans lequel l'amino peut être éventuellement substitué avec un alkyle en C₁ à C₆,
(s) un pyrazolidinyle,
(t) un pipéridinyle,
(u) un pyrrolidinyle,
(v) un pyrimidinyle,
(w) un cycloalkyle en C₃ à C₁₀,
(x) un α-alkyle en C₁ à C₃ benzyle ou un benzyle mono ou di substitué ou un pyridylémthyle mono ou di substitué, dans lequel les substituants sont X₁ et X₂,
dans lequel
X₁ est
(1) un hydrogène,
(2) un halogéno,
(3) un alkyle en C₁ à C₆,
(4) un halogéno-alkyle en C₁ à C₆,
(5) un alcényle en C₂ à C₆,
(6) un hydroxy-alkyle en C₁ à C₆,
(7) un alkylcarbonyle en C₁ à C₆,
(8) un alkylcarbonylamino en C₁ à C₆,
(9) un dialkylamino en C₁ à C₃ ; ou
(10) un carboxy,
X₂ est un hydrogène, un halogéno ou un alkyle en C₁ à C₆ ;
N vaut 1, 2, 3, ou 4 ;
R₉ est choisi parmi l'hydrogène, un alkyle en C₁ à C₄, et un alcoxy en C₁ à C₃-alkyle en C₁ à C₃ ;
R₁₀ et R₁₁ sont chacun choisis indépendamment parmi l'hydrogène, un alkyle en C₁ à C₄, et un alcoxy en C₁ à C₃ alkyle en C₁ à C₃ ; ou
R₇ et R₈ sont joints ensemble pour former un cycle mono ou di substitué choisis parmi
(1) le pipéridinyle,
(2) le pipérazinyle,
(3) le morpholinyle,
(4) le pyrrolidinyle,
(5) le pyrryle,
(6) l'imidazolyle,
(7) le triazolyle,
(8) le tétrazolyle,
(9) le pyrazolidinyle, ou
(13) l'azétidinyle,
dans lequel les substituants sont chacun choisis dans le groupe comprenant l'hydrogène et un alkyle en C₁ à C₃, le benzyloxycarbonyle, un carboxy, un phényl alkyle en C₁ à C₃ amino carbonyle, le pyrrolidinylméthyle, un hydroxy alkyle en C₁ à C₃, un alkyloxy en C₁ à C₆, un alkyloxy en C₁ à C₄ carbonyle, et oxo ; ou R₈ et R₉ sont joints ensemble pour former un cycle monocyclique saturé mono ou di substitué de 6 à 7 atomes et ayant deux atomes hétéro qui sont les azotes auxquels R₈ et R₉ sont attachés ; lesdits cycles comprennent le pipérazinyle et l'homopipérazinyle ; ou R₉ et R₁₀ sont joints ensemble pour former un cycle saturé monocyclique mono ou di substitué de 5 à 7 atomes et ayant un atome hétéro qui est l'azote auquel R₉ est attaché ; ou
R₉ et R₁₂ sont joints ensemble pour former un cycle monocyclique saturé mono ou di substitué de 5, 6, ou 7 atomes de carbone, ledit cycle ayant un atome hétéro qui est l'azote auquel R₉ est attaché ; ou
R₁₀ et R₁₂ sont joints ensemble pour former un cycle monocyclique saturé mono ou di substitué de 5, 6 ou 7 atomes de carbone ; ou
R₈ et R₁₁ sont joints ensemble pour former un cycle monocyclique saturé mono ou di substitué de 5, 6 ou 7 atomes, ledit cycle ayant un atome hétéro qui est l'azote auquel R₈ est attaché ; et les substituants sont choisis indépendamment parmi l'hydrogène et un alkyle en C₁ à C₃.

3. Composé selon la revendication 2, dans lequel
R est un alkyle en C₁ à C₃ ;
R₁ est un alkyle en C₁ à C₃ ;
M est
(a) un alkyle en C₁ à C₆, ou
(b) un alcényle en C₂ à C₆ ;
R² est
(a) un hydrogène,
(b) un alkyle en C₁ à C₆, ou un alcoxy en C₁ à C₆, et
R³ est un hydrogène, ou
R² et R³ sont joints ensemble pour former un groupe méthylènedioxy ou un cycle furane ;
R₇ et R₈ sont choisis chacun indépendamment parmi
(a) un hydrogène,
(b) un alkyle en C₁ à C₃,
(c) un alcoxy en C₁ à C₃ alkyle en C₂ à C₃,
(d) un cycloalkyle en C₃ à C₇,
(e) un hydroxy alkyle en C₂ à C₃,
(f) un carbo alcoxyméthyle en C₁ à C₄,
(g) un benzyle substitué dans lequel les substituants sont X₁ et X₂
dans lesquels X₁ est un hydrogène et X₂ est
(1) un hydrogène,
(2) un halogéno, ou
(3) un alkyle en C₁ à C₃ ;
n vaut 1, 2 ou 3, et
R₉, R₁₀ et R₁₁ sont choisis chacun indépendamment parmi l'hydrogène, un alkyle en C₁ à C₄, et un alcoxy en C₁ à C₃ alkyle en C₁ à C₃ ; ou
R₇ et R₈ sont joints ensemble pour former un cycle substitué choisi parmi
(a) le pipéridinyle,
(b) le pipérazinyle, et
(c) le morpholinyle ;
ou
R₈ et R₉ sont joints ensemble pour former un cycle monocyclique saturé mono ou di substitué de 6 ou 7 atomes, ledit cycle ayant deux atomes hétéro qui sont les azotes auxquels R₈ et R₉ sont attachés.

4. Composé selon la revendication 1, de formule : 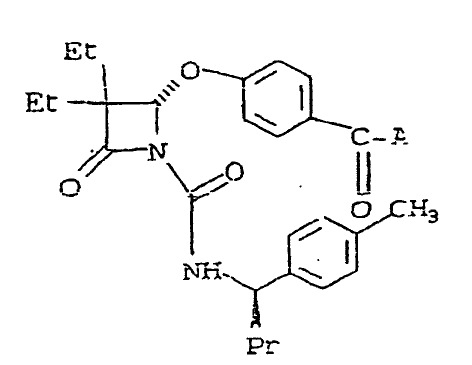 dans laquelle A est :
-NHCH₂CH₂N(CH₃)₂
-N(CH₃)CH₂CH₂N(CH₃)₂
-N(Et)CH₂CH₂N(CH₃)₂
-NHCH₂CH₂N(Et)₂
-NHCH₂CH₂-(4-morpholinyl)
-N(CH₃)CH₂CH₂-(4-morpholinyl)
-N(CH₃)CH₂CH₂N(CH₂CH₂OCH₃)₂
-N(CH₃)CH₂CH₂N(Et)₂
-N(Ph)CH₂CH₂N(CH₃)₂
-N(CH₃)CH₂CH₂CH₂N(CH₃)₂
-NHCH₂CH₂N(i-Pr)₂
-N(CH₃)CH₂CH₂N(O)(CH₃)₂
-N(CH₃)CH₂CH₂N(i-Pr)₂
-NH-SO₂CH₂CH₂-(4-morpholinyl)
-NH-SO₂CH₂CH₂N(CH₃)₂
-NHCH₂CH₂-(4-imidazolyl)
-NHCH₂CH₂-(1-pipéridinyl)
-N(CH₃)CH₂CH₂-(1-pipéridinyl)
-N(CH₃)CH₂CH₂NHCH₃
-N(CH₃)CH₂CH₂N(CH₃)Ac
-NHCH₂CH₂-(1-pyrrolidinyl)
-N(CH₃)CH₂CH₂-(1-pyrrolidinyl)
-NHCH₂CH₂-(1H-1,2,4-triazol-1-yl)
-NH-CH₂CH₂-(1-imidazolyl)
-NH-CH₂CH₂-(3-azabicyclo[3.2.2]non-3-yl)
-NH-CH₂CH₂-(3-azaspiro[5.5]-undéc-3-yl)
-NH-CH₂CH₂-(2H-tétrazol-2-yl)
-NH-CH₂CH₂-(2H-tétrazol-1-yl)
-NHCH₂C(O)-Pro-NHCH₂Ph
-N(CH₃)CH₂CH₂-(3-azabicyclo[3.2.2]non-3-yl)
-N(CH₃)CH₂CH₂-(4-imidazolyl)
-N(CH₃)CH₂CH₂N(CH₃)Ac
-N(CH₃)CH₂CH₂N(CH₃)C(O)NHCH₃
-N(CH₃)CH₂CH₂N(CH₃)SO₂CH₃
-N(CH₃)CH₂CH₂(3-azabicyclo[3.2.2]non-3-yl)
-NH-CH₂CH₂-(1,1-dioxo-4-thiamorpholinyl)
-N(CH₃)CH₂CH₂-(1,1-dioxo-4-thiamorpholinyl)
4-diméthylaminoanilino
-NHCH₂CH₂-(1-benzyl-1H-imidazol-2-yl)
-N(CH₃)CH₂CH₂(2-pyridyle)
-N(CH₃)(1-azabicyclo[2.2.2]oct-3-yl)
-NHCH₂CH₂(4-benzyloxycarbonyl-1-pipérazinyl)
1,2-diéthylpyrazolidin-4-ylamino
2-(1-S-pyrrolidinylméthyl)-1-pyrrolidinyl
-NHCH₂CH₂(4-hydroxy-1-pipéridinyl)
-NHCH₂CH₂(1-homopipéridinyl)
-N(CH₃)CH₂CH₂(1-homopipéridinyl)
-NHCH₂CH₂(3-hydroxy-1-pipéridinyl)
-N(CH₃)CH₂CH₂(3-hydroxy-1-pipéridinyl)
-N(CH₃)CH₂CH₂N(CH₃)CH₂Ph
-N(CH₃)CH₂CH₂N(4-benzyloxy-1-pipéridinyl)
-N(CH₃)CH₂CH₂(4-hydroxy-1-pipéridinyl)
-N(CH₃)CH₂CH₂(4-oxo-1-pipéridinyl)
-NHCH₂CH₂(3-hydroxy-1-pyrrolidinyl)
-N(Et)CH₂CH₂(1-pipéridinyl)
-N(CH₂Ph)CH₂CH₂(1-pipéridinyl)
-N(CH₂Ph)CH₂CH₂N(CH₃)₂
-N(CH₃)CH₂CH₂(3-hydroxy-1-pyrrolidinyl)
-N(3-picolyl)CH₂CH₂(1-pipéridinyl)
-NHCH(CH₃)CH₂CH₂CH₂N(Et)₂
-NHCH₂CH₂(2-S-hydroxyméthyl-1-pyrrolidinyl)
-N(CH₃)CH₂CH₂(4-t-butoxycarbonyl-1-pipérazinyl)
-N[CH₂CH₂N(CH₃)₂]₂
-N[CH₂CH₂N(Et)₂]₂
-N(CH₃)CH₂CH₂N(CH₃)(3-picolyl)
3,5-diméthyl-1-pipérazinyl
-N(CH₃)CH₂CH₂N(O)(CH₃)CH₂Ph
-N(CH₃)CH₂CH₂N(CH₃)(4-picolyl)
2-S-(N-benzyl-N-méthylaminométhyl)-1- pyrrolidinyl
-N(CH₃)CH₂CH₂N(CH₃)(2-picolyl)
-N(CH₃)CH₂CH₂(1-pipérazinyl)
1-homopipérazinyl
-N(CH₃)CH₂CH₂N(CH₃)CH₂CH₂Ph
2-(1-R-pyrrolidinylméthyl)-1-pyrrolidinyl
4-benzyl-1-homopipérazinyl
-N(CH₃)CH₂CH₂N(CH₃)(1-naphtalénylméthyl)
-N(CH₃)CH₂CH₂N(CH₃)(2-naphtalénylméthyl)
-N(CH₃)CH₂CH₂N(CH₃)CH(CH₃)Ph
-N(CH₃)CH₂CH₂N(CH₂Ph)₂
1-éthyl-3-pipéridinylamino
-N(CH₃)CH₂CH₂N(CH₃)(2-furfuryl)
-N(CH₃)CH₂CH₂CH₂C(O)NHSO₂Ph
-N(CH₃)CH₂CH₂N(CH₃)CH₂CH=CH₂
-N(CH₃)CH₂CH₂CH₂N(CH₃)CH₂Ph
-N(CH₃)-(CH₂)₆-N(CH₃)CH₂Ph
-N(CH₃)CH₂CH₂OC(O)N(CH₃)₂
-N(CH₃)CH₂CH₂N(CH₃)CH₂CO₂-t-Bu
-N(CH₃)(1-éthyl-3-pipéridinyl)
-N(CH₃)CH₂CH₂N(CH₃)(tétrahydro-2H-pyran-2-yl-méthyl)
2,2,6,6-tétraméthylpipéridin-4-ylamino
-N(CH₃)CH₂CH₂N(CH₃)(4-cyanobenzyl)
-N(CH₃)CH₂CH₂N(CH₃)(4-méthylbenzyl)
-N(CH₃)CH₂CH₂N(CH₃)(3-cyanobenzyl)
-N(CH₃)CH₂CH₂N(CH₃)(4-trifluorométhylbenzyl)
-N(CH₃)CH₂CH₂N(CH₃)(3-trifluorométhylbenzyl)
-N(CH₃)CH₂CH₂N(CH₃)(cyclopropylméthyl)
-N(CH₃)CH₂CH(Ph)N(CH₃)₂
-N(CH₃)(1-benzyl-3-pipéridinyl)
-N(3-picolyl)CH₂CH₂N(CH₃)CH₂Ph
-N(CH₃)CH₂CH₂N(CH₃)(4-méthoxybenzyl)
-N(4-picolyl)CH₂CH₂N(CH₃)CH₂Ph
-N(2-picolyl)CH₂CH₂N(CH₃)CH₂Ph
-N(CH₃)CH₂CH₂N(CH₃)(2,4-diméthylbenzyl)
-N(CH₃)CH₂CH₂(2,6-diméthyl-4-morpholinyl)
-N(CH₃)CH₂CH₂N(CH₃)C(=N-CN)NHPh
-N(CH₃)CH₂CH₂N(CH₃)(3-fluorobenzyl)
-N(CH₃)CH₂CH₂N(CH₃)(2-chlorobenzyl)
-N(CH₃)CH₂CH₂N(CH₃)(3-méthoxybenzyl)
-N(CH₃)CH₂CH₂N(CH₃)(3,5-diméthoxybenzyl)
-N(CH₃)(1-benzyl-4-pipéridinyl)
-N(CH₃)CH₂CH₂N(CH₃)(2-adamantyl)
-N(CH₃)(4-pipéridinyl)
5-méthyl-2,5-diazabicyclo[2.2.1]hept-2-yl
-N(CH₃)CH₂CH₂CH₂N(CH₃)CH₂CH₃
-N(CH₃)(1-méthyl-4-pipéridinyl)
-N(CH₃)(1-propyl-4-pipéridinyl)
-N(CH₃)(1-éthyl-4-pipéridinyl)
-N(CH₃)CH₂CH(CH₃)N(CH₃)CH₂Ph
-N(CH₃)CH₂CH(CH₃)N(CH₃)₂
-N(CH₃)CH₂CH₂N(CH₃)(bicyclo[2.2.1]hept-2-yl)
-N(CH₃)CH₂CH₂NH(2-adamantyl)
-N(CH₃)CH₂CH₂N(CH₃)(6, 6-diméthylbicyclo[3.1.1]hept-2-yl)
-N(CH₃)CH₂CH₂N(CH₃)(bicyclo[3.2.1]oct-2-yl)
-N(CH₃)CH₂CH₂N(CH₃)(1-cyclohexèn-1-yl)
-N(CH₃)CH₂CH₂NHC(CH₃)₂CH=CH₂
2-S-carboxamido-1-pyrrolidinyl
2-hydroxyméthyl-1-pipéridinyl
3-diméthylamino-1-pyrrolidinyl
-N(CH3)CH2CH2N(CH3)(cyclohexylméthyl)
-N(CH₃)CH₂CH₂N(CH₂CH=CH₂)C(CH₃)₂CH=CH₂
-N(CH₃)CH₂CH₂N(CH₃) (4-éthylcyclohexyl)
-N(CH₃)CH₂CH₂N(CH₃) (2-éthylcyclohexyl)
-N(CH₃)CH₂CH₂N(CH₃) (4-méthylcyclohexyl)
-N(CH₃)CH₂CH₂N(CH₃) (cyclohexyl)
-N(CH₃)CH₂CH₂N(CH₃)CH₂CO₂H-TFA
-N(CH₃)CH₂CH₂N(CH₃)CH₂C(O)N(CH₃)₂
-N(CH)CH₂CH₂N(CH₃)(cyclohexylméthyl) ou
-NHCH₂CH₂N(Et)CH₂CH₂OCH₃

5. Composé selon la revendication 1, de formule : 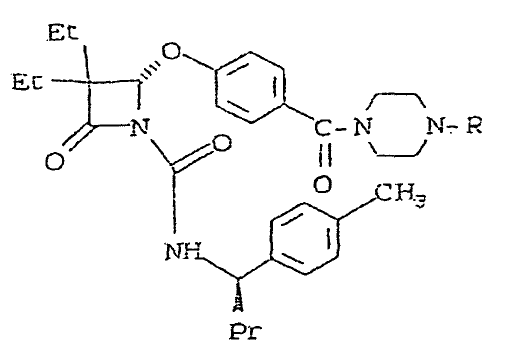 dans laquelle R est :
-CH₃
le 4-fluorophényle
le 3-chlorophényle
le phényle
le benzyle
H
i-Pr
i-Bu
-CH₂CO₂Et
-CH₂CO₂H
Et
Pr
le 2-pyrimidinyle
-CH₂CH₂OC(O)NHCH₃
le cyclopropyle ou
-CH₂CH₂OH

6. Composé selon la revendication 1, de formule : 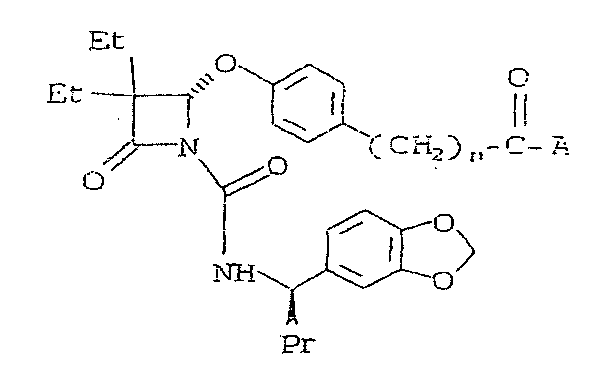 dans laquelle n vaut 0 et A est :
-N(CH₃)CH₂CH₂N(CH₃)₂
le 4-méthyl-1-pipérazinyle
-N(CH₃)CH₂CH₂N(CH₃)CH₂Ph
le 4-cyclopropyl-1-pipérazinyle
le 1-pipérazinyle ou
le 4-(2-hydroxyéthyl)-1-pipérazinyle.

7. Composé selon la revendication 1, de formule : 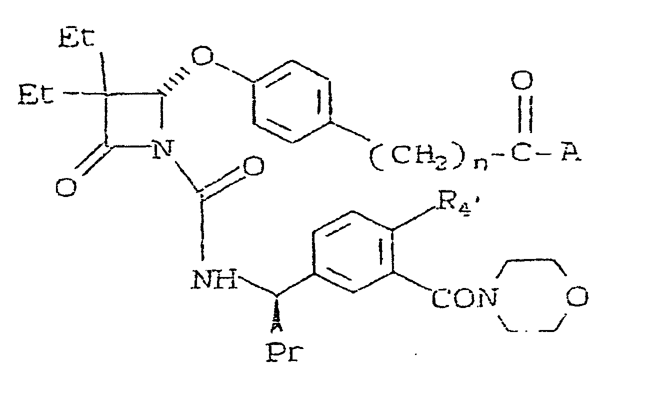 dans laquelle n vaut 0 et R_{4'} et A sont tels que montrés ci-dessous :
| R₄' | A |
|---|---|
| Et | -N(CH₃)CH₂CH₂N(CH₃)₂. |

8. Composé selon la revendication 1, de formule : 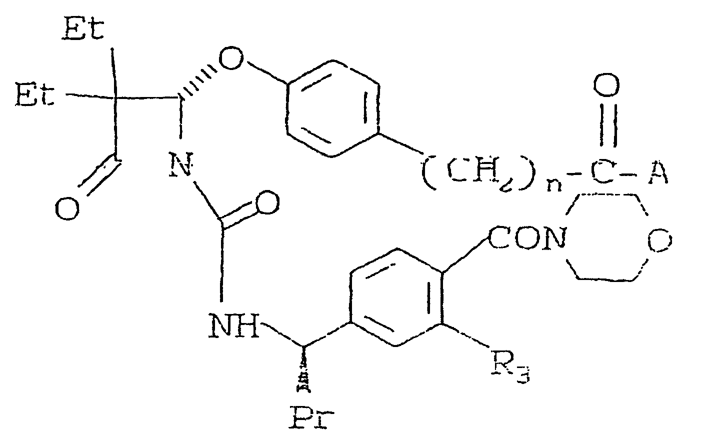 dans laquelle n vaut 0 et R₃ et A sont tels que montrés ci-dessous
| R₃ | A |
|---|---|
| H | -N(CH₃)CH₂CH₂N(CH₃)₂ |

9. Composé selon la revendication 1, de formule : 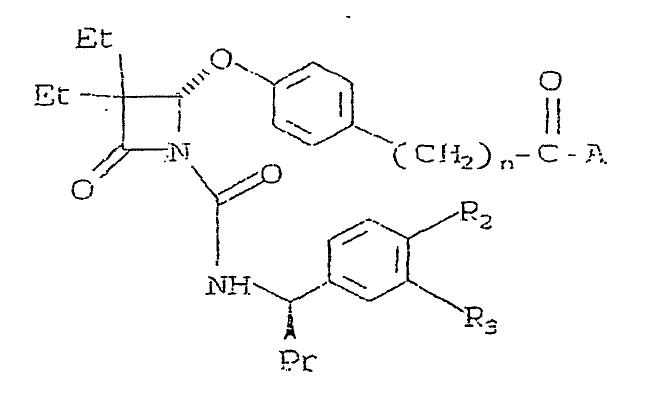 dans laquelle n vaut 0 et R₂, R₃ et A sont tels que montrés ci-dessous :
| R₃ | R₂ | A |
|---|---|---|
| H | -CON(CH₃)₂ | -N(CH₃)CH₂CH₂N(CH₃)₂ |
| H | -CO₂H | -N(CH₃)CH₂CH₂N(CH₃)₂ |
| H | -CH₂OH | -N(CH₃)CH₂CH₂N(CH₃)₂ |
| H | OCH₃ | -N(CH₃)CH₂CH₂N(CH₃)₂ |
| OCH₃ | H | -N(CH₃)CH₂CH₂N(CH₃)₂ |
| H | CN | -N(CH₃)CH₂CH₂N(CH₃)₂ |
| H | OEt | -N(CH₃)CH₂CH₂N(CH₃)CH₂Ph |
| H | 2-(4-morpholinocarbonylphényl) | -N(CH₃)CH₂CH₂N(CH₃)CH₂Ph |
| H | OCH₃ | 4-méthyl-1-pipérazinyl |
| H | Cl | -N(CH₃)CH₂CH₂N(CH₃)₂ |
| H | Cl | -N(CH₃)CH₂CH₂N(Et)₂ |
| H | Cl | -N(CH₃)CH₂CH₂N(i-Pr)₂ |
| H | Cl | -N(CH₃)CH₂CH₂N(CH₃)CH₂Ph ou |
| OCH₃ | CH₃ | -N(CH₃)CH₂CH₂N(CH₃)₂ |

10. Composé selon la revendication 1, de formule : 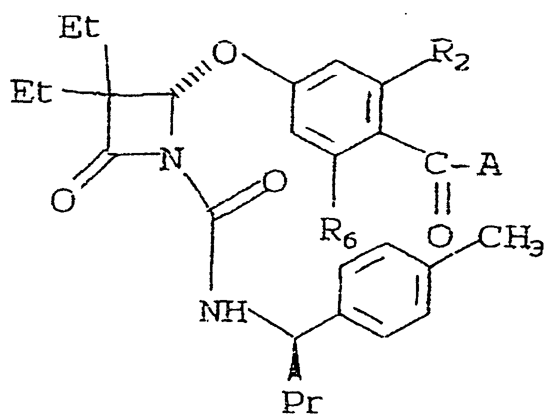 dans laquelle R₂, R₆ et A sont tels que montrés ci-dessous :
| R₂ | R₆ | A |
|---|---|---|
| CH₃ | CH₃ | -N(CH₃)CH₂CH₂N(CH₃)₂ |
| CH₃ | CH₃ | -N(CH₃)CH₂CH₂N(Et)₂ |
| CH₃ | CH₃ | -N(Et)CH₂CH₂N(CH₃)₂ ou |
| H | OH | -N(CH₃)CH₂CH₂N(CH₃)₂ |

11. [S-(R*,S*)]-2-[[(4-méthyl)pipérazin-1-yl]carbonyl]phénoxy]-3,3-diéthyl-N-[1-(3,4-méthylènedioxyphényl)butyl]-4-oxo-1-azétidinecarboxamide ou un sel pharmaceutiquement acceptable de celui-ci.

12. Composition pharmaceutique comprenant un composé selon l'une quelconque des précédentes revendications et un véhicule pharmaceutiquement acceptable.

13. Utilisation d'un composé selon l'une quelconque des revendications 1 à 11 pour la fabrication d'un médicament pour l'inhibition de l'élastase leucocytaire humaine.
